# EUROPEAN PATENT SPECIFICATION

(11) **EP 4 559 921 B1**
(45) Date of publication and mention of the grant of the patent: **26.11.2025**
(21) Application number: 23873055.0
(22) Date of filing: 26.09.2023
(51) Int. Cl.: C07F 7/02, C07F 9/50, C05F 11/00, B01J 31/34, C07C 11/02, C07C 11/107, C07C 2/30, B01J 31/18

(54) **LIGAND COMPOUND, ORGANIC CHROMIUM COMPOUND AND CATALYST COMPOSITION COMPRISING SAME**
LIGANDENVERBINDUNG, ORGANISCHE CHROMVERBINDUNG UND KATALYSATORZUSAMMENSETZUNG DAMIT
COMPOSÉ LIGAND, COMPOSÉ DE CHROME ORGANIQUE ET COMPOSITION DE CATALYSEUR LE COMPRENANT

(30) Priority: 28.09.2022 KR 20220123358
(43) Date of publication of application: 28.05.2025
(73) Proprietor: LG Chem, Ltd., Yeongdeungpo-gu Seoul 07336 (KR)
(72) Inventor: LIM, Won Taeck, Daejeon 34122 (KR); KIM, Seok Sun, Daejeon 34122 (KR); CHO, Yeon Ho, Daejeon 34122 (KR); JUNG, Seung Hwan, Daejeon 34122 (KR); SHIN, Eun Ji, Daejeon 34122 (KR); KIM, Tae Hee, Daejeon 34122 (KR); SA, Seok Pil, Daejeon 34122 (KR); MIN, Se Hye, Daejeon 34122 (KR)
(74) Representative: Hoffmann Eitle
(86) International application number: PCT/KR2023/014731
(87) International publication number: WO 2024/071940

(56) References cited:
- CN-A- 111 905 832
- KR-A- 20190 077 735
- KR-A- 20220 036 234
- KR-A- 20220 036 234
- US-A1- 2010 081 842
- US-A1- 2016 075 616
- CAO CHENGANG ET AL: "Chromium Catalysts Based on Unsymmetrical PNP Ligands for Selective Ethylene Tri-/Tetramerization: Effect of Electron-Withdrawing/Donating Substituents on Catalytic Performance", CATALYSTS, vol. 12, no. 9, 25 August 2022 (2022-08-25), CH, pages 944, XP093155787, ISSN: 2073-4344, DOI: 10.3390/catal12090944
- CHENGANG CAO: "Chromium Catalysts Based on Unsymmetrical PNP Ligands for Selective Ethylene Tri-/Tetramerization: Effect of Electron-Withdrawing/Donating Substituents on Catalytic Performance", CATALYSTS, M D P I AG, CH, vol. 12, no. 9, 25 August 2022 (2022-08-25), CH , pages 944, XP093155787, ISSN: 2073-4344, DOI: 10.3390/catal12090944

## Description

### TECHNICAL FIELD

### Cross-reference to Related Application

This application claims the benefit of Korean Patent Application No. 10-2022-0123358, filed on September 28, 2022, in the Korean Intellectual Property Office

### Technical Field

The present invention relates to a ligand compound, an organic chromium compound, a catalyst composition including the organic chromium compound, and a method for oligomerizing ethylene by using the same. (method not part of the invention).

### BACKGROUND ART

Linear alpha-olefins such as 1-hexene and 1-octene are used as cleaning agents, lubricants, plasticizers, etc., and particularly, are mainly used as a co-monomer for polymer density control during the manufacture of linear low-density polyethylene (LLDPE).

In a typical manufacturing process of linear low-density polyethylene (LLDPE), in order to control the density by forming a branch in a polymer backbone with ethylene, copolymerization is performed with a co-monomer such as an alpha-olefin, for example, 1-hexene or 1-octene.

Therefore, in order to manufacture LLDPE having a high co-monomer content, there has been a problem in that the price of the co-monomer accounts for a large portion of the manufacturing cost. There have been many attempts to solve the above-described problem.

Such linear alpha-olefins have been mainly manufactured through the Shell Higher Olefin Process. However, the above-described method simultaneously synthesizes alpha-olefins having various lengths according to the Schultz-Flory distribution, so that a separate separation process is required to obtain a specific alpha-olefin, which is cumbersome.

In order to solve the above-described problem, a method for selectively synthesizing 1-hexene through a trimerization reaction of ethylene or selectively synthesizing 1-octene through a tetramerization reaction of ethylene has been proposed. In addition, many studies have been conducted on a catalyst system that enables such selective oligomerization of ethylene.

### [Prior Art Document]

### [Patent Document]

(Patent Document 1) US 5064802 B2, (Patent document 2) KR 2022 0036234 A discloses oligomerization catalysts based on a PNP ligand, a chromium source and a co-catalyst. The PNP ligand bears all trialkylsilyl groups in para position.

### DISCLOSURE OF THE INVENTION

### TECHNICAL PROBLEM

An aspect of the present invention provides a ligand compound having a novel structure, which exhibits high catalytic activity while exhibiting high 1-hexene and 1-octene selectivity, thereby allowing ethylene oligomerization to be achieved with excellent efficiency, an organic chromium compound, and a catalyst composition including the organic chromium compound.

### TECHNICAL SOLUTION

According to an aspect of the present invention, there are provided a ligand compound, an organic chromium compound, a catalyst composition, and an ethylene oligomerization method.
(1) The present invention provides a ligand compound represented by Formula 1 below. 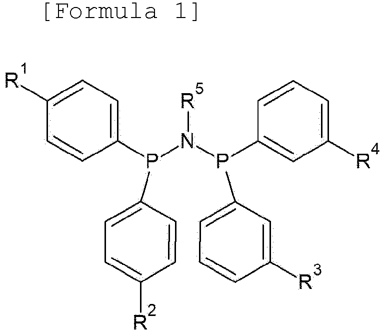 In Formula 1 above, R¹ to R⁴ are each independently an alkyl group having 5 to 20 carbon atoms, a cycloalkyl group having 5 to 20 carbon atoms, an alkoxyalkyl group having 6 to 20 carbon atoms, an arylalkoxyalkyl group having 10 to 30 carbon atoms, or a trialkylsilyl group, wherein the alkyl groups of the trialkylsilyl group are each independently an alkyl group having 1 to 10 carbon atoms, and R⁵ is an alkyl group having 1 to 20 carbon atoms, an alkyl group having 1 to 20 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.
(2) In (1) above, the present invention provides a ligand compound, wherein R¹ to R⁴ are each independently an alkyl group having 8 to 12 carbon atoms, a tripropylsilyl group, or a tributylsilyl group.
(3) In (1) or (2) above, the present invention provides a ligand compound, wherein R¹ to R⁴ are each independently an n-decyl group, a tripropylsilyl group, or a tributylsilyl group.
(4) In (1) to (3) above, the present invention provides a ligand compound, wherein R⁵ is an alkyl group having 3 to 5 carbon atoms, an alkyl group having 1 to 5 carbon atoms substituted with an aryl group having 6 to 10 carbon atoms, a cycloalkyl group having 5 to 8 carbon atoms, or a cycloalkyl group having 5 to 8 carbon atoms fused with an aryl group having 6 to 10 carbon atoms.
(5) In any one of (1) to (4) above, the present invention provides a ligand compound, wherein the ligand compound represented by Formula 1 above is represented by Formula 2 below. 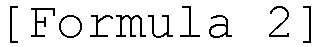 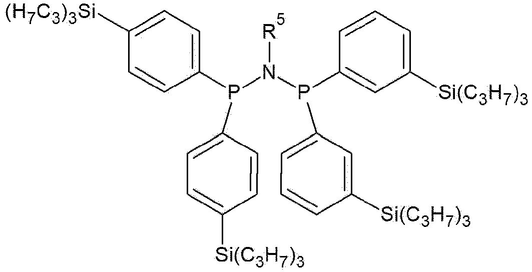 In Formula 2 above, R⁵ is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.
(6) In (5) above, the present invention provides a ligand compound, wherein the ligand compound represented by Formula 2 above is one selected from the group consisting of ligand compounds represented by Formula 2-1 to Formula 2-10 below. 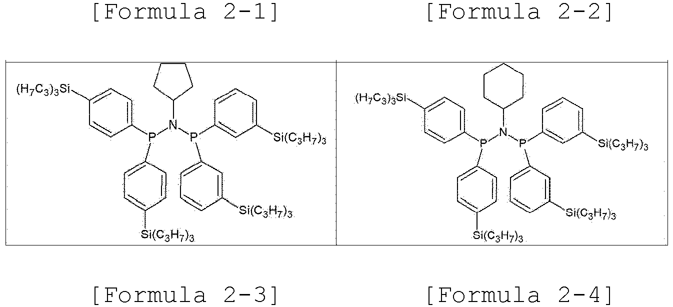 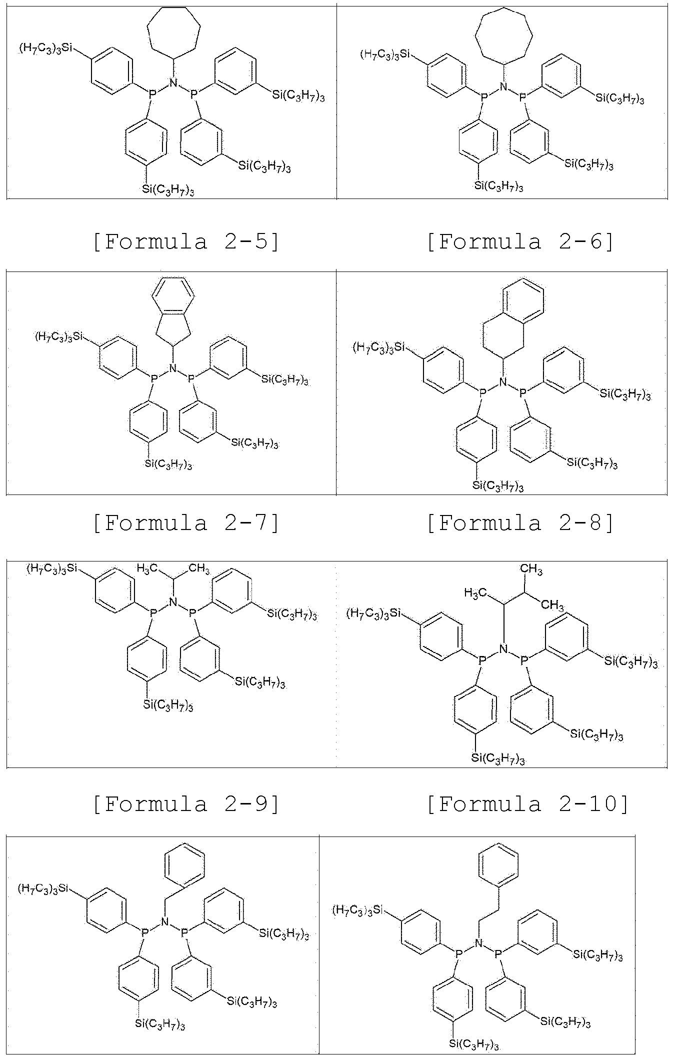
(7) In any one of (1) to (4) above, the present invention provides a ligand compound, wherein the ligand compound represented by Formula 1 above is represented by Formula 3 below. 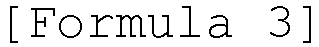 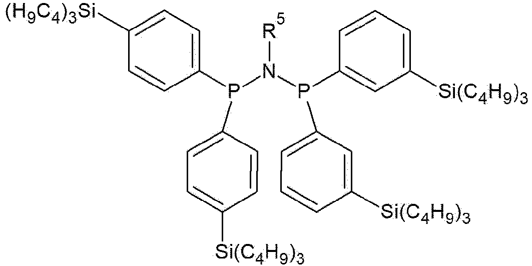 In Formula 3 above, R⁵ is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.
(8) In (7) above, the present invention provides a ligand compound, wherein the ligand compound represented by Formula 3 above is one selected from the group consisting of ligand compounds represented by Formula 3-1 to Formula 3-10 below. 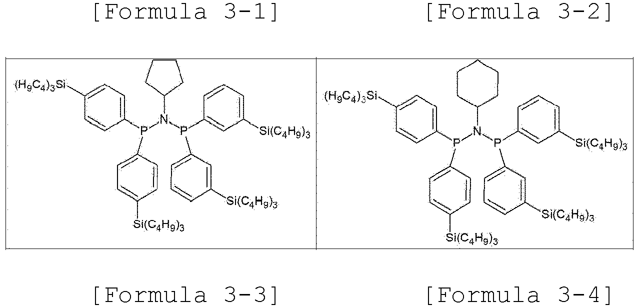 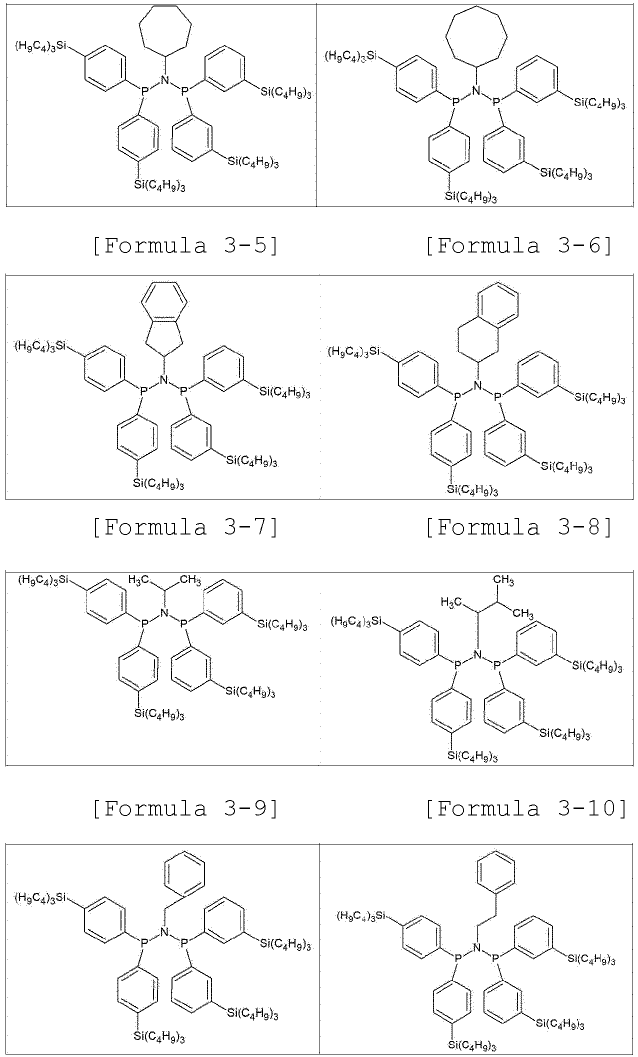
(9) In any one of (1) to (4) above, the present invention provides a ligand compound, wherein the ligand compound represented by Formula 1 above is represented by Formula 4 below. 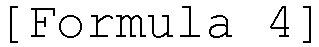 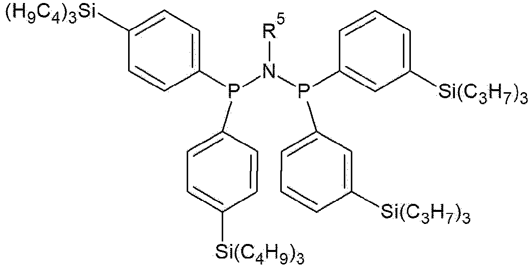 In Formula 4 above, R⁵ is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.
(10) In (9) above, the present invention provides a ligand compound, wherein the ligand compound represented by Formula 4 above is one selected from the group consisting of ligand compounds represented by Formula 4-1 to Formula 4-10 below. 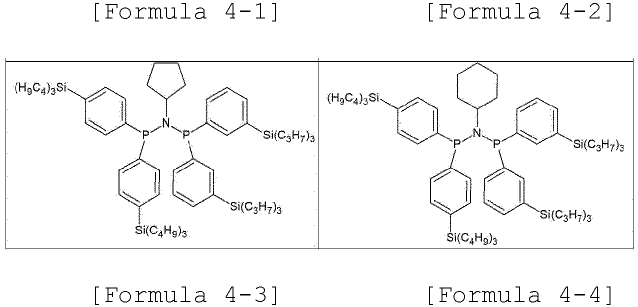 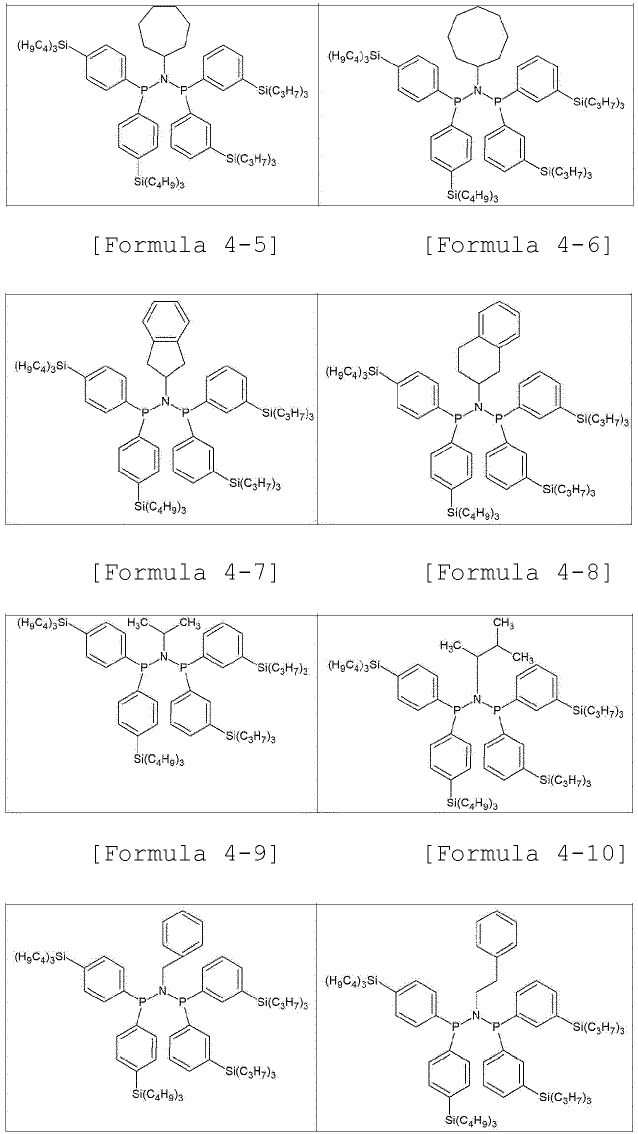
(11) In any one of (1) to (4) above, the present invention provides a ligand compound, wherein the ligand compound represented by Formula 1 above is represented by Formula 5 below. 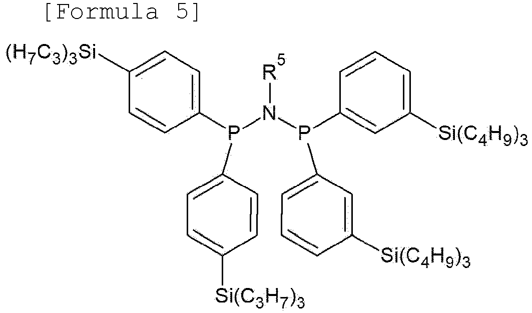 In Formula 5 above, R⁵ is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.
(12) In (11) above, the present invention provides a ligand compound, wherein the ligand compound represented by Formula 5 above is one selected from the group consisting of ligand compounds represented by Formula 5-1 to Formula 5-10 below. 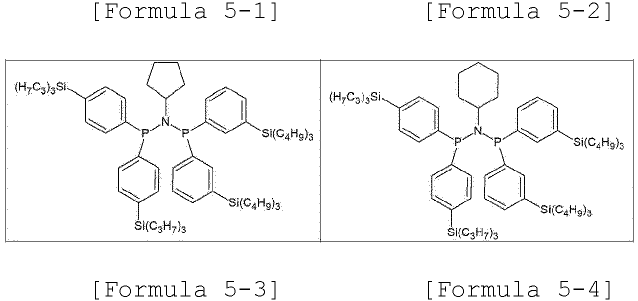 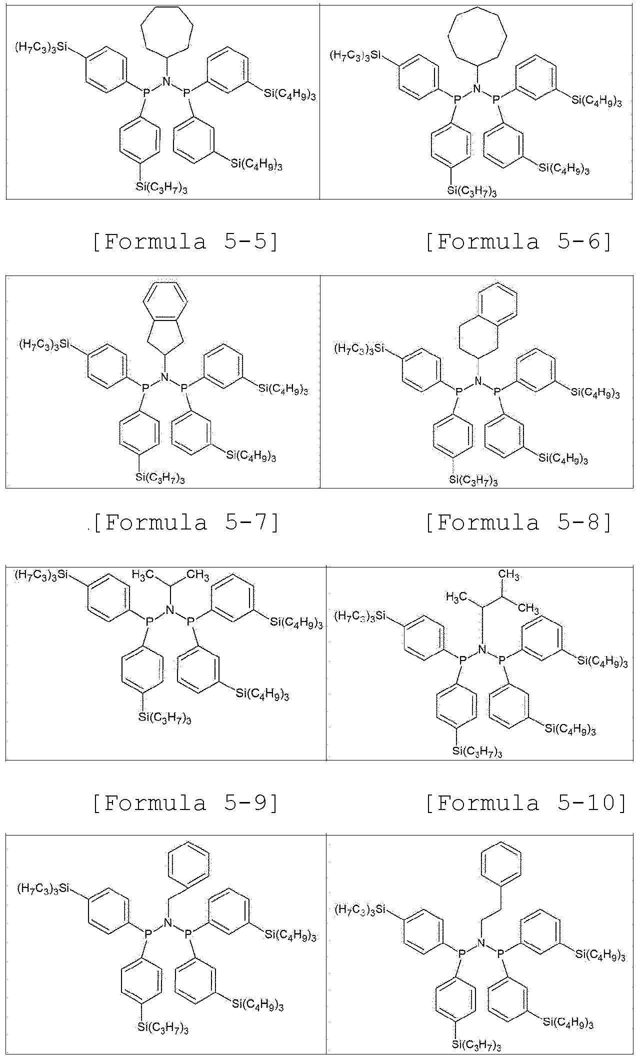
(13) In any one of (1) to (4) above, the present invention provides a ligand compound, wherein the ligand compound represented by Formula 1 above is represented by Formula 6 below. 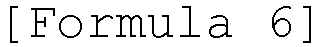 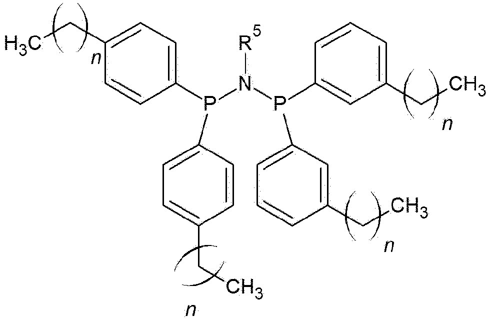 In Formula 6 above, R⁵ is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms, and n is an integer selected from 4 to 14.
(14) In (13) above, the present invention provides a ligand compound, wherein the ligand compound represented by Formula 6 above is one selected from the group consisting of ligand compounds represented by Formula 6-1 to Formula 6-10 below. 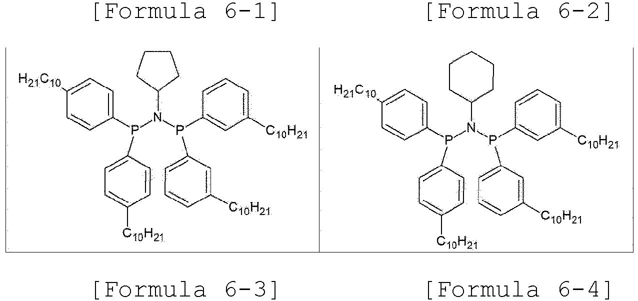 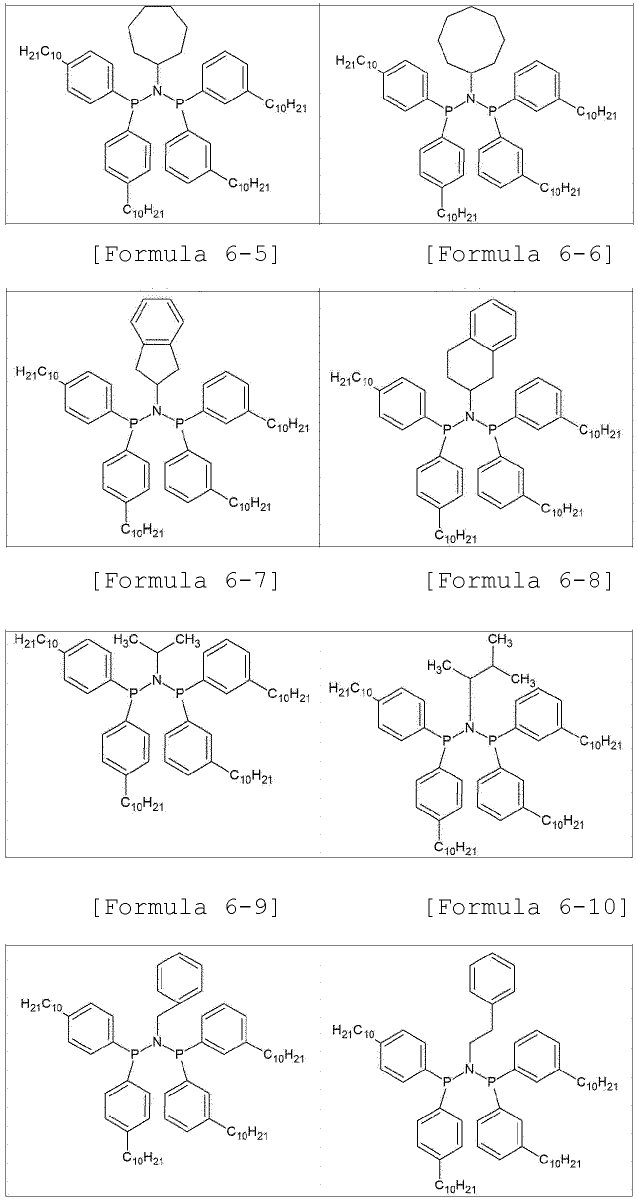
(15) The present invention provides an organic chromium compound including the ligand compound according to any one among (1) to (14) above and chromium coordinated to the ligand compound.
(16) In (15) above, the present invention provides an organic chromium compound, wherein in the ligand compound represented by Formula 1 above, at least one unshared electron pair of N and two Ps is in the form of being coordinated to chromium.
(17) The present invention provides a catalyst composition including the ligand compound according to any one among (1) to (14) above, chromium, and a co-catalyst.
(18) In (17) above, the present invention provides a catalyst composition, wherein the chromium is derived from a chromium source, wherein the chromium source includes at least one selected from the group consisting of chromium(III) acetylacetonate, chromium(III) chloride tetrahydrofuran, chromium(III) 2-ethylhexanoate, chromium(III) acetate, chromium(III) butyrate, chromium(III) pentanoate, chromium( III) laurate, chromium(III) tris(2,2,6,6-tetramethyl-3.5-heptanedionate), and chromium(III) stearate.
(19) In (17) or (18) above, the present invention provides a catalyst composition, wherein the co-catalyst is at least one selected from the group consisting of compounds represented by Formula 7 to Formula 10 below.

   [Formula 7] - [Al(R¹³)-O]ₐ-

   In Formula 7 above, R¹³ is each independently a halogen group, a hydrocarbyl group having 1 to 20 carbon atoms, or a hydrocarbyl group having 1 to 20 carbon atoms substituted with a halogen group, and a is an integer of 2 or greater, and

   [Formula 8] E(R¹⁴)₃

   in Formula 8 above, E is aluminum or boron, and R¹⁴ is each independently hydrogen, a halogen group, a hydrocarbyl group having 1 to 20 carbon atoms, or a hydrocarbyl group having 1 to 20 carbon atoms substituted with a halogen group, and

   [Formula 9] [L-H]⁺[G(Y)₄]⁻

   [Formula 10] [L]⁺[G(Y)₄]⁻

   in Formulas 9 and 10 above, L is a neutral or cationic Lewis acid, [L-H]⁺ is a Bronsted acid, G is a Group 13 element, and Y is each independently a substituted or unsubstituted alkyl group having 1 to 20 carbon atoms or a substituted or unsubstituted aryl group having 6 to 20 carbon atoms, where when the alkyl group or aryl group is substituted, the substituent is a halogen group, a hydrocarbyl group having 1 to 20 carbon atoms, an alkoxy group with 1 to 20 carbon atoms, or an aryloxy group with 6 to 20 carbon atoms.
(20) The present invention provides a method for manufacturing a linear alpha-olefin, the method including a step (S10) of oligomerizing ethylene in the presence of the catalyst composition according to any one among (17) to (19) above.
(21) In (20) above, the present invention provides a method for manufacturing a linear alpha-olefin, wherein the linear alpha-olefin is 1-hexene, 1-octene, or a mixture thereof.

### ADVANTAGEOUS EFFECTS

When ethylene oligomerization is performed using an organic chromium compound including a ligand compound of the present invention and a catalyst composition, it is possible to manufacture linear alpha-olefins with high 1-hexene and 1-octene selectivity while having excellent productivity due to high catalytic activity.

### MODE FOR CARRYING OUT THE INVENTION

Hereinafter, the present invention will be described in more detail to facilitate understanding of the present invention.

It will be understood that words or terms used in the description and claims of the present invention shall not be construed as being limited to having the meaning defined in commonly used dictionaries. It will be further understood that the words or terms should be interpreted as having meanings that are consistent with their meanings in the context of the relevant art and the technical idea of the invention, based on the principle that an inventor may properly define the meaning of the words or terms to best explain the invention.

### Ligand compound

The present invention provides a ligand compound applicable to a catalyst used in an ethylene oligomerization reaction. When the ligand compound is applied to an ethylene oligomerization reaction, specifically a catalyst composition for forming a linear alpha-olefin, the selectivity with respect to linear alpha-olefins is high while excellent catalytic activity is exhibited, and particularly, when compared with a catalyst including a symmetrical ligand compound as well as a typical PNP-based catalyst, the production amount of solid polyethylene is small even under the same reaction condition, so that linear alpha-olefins may be more efficiently prepared.

According to an embodiment of the present invention, an organic chromium compound to which the ligand compound is coordinated may be utilized for manufacturing a linear alpha-olefin using ethylene, and an oligomerization reaction proceeds in a reaction under the ethylene condition, so that an alpha-olefin in a liquid form, specifically, 1-hexene or 1-octene in a liquid form, may be formed with high selectivity. This is because selectivity with respect to alpha-olefins of a certain length increases through a transition state that forms metallacycles in the oligomerization reaction of ethylene.

According to an embodiment of the present invention, the ligand compound includes a diphosphino aminyl moiety, wherein an aryl having a specific substituent is connected to the end of the diphosphino aminyl moiety, and thus, may have a form that is capable of serving as a strong electron donating group itself. Due to the structural feature, the ligand compound may be applied to an oligomerization catalyst system for ethylene and exhibit high activity, and particularly, may exhibit high selectivity with respect to 1-hexene, 1-octene, or the like. This may be due to an interaction between adjacent chromium activity points, and particularly, when an aryl substituted with a specific substituent is connected to a phosphorus (P) atom of diphosphinoaminyl, the electron density is increased in the phosphorus (P) atom and a nitrogen (N) atom included in the diphosphinoaminyl, and the electrical and steric properties of the entire ligand compound change. Accordingly, since there is a change in the bond between a ligand and a chromium atom, the structure of a catalyst may become more stable, and compared to a typical metallacycloheptane or metallacyclonane form, it is possible to form alpha-olefins with higher activity and selectivity by changing the energy (activation energy) in the transition state, and to further reduce the amount of by-products of high-molecular weight solid alpha-olefins and the like, such as polyethylene wax (PE wax).

According to an embodiment of the present invention, the ligand compound is characterized in that phenyl positioned at the end of the diphosphino aminyl moiety has, as a substituent, an alkyl group of a specific carbon number, or a silyl group substituted with an alkyl group of a specific carbon number, at a meta position and a para position, respectively, in an asymmetric form. Considering the three-dimensional structure of the ligand compound itself and the structure of the catalyst bonded to chromium, the substituent substituted at the meta position of the phenyl causes relatively greater steric hindrance than the substituent substituted at the para position thereof. Therefore, the substituent substituted at the meta position of the phenyl may improve catalyst stability and simultaneously, may control the structure through steric hindrance, thereby further improving selectivity with respect to 1-hexene and 1-octene. However, if the steric hindrance is too large, the access of ethylene to the catalyst is prevented, resulting in degrading the activity of the catalyst, so that it is important to control the steric hindrance caused by the ligand compound through controlling the substituent substituted at the meta position of the phenyl. Meanwhile, since the substituent substituted at the para position of the phenyl causes less steric hindrance than the substituent substituted at the meta position of the phenyl, the access of ethylene to the catalyst is facilitated, resulting improving the activity of the catalyst, but the stability of the catalyst may be degraded due to rotation of the bond of nitrogen and phosphorus and the bond of nitrogen and carbon, and high-molecular weight by-products may be generated due to overreaction of ethylene, so that it is also important to control steric hindrance caused by the ligand compound through controlling the substituent substituted at the para position of the phenyl. The ligand compound according to the present invention simultaneously adjusts the substituents substituted at the meta position and the para position of the phenyl, thereby controlling steric hindrance caused by the ligand compound, and at the same time, lowers the possibility of rotation of the nitrogen-phosphorus bond, thereby preventing the ligand compound from dissociating from the catalyst, so that it is possible to manufacture a chromium catalyst having high stability and excellent activity and selectivity.

According to an embodiment of the present invention, in the ligand compound, a nitrogen atom to which two phosphorus atoms are bonded is bonded to a bulky substituent such as a cycloalkyl group or phenyl group, wherein the bulky substituent bonded to nitrogen may prevent the bond of nitrogen and phosphorus from rotating, so that the stability and the activity of the catalyst may be further improved. At this time, the activity, stability, and selectivity of the catalyst change according to the steric properties of the substituent entity bonded to the nitrogen atom. If the steric strain of the substituent bonded to the nitrogen atom is too high, there is a problem in that ligand synthesis and formation of a metal complex compound become difficult, and the generated complex compound becomes unstable. In addition, if the steric strain of the substituent bonded to the nitrogen atom is too high, there is a problem in that the access to raw materials such as ethylene become difficult, thereby degrading the activity of the catalyst. In addition, if the steric strain of the substituent bonded to the nitrogen atom is too low, it is not possible to prevent the rotation of the bond of the nitrogen atom and the phosphorus atom, and to protect a metal center atom, thereby causing the activity and stability of the catalyst to be degraded. That is, if the steric strain of the substituent bonded to the nitrogen atom is either too high or too low, the activity of the catalyst is lowered, and the stability thereof is degraded, so that there is a problem in which the amount of generated by-products such as polyethylene wax will increase. Therefore, it is very important to select a substituent bonded to the nitrogen atom that has a suitable level of steric strain with respect to a substituent bonded to the phosphorus atom. In the ligand compound according to the present invention, the steric strain around a P-N-P functional group increases due to the introduction of a substituent at a meta position of a phenyl group bonded to a phosphorus atom, and therefore, by introducing a substituent represented by R⁵ of Formula 1 in order to prevent the steric strain of a substituent bonded to a nitrogen atom from increasing significantly, it is possible to improve the yield and selectivity during an oligomerization reaction of ethylene by using a catalyst composition including the same. Particularly, when a substituent in the form of a secondary alkyl group is introduced as the substituent represented by R⁵ of Formula 1, the efficiency is further improved, and a substituent in the form of a primary alkyl group may form a suitable steric strain by introducing an aryl group such as a phenyl group at positions of carbon number 1 and carbon number 2 to compensate for a low steric strain, so that yield and selectivity may be improved.

According to an embodiment of the present disclosure, the ligand compound may be represented by Formula 1 below.

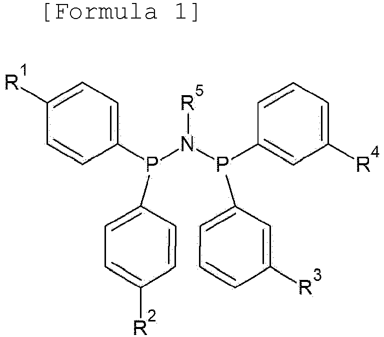

In Formula 1 above, R¹ to R⁴ are each independently an alkyl group having 5 to 20 carbon atoms, a cycloalkyl group having 5 to 20 carbon atoms, an alkoxyalkyl group having 6 to 20 carbon atoms, an arylalkoxyalkyl group having 10 to 30 carbon atoms, or a trialkylsilyl group, wherein the alkyl groups of the trialkylsilyl group are each independently an alkyl group having 1 to 10 carbon atoms, and R⁵ is an alkyl group having 1 to 20 carbon atoms, an alkyl group having 1 to 20 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.

In the present invention, "alkyl group" may refer to a linear or branched hydrocarbon moiety, and a specific example thereof may be, depending on the number of carbon atoms defined, a methyl group, an ethyl group, an n-propyl group, an isopropyl group, an n-butyl group, an isobutyl group, a t-butyl group, an n-pentyl group, an isopentyl group, a hexyl group, or the like.

In the present invention, "cycloalkyl group" may refer to a cyclic hydrocarbon moiety, and a specific example thereof may be, depending on the number of carbon atoms defined, a cyclopentyl group, a cyclohexyl group, a cycloheptyl group, a cyclooctyl group, or the like.

In the present invention, "alkoxyalkyl group" may refer to an alkyl group substituted with an alkoxy group, and the carbon number of the alkoxyalkyl group may refer to the sum of the carbon number of the alkoxy group and the carbon number of the alkyl group.

In the present invention, "arylalkoxyalkyl group" may refer to an alkyl group substituted with an alkoxy group including an aryl group as a substituent, and the carbon number of the arylalkoxyalkyl group may refer to the sum of the carbon number of the alkoxy group and the carbon number of the alkyl group.

In the present invention, "trialkylsilyl group" is represented by -SiR₃, and refers a substituent in which each R is independently an alkyl group, and the carbon number of the trialkylsilyl group may refer to the sum of the carbon numbers of all of R.

In the present invention, unless otherwise specified, "aryl" refers to an arbitrarily substituted benzene ring, or to a ring system which may be formed by fusing one or more arbitrary substituents. An example of the arbitrary substituent includes a substituted alkyl group having 1 to 2 carbon atoms, a substituted alkenyl group having 2 to 3 carbon atoms, a substituted alkynyl group having 2 to 3 carbon atoms, a heteroaryl group, a heterocyclic group, an aryl group, an alkoxy arbitrarily having 1 to 3 fluorine substituents, an aryloxy, an aralkoxy, an acyl, an aroyl, a heteroaroyl, an acyloxy, an aroyloxy, a heteroaroyloxy, a sulfanyl, a sulfinyl, a sulfonyl, an aminosulfonyl, a sulfonylamino, a carboxyamide, an aminocarbonyl, a carboxy, an oxo, a hydroxy, a mercapto, an amino, a nitro, a cyano, a halogen or an ureido. Such a ring or ring system may be arbitrarily fused to an aryl ring arbitrarily having one or more substituents (e.g., a benzene ring), a carbocyclic ring, or a heterocyclic ring. It may include, but is not limited to, phenyl, naphthyl, tetrahydronaphthyl, biphenyl, indanyl, anthracyl, or phenanthryl, and a substituted derivative thereof.

According to an embodiment of the present invention, R¹ to R⁴ may each independently be an alkyl group having 8 to 12 carbon atoms, a tripropylsilyl group, or a tributylsilyl group, and as a specific example, R¹ to R⁴ may each independently be a tripropylsilyl group, a tributylsilyl group, or an n-decyl group. That is, in the ligand compound, phenyl positioned at the end of a diphosphino aminyl moiety has, as a substituent, an alkyl group having 10 carbon atoms, or a silyl group substituted with an alkyl group having 3 or 4 carbon atoms at a meta position.

According to an embodiment of the present invention, in the ligand compound, i) R¹ and R² may be the same as each other, and R³ and R⁴ may be the same as each other, ii) R¹ and R³ may be the same as each other, and R² and R⁴ may be the same as each other, iii) R¹ to R⁴ may be the same as each other.

According to an embodiment of the present invention, R⁵ may be an alkyl group having 3 to 5 carbon atoms, an alkyl group having 1 to 5 carbon atoms substituted with an aryl group having 6 to 10 carbon atoms, a cycloalkyl group having 5 to 8 carbon atoms, or a cycloalkyl group having 5 to 8 carbon atoms fused with an aryl group having 6 to 10 carbon atoms.

Hereinafter, specific examples of the ligand compound represented by Formula 1 above according to the present invention have been distinguished and described as ligand compounds represented by Formulas 2 to 6, but this is only for convenience of classification, and each of the ligand compounds represented by Formulas 2 to 6 is a specific example of the ligand compound represented by Formula 1 above, which all exhibit the same effect.

According to an embodiment of the present disclosure, the ligand compound represented by Formula 1 above may be represented by Formula 2 below.

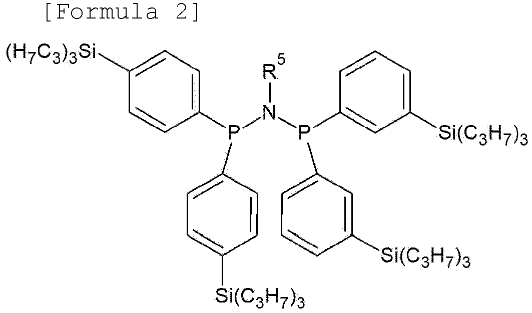

In Formula 2 above, R⁵ is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.

According to an embodiment of the present invention, in Formula 2 above, "C₃H₇" may be an n-propyl group.

According to an embodiment of the present invention, the ligand compound represented by Formula 2 above may be one selected from the group consisting of ligand compounds represented by Formula 2-1 to Formula 2-10 below.

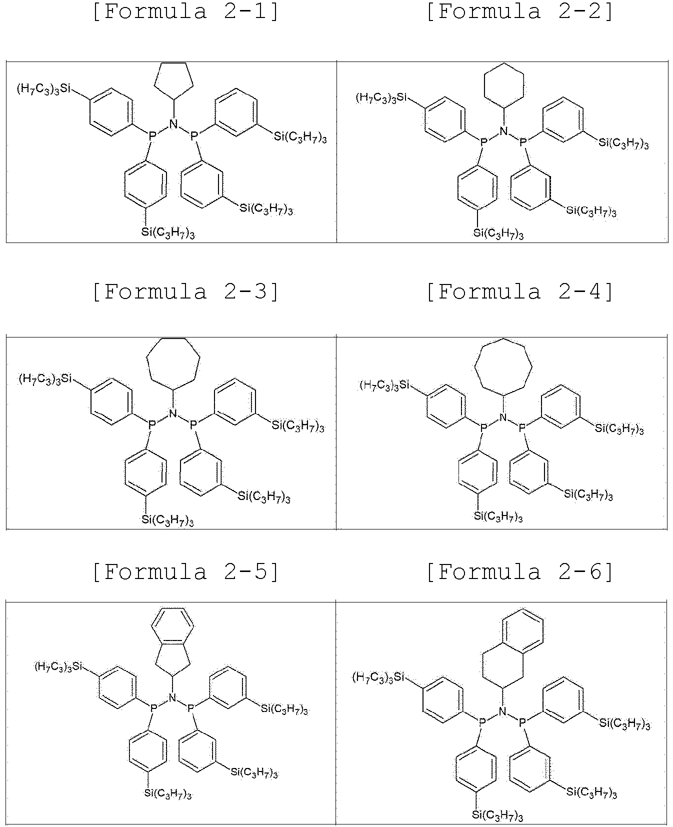

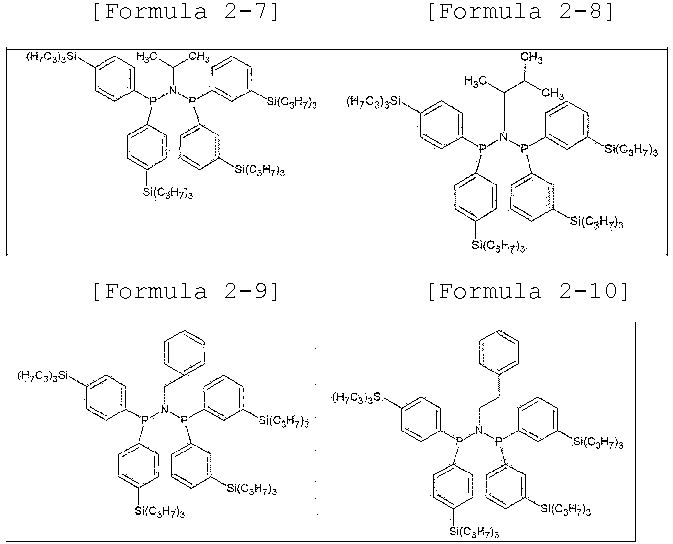

According to an embodiment of the present disclosure, the ligand compound represented by Formula 1 above may be represented by Formula 3 below.

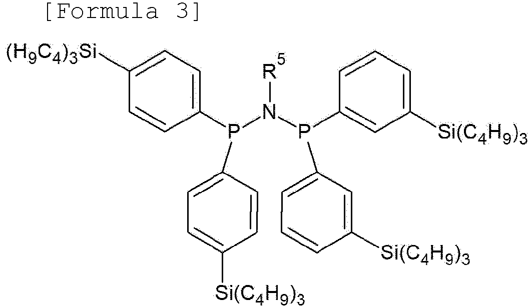

In Formula 3 above, R⁵ is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.

According to an embodiment of the present invention, in Formula 3 above, "C₄H₉" may be an n-butyl group.

According to an embodiment of the present invention, the ligand compound represented by Formula 3 above may be one selected from the group consisting of ligand compounds represented by Formula 3-1 to Formula 3-10 below.

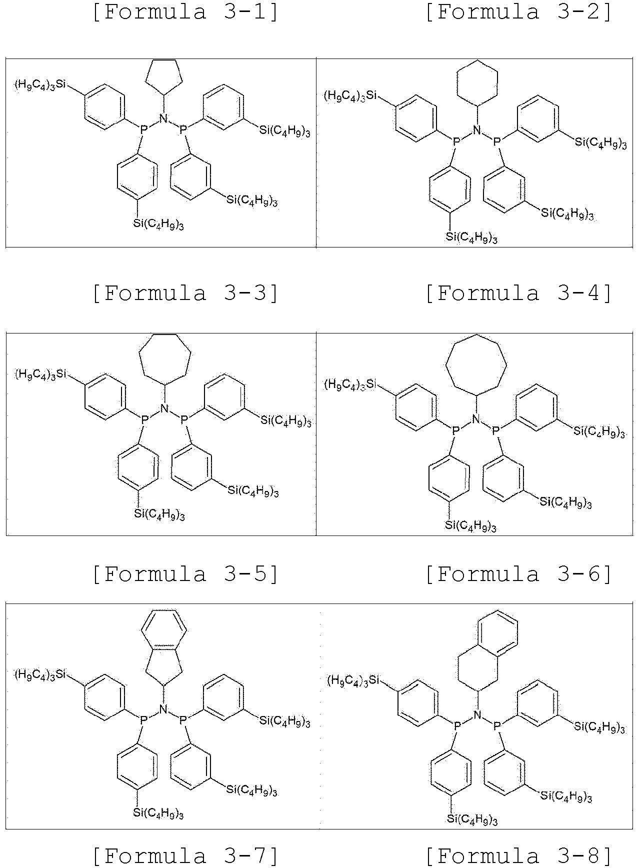

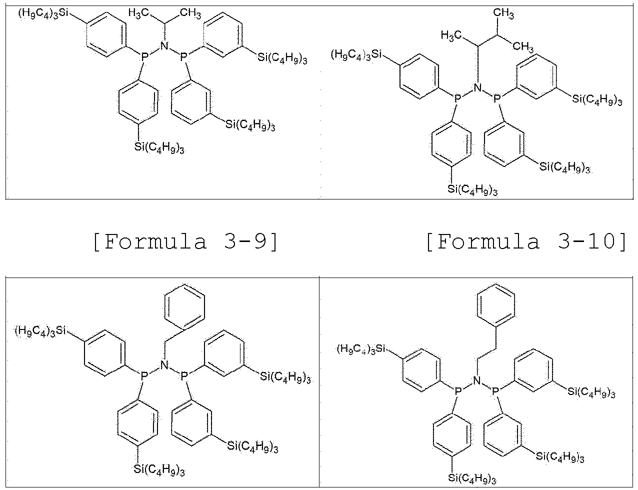

According to an embodiment of the present disclosure, the ligand compound represented by Formula 1 above may be represented by Formula 4 below.

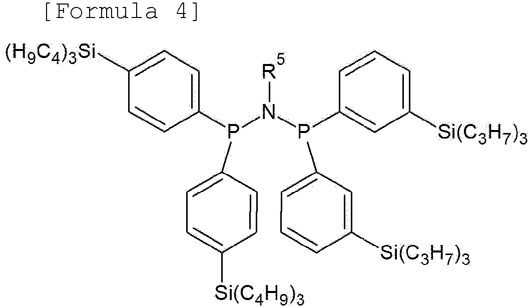

In Formula 4 above, 5 is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.

According to an embodiment of the present invention, in Formula 4 above, "C₃H₇" may be an n-propyl group, and "C₄H₉" may be an n-butyl group.

According to an embodiment of the present invention, the ligand compound represented by Formula 4 above may be one selected from the group consisting of ligand compounds represented by Formula 4-1 to Formula 4-10 below.

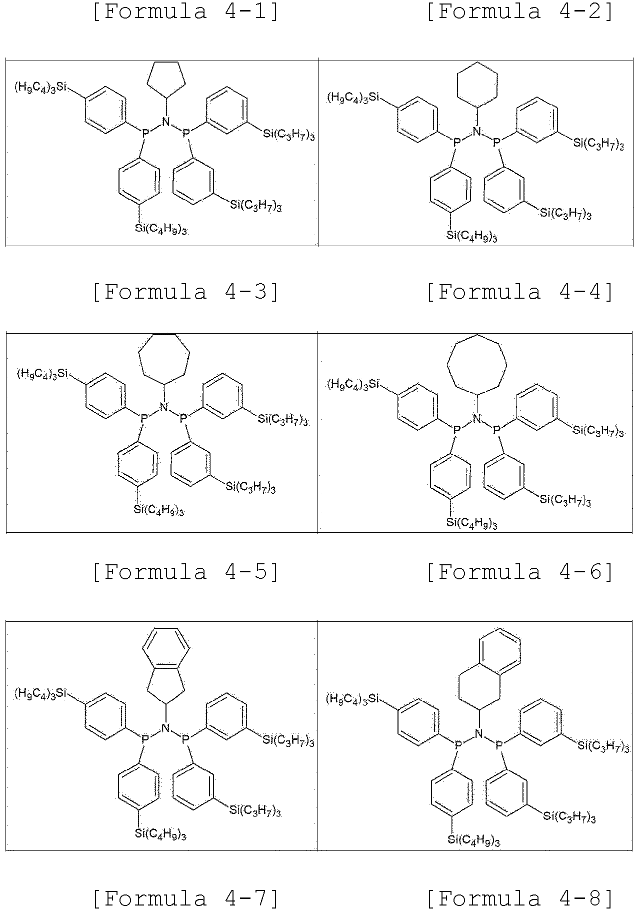

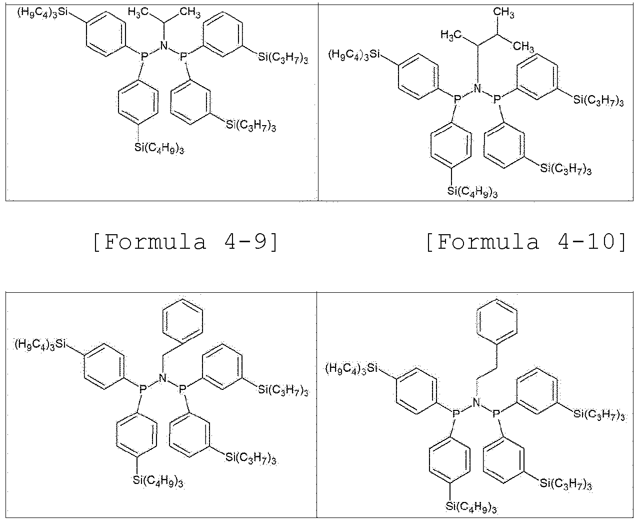

According to an embodiment of the present disclosure, the ligand compound represented by Formula 1 above may be represented by Formula 5 below.

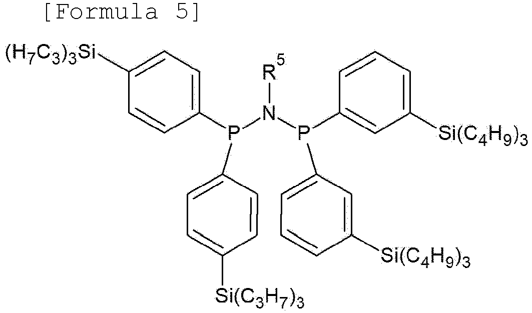

In Formula 5 above, R⁵ is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.

According to an embodiment of the present invention, in Formula 5 above, "C₃H₇" may be an n-propyl group, and "C₄H₉" may be an n-butyl group.

According to an embodiment of the present invention, the ligand compound represented by Formula 5 above may be one selected from the group consisting of ligand compounds represented by Formula 5-1 to Formula 5-10 below.

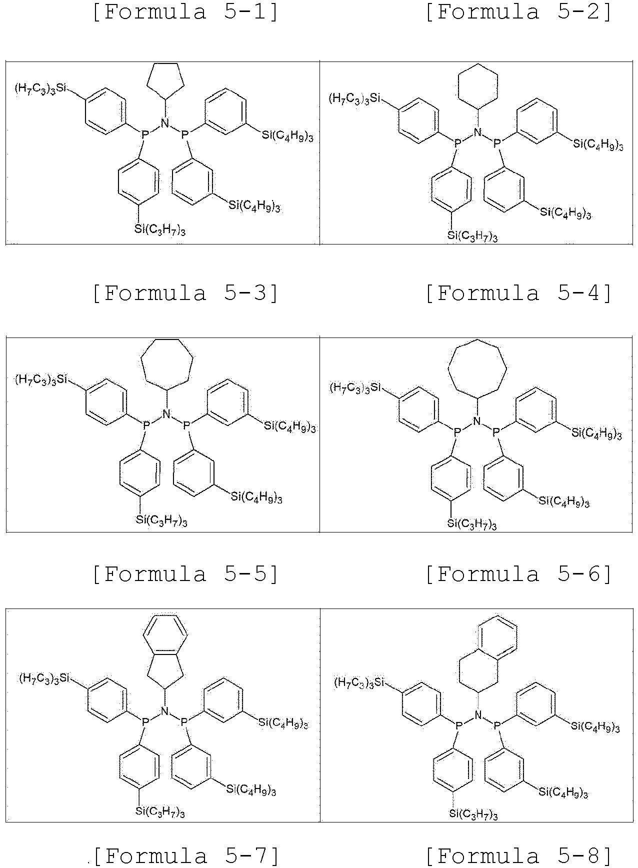

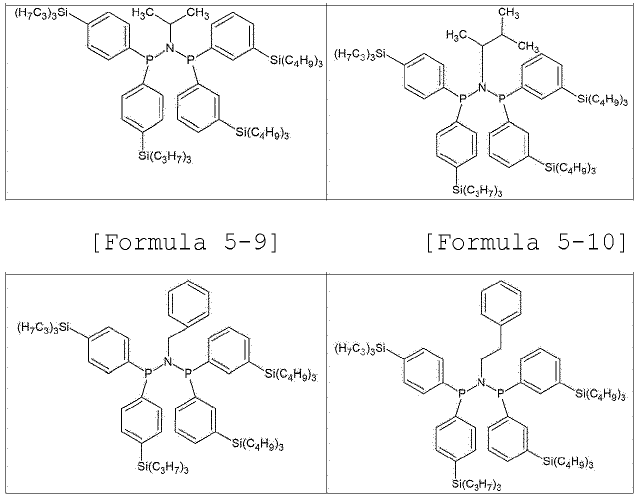

According to an embodiment of the present disclosure, the ligand compound represented by Formula 1 above may be represented by Formula 6 below.

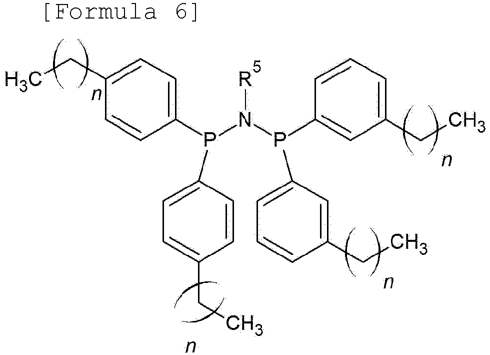

In Formula 6 above, R⁵ is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms, and n is an integer selected from 4 to 14.

According to an embodiment of the present invention, the ligand compound represented by Formula 6 above may be one selected from the group consisting of ligand compounds represented by Formula 6-1 to Formula 6-10 below.

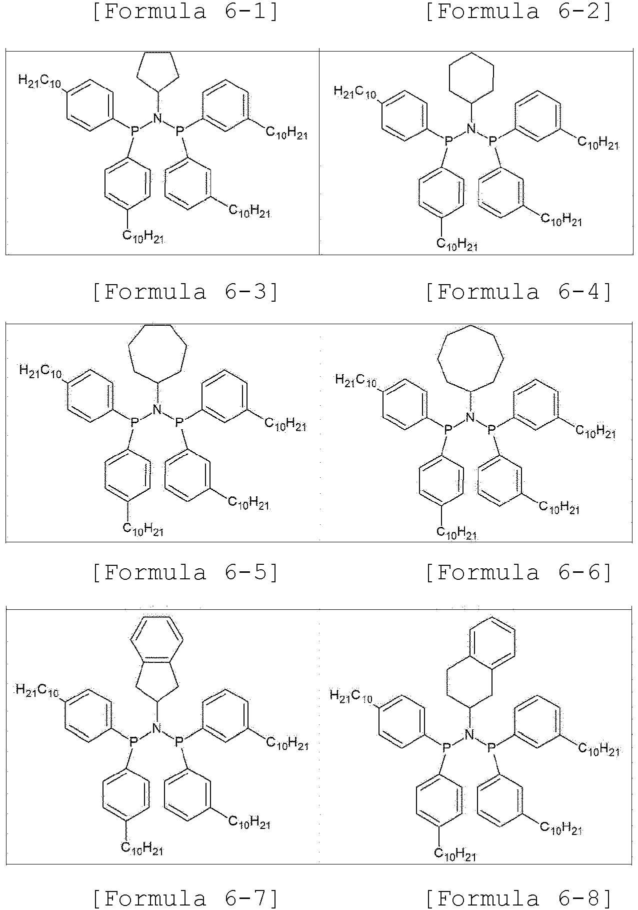

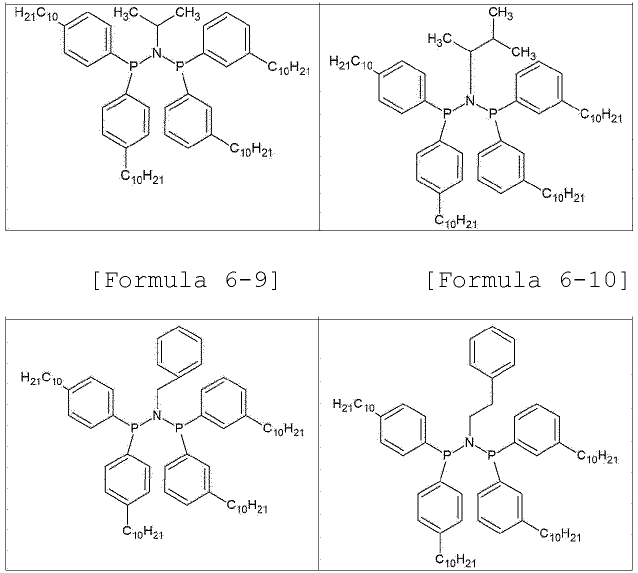

According to an embodiment of the present invention, in Formulas 6-1 to 6-10, "C₁₀H₂₁" may be an n-decyl group.

According to an embodiment of the present invention, in addition to the above-described embodiments, the ligand compound may be implemented in various combinations within a range that satisfies the above-described conditions, and any compound represented by Formula 1 may be applied as the ligand compound of the present invention.

### Organic chromium compound and catalyst composition

The present invention provides an organic chromium compound including the ligand compound represented by Formula 1 above and chromium (Cr) coordinated to the ligand compound.

According to an embodiment of the present invention, the organic chromium compound is a chromium complex compound of the ligand compound, in which chromium of a chromium source may have a form in which at least one unshared electron pair of N and two Ps has a coordinated bond in the ligand compound represented by Formula 1. That is, it is a structure in which a phosphorus atom or a nitrogen atom of a diphosphino aminyl moiety provides an unshared electron pair to a chromium atom, and particularly, a bidentated state in which two pairs of unshared electron pairs are coordinated may be preferred. The organic chromium compound may be applied to a catalyst system for an oligomerization reaction of ethylene and exhibit excellent catalytic activity and high selectivity with respect to 1-hexene or 1-octene.

In the present invention, "catalyst composition" may refer to one in a state in which three components including a chromium source, a ligand compound, and a co-catalyst, or two components of a transition metal compound and a co-catalyst are added simultaneously or in an arbitrary order to be obtained as an active catalyst composition. Here, the catalyst composition may also be referred to as a catalyst system, and in the present invention, the catalyst composition and catalyst system represent the same meaning. The three or two components of the catalyst composition may be added in the presence or absence of a solvent and a monomer, and may be used in a supported or non-supported state.

The present invention provides a catalyst composition including the above-described ligand compound, chromium, and a co-catalyst. As described above, the ligand compound represented by Formula 1 above and chromium may be coordinated to form an organic chromium compound. That is, the catalyst system may be a three-component catalyst system including chromium, the ligand compound represented by Formula 1 above, and a co-catalyst, or a two-component catalyst system including the organic chromium compound and a co-catalyst. As a specific example, the catalyst composition may include the ligand compound, an organic chromium compound including chromium coordinated to the ligand compound, and a co-catalyst. In addition, the catalyst composition may include a chromium compound in which some components of the co-catalyst are bonded to the organic chromium compound.

According to an embodiment of the present invention, the chromium may be derived from a chromium source, and the chromium source may be an organic or inorganic chromium compound in which the oxidation state of chromium is about 0 to about 6. As a specific example, the chromium source may be a chromium metal, or a compound in which any organic or inorganic radical is bonded to chromium. Here, the organic radical may be an alkyl radical, an alkoxy radical, an ester radical, a ketone radical, an amido radical, a carboxylate radical, or the like having 1 to 20 carbon atoms per radical, and the inorganic radical may be a halide, a sulfate, an oxide, or the like.

According to an embodiment of the present invention, the chromium source is a compound which exhibits high activity in oligomerization of olefins, and which is easy to use and obtain, and may be at least one compound selected from the group consisting of chromium(III) acetylacetonate, chromium(III) chloride tetrahydrofuran, chromium(III) 2-ethylhexanoate, chromium(III) acetate, chromium(III) butyrate, chromium(III) pentanoate, chromium( III) laurate, chromium(III) tris(2,2,6,6-tetramethyl-3.5-heptanedionate), and chromium(III) stearate.

According to an embodiment of the present invention, the co-catalyst may be at least one selected from the group consisting of compounds represented by Formula 7 to Formula 10 below.

[Formula 7] -[Al(R¹³)-O]ₐ-

In Formula 7 above, R¹³ is each independently a halogen group, a hydrocarbyl group having 1 to 20 carbon atoms, or a hydrocarbyl group having 1 to 20 carbon atoms substituted with a halogen group, and a is an integer of 2 or greater.

[Formula 8] E(R¹⁴)₃

In Formula 8 above, E is aluminum or boron, and R¹⁴ is each independently hydrogen, a halogen group, a hydrocarbyl group having 1 to 20 carbon atoms, or a hydrocarbyl group having 1 to 20 carbon atoms substituted with a halogen group.

[Formula 9] [L-H]⁺[G(Y)₄]⁻

[Formula 10] [L]⁺[G(Y)₄]⁻

In Formulas 9 and 10 above, L is a neutral or cationic Lewis acid, [L-H]⁺ is a Bronsted acid, G is a Group 13 element, and Y is each independently a substituted or unsubstituted alkyl group having 1 to 20 carbon atoms or a substituted or unsubstituted aryl group having 6 to 20 carbon atoms, where when the alkyl group or aryl group is substituted, the substituent is a halogen group, a hydrocarbyl group having 1 to 20 carbon atoms, an alkoxy group with 1 to 20 carbon atoms, or an aryloxy group with 6 to 20 carbon atoms.

According to an embodiment of the present invention, the catalyst composition may be manufactured by a plurality of methods. As a specific example, first, the catalyst composition may be manufactured by contacting the organic chromium compound with a compound represented by Formula 7 or Formula 8 above. Second, the catalyst composition may be manufactured by contacting the organic chromium compound with a compound represented by Formula 7 or Formula 8 above to obtain a mixture, and adding a compound represented by Formula 9 or Formula 10 above to the mixture. Third, the catalyst composition may be manufactured by contacting the organic chromium compound with a compound represented by Formula 9 or Formula 10 above. Fourth, the catalyst composition may be manufactured by contacting the organic chromium compound with a compound represented by Formula 9 or Formula 10 above to obtain a mixture, and adding a compound represented by Formula 7 or Formula 8 above to the mixture. Fifth, the catalyst composition may be manufactured by contacting the chromium source with a compound represented by Formula 9 or Formula 10 above to obtain a reactant, and contacting the reactant with the ligand compound.

According to an embodiment of the present invention, in the case of the first method or the third method among the above-described methods for manufacturing the catalyst composition, the molar ratio of the compound represented by Formula 7 or Formula 8 above with respect to the organic chromium compound may be 1:2 to 5,000 respectively, as a specific example, may be 1:100 to 3,000, and as a more specific example, may be 1:300 to 1,500, and within the above-described range, alkylation of the organic chromium compound may be completely achieved, so that the activity of the catalyst composition may be improved, and degradation in the activation of the alkylated organic chromium compound due to a side reaction with the remaining alkylating agent may be prevented, and at the same time, economic feasibility and purity of manufactured linear alpha-olefins may be improved.

According to an embodiment of the present invention, in the case of the second method among the above-described methods for manufacturing the catalyst composition, the molar ratio of the compound represented by Formula 9 or Formula 10 above with respect to the organic chromium compound may be 1:1 to 500 respectively, as a specific example, may be 1:1 to 50, and as a more specific example, may be 1:1 to 25, and within the above-described range, the amount of an activator is sufficient, so that activation of a metal compound may be completely achieved, thereby improving the activity of the catalyst composition, and the remaining activator is reduced to the minimum, so that economic feasibility and purity of manufactured linear alpha-olefins may be improved.

According to an embodiment of the present invention, the compound represented by Formula 7 above may be alkylaluminoxane, may be methylaluminoxane, ethylaluminoxane, isobutylaluminoxane, butylaluminoxane, or the like as a specific example, and may be methylaluminoxane as a more specific example.

According to an embodiment of the present invention, the compound represented by Formula 8 above may be trialkyl aluminum, dialkyl aluminum halide, alkyl aluminum dihalide, dialkyl aluminum hydride, alkyl aluminum dihydride, trialkyl boron, or the like. As a specific example, the compound represented by Formula 8 above may be trialkyl aluminum such as trimethyl aluminum, triethyl aluminum, triisobutyl aluminum, tripropyl aluminum, tributyl aluminum, triisopropyl aluminum, tri-s-butyl aluminum, tricyclopentyl aluminum, tripentyl aluminum, triisopentyl aluminum, trihexyl aluminum, trioctyl aluminum, ethyldimethyl aluminum, methyldiethyl aluminum, triphenyl aluminum, and tri-p-tolyl aluminum; dialkyl aluminum halide such as diethylaluminum chloride; dialkyl aluminum hydride such as diethyl aluminum hydride, di-n-propyl aluminum hydride, diisopropyl aluminum hydride, di-n-butyl aluminum hydride, dibutyl aluminum hydride, diisobutyl aluminum hydride (DIBAH), di-n -octyl aluminum hydride, diphenyl aluminum hydride, di-p-tolyl aluminum hydride, dibenzyl aluminum hydride, phenylethyl aluminum hydride, phenyl-n-propyl aluminum hydride, phenylisopropyl aluminum hydride, phenyl-n-butyl aluminum hydride, phenylisobutyl aluminum hydride, phenyl-n-octyl aluminum hydride, p-tolylethyl aluminum hydride, p-tolyl-n-propyl aluminum hydride, p-tolylisopropyl aluminum hydride, p-tolyl-n-butyl aluminum hydride, p-tolylisobutyl aluminum hydride, p-tolyl-n-octyl aluminum hydride, benzylethyl aluminum hydride, benzyl-n-propyl aluminum hydride, benzylisobutyl hydride, benzyl-n-butyl aluminum hydride, benzylisobutyl aluminum hydride, and benzyl-n-octyl aluminum hydride; alkyl aluminum dihydride such as n-propyl aluminum dihydride, isopropyl aluminum dihydride, n-butyl aluminum dihydride, isobutyl aluminum dihydride, and n-octyl aluminum dihydride; trialkyl boron such as trimethyl boron, triethyl boron, triisobutyl boron, tripropyl boron, and tributyl boron, or the like.

According to an embodiment of the present invention, the compound represented by Formula 9 or Formula 10 may be trimethylammonium tetraphenylborate, triethylammonium tetraphenylborate, tripropylammonium tetraphenylborate, tributylammonium tetraphenylborate, N,N-dimethylanilinium tetraphenylborate, N,N-diethylanilinium tetraphenylborate, trimethylammonium tetra(p-tolyl)borate, triethylammonium tetra(p-tolyl)borate, tripropylammonium tetra(p-tolyl) Borate, tributylammonium tetra (p-tolyl)borate, N,N-dimethylanilinium tetra(p-tolyl)borate, N,N-diethylanilinium tetra(p-tolyl) borate, trimethylammonium tetra(o,p-dimethylphenyl) borate, triethylammonium tetra(o,p-dimethylphenyl) borate, tripropylammonium tetra(o,p-dimethylphenyl) borate, tributylammonium tetra(o,p-dimethylphenyl)borate, N,N-dimethylanilinium tetra(o,p-dimethylphenyl)borate, N,N-diethylanilinium tetra(o,p-dimethylphenyl) borate, trimethylammonium tetrakis(p-trifluoromethylphenyl) borate, triethylammonium tetrakis(p-trifluoromethylphenyl) borate, tripropylammonium tetrakis(p-trifluoromethylphenyl) borate, tributylammonium tetrakis(p-trifluoromethylphenyl)borate, N,N-dimethylanilinium tetrakis(p-trifluoromethylphenyl)borate, N,N-diethylanilinium tetrakis(p-trifluoromethylphenyl)borate, trimethylammonium tetrakis(pentafluorophenyl)borate, triethylammonium tetrakis(pentafluorophenyl)borate, tripropylammonium tetrakis(pentafluorophenyl)borate, tributylammonium tetrakis(pentafluorophenyl)borate, N,N-dimethylanilinium tetrakis(pentafluorophenyl)norate, N,N-diethylanilinium tetrakis(pentafluorophenyl)borate, N,N-dioctadecylanilinium tetrakis(pentafluorophenyl)borate, trimethylphosphonium tetraphenylborate, triethylphosphonium tetraphenylborate, tripropylphosphonium tetraphenylborate, tributylphosphonium tetraphenylborate, trimethylcarbonium tetraphenylborate, triethylcarbonium tetraphenylborate, tripropylcarbonium tetraphenylborate, tributylcarbonium tetraphenylborate , trimethylammonium tetraphenylaluminate, triethylammonium tetraphenylaluminate, tripropylammonium tetraphenylaluminate, tributylammonium tetraphenylaluminate, trimethylammonium tetra(p-tolyl)aluminate, triethylammonium tetra(p-tolyl) aluminate, tripropylammonium tetra(p-tolyl)aluminate, tributylammonium tetra(p-tolyl)aluminate, or the like.

According to an embodiment of the present invention, the content ratio of components for forming the catalyst composition may be determined in consideration of catalyst activity and selectivity to a linear alpha-olefin. As a specific example, when the catalyst composition is a three-component catalyst composition, the molar ratio of the diphosphino aminyl moiety of the ligand compound:chromium source:co-catalyst may be controlled to about 1:1:1 to about 10:1:10,000, or to about 1:1:100 to about 5:1:3,000. In addition, when the catalyst composition is a two-component catalyst composition, the molar ratio of the diphosphino aminyl moiety of the ligand compound:co-catalyst may be controlled to about 1:1 to about 1:10,000, about 1:1 to about 1:5,000, or about 1:1 to about 1:3,000.

According to an embodiment of the present invention, when manufacturing the catalyst composition, as a reaction solvent, a hydrocarbon-based solvent such as pentane, hexane, and heptane, or an aromatic solvent such as benzene and toluene may be used.

According to an embodiment of the present invention, the components for forming the catalyst composition may be added simultaneously or in an arbitrary order in the presence or absence of a suitable solvent and a monomer, and act as an active catalyst composition. At this time, the suitable solvent may be heptane, toluene, cyclohexane, methylcyclohexane, 1-hexene, 1-octene, diethylether, tetrahydrofuran, acetonitrile, dichloromethane, chloroform, chlorobenzene, methanol, acetone, or the like.

According to an embodiment of the present invention, the organic chromium compound and the co-catalyst may be used in the form of being supported on a carrier, and at this time, the carrier may be silica or alumina.

According to an embodiment of the present invention, the catalyst composition may further include a carrier. As a specific example, the ligand compound, represented by Formula 1 above may be applied to an ethylene oligomerization reaction in the form of being supported on a carrier. The carrier may be a metal, a metal salt, or a metal oxide applied to a carrier catalyst, and as a specific example, the carrier may be silica, silica-alumina, silica-magnesia, or the like, and may include an oxide, carbonate, sulfate, or nitrate component of a metal, such as Na₂O, K₂CO₃, BaSO₄, and Mg(NO₃)₂.

According to an embodiment of the present invention, the catalyst composition may be used for a trimerization or tetramerization reaction of ethylene, and may produce 1-hexene or 1-octene due to the high selectivity described above.

### Ethylene oligomerization method

The present invention provides a linear alpha-olefin manufacturing method as a method for oligomerizing ethylene, wherein the method includes a step (S10) of oligomerizing ethylene in the presence of the catalytic composition.

In the present invention, "oligomerization" refers to polymerization of an olefin. Depending on the number of olefins polymerized, the "oligomerization" is called trimerization or tetramerization, which is collectively referred to as multimerization. Particularly, in the present specification, the "oligomerization" may refer to selectively manufacturing 1-hexene and 1-octene, which are main comonomers of LLDPE, from ethylene.

According to an embodiment of the present invention, the oligomerization reaction of ethylene may be a trimerization or tetramerization reaction of ethylene, and as a result of the reaction, 1-hexene or 1-octene is formed as reaction products, so that the linear alpha-olefin may be 1-hexene, 1-octene, or a mixture thereof.

According to an embodiment of the present invention, the oligomerization method of ethylene may be performed by using ethylene as a raw material and applying the previously described catalyst composition, a typical apparatus, and a contact technique. As a specific example, the oligomerization reaction of ethylene may be performed, in the presence or absence of an inert solvent, as a homogeneous liquid-phase reaction, a slurry reaction in which some or all of the catalyst composition is in an undissolved form, a bulk-phase reaction in which an alpha-olefin, which is a product, serves as a main medium, or a gas-phase reaction.

According to an embodiment of the present invention, the oligomerization reaction of ethylene may be performed in an inert solvent. As a specific example, the inert solvent may be benzene, toluene, xylene, cumene, chlorobenzene, dichlorobenzene, heptane, cyclohexane, methylcyclohexane, methylcyclopentane, n-hexane, 1-hexene, 1-octene, 2,2,4-trimethylpentane, or the like.

According to an embodiment of the present invention, the oligomerization reaction of ethylene may be performed at a temperature of about 0 °C to about 200 °C, about 0 °C to about 150 °C, about 30 °C to about 100 °C, or about 50 °C to about 100 °C. In addition, the reaction may be performed under a pressure of about 15 psig to about 3000 psig, about 15 psig to about 1500 psig, or about 15 psig to about 1,000 psig.

Hereinafter, embodiments of the present invention will be described in detail so that those skilled in the art may easily carry out the present invention. However, the present invention may be embodied in many different forms, and is not limited to the embodiments set forth herein.

### Synthesis Examples and Comparative Examples

In the following synthesis examples and comparative synthesis examples, -C₃H₇ is an n-propyl group, -C₄H₉ is an n-butyl group, and -C₁₃H₂₁ is an n-decyl group.

### Synthesis Example 1: Synthesis of ligand compound represented by Formula 2-1

0.94 g (11 mmol, 2.2 eq) of cyclopentylamine and 10 ml of dichloromethane were introduced into a dried flask under a nitrogen atmosphere and stirred. 2.66 g (5 mmol, 1 eq) of chlorobis(3-(tripropylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.0 g (5 mmol) of N-cycloheptyl-1,1-bis(3-(tripropylsilyl)phenyl)phosphanamine, which is Intermediate Compound 1, was obtained through column chromatography.

### <Intermediate Compound 1>

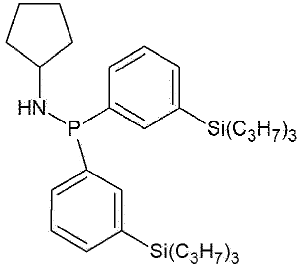

¹H NMR (500 MHz, DMSO) : δ 7.62(m, 2H), 7.49 (m, 2H), 7.37(m, 2H), 7.20(m, 2H), 2.65(m, 1H), 1.85(m, 4H), 1.77(m, 4H), 1.57(m, 1H), 1.52(m, 12H), 1.36(m, 12H), 0.98(m, 18H)

Subsequently, 2.9 g (5 mmol, 1 eq) of N-cyclopentyl-1,1-bis(3-(tripropylsilyl)phenyl)phosphanamine, which is Intermediate Compound 1 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.3 g (4 mmol, yield 80%) of N-(bis(3-(tripropylsilyl)phenyl)phosphaneyl)-N-cyclopentyl-1,1-bis(4-(tripropylsilyl)phenyl)phosphanamine, which is a compound represented by Formula 2-1, was obtained through column chromatography.

### <Ligand compound represented by Formula 2-1>

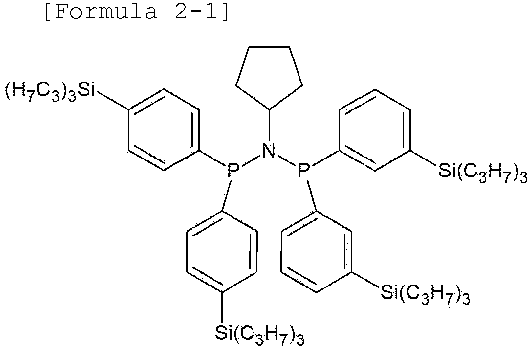

¹H NMR (500 MHz, DMSO) : δ 7.56-7.16 (m, 16H) , 2. 64 (m, 1H), 1.94-1.31(m, 56H), 0.96(m, 36H)

### Synthesis Example 2: Synthesis of ligand compound represented by Formula 2-2

1.09 g (11 mmol, 2.2 eq) of cyclohexylamine and 10 ml of dichloromethane were introduced into a dried flask under a nitrogen atmosphere and stirred. 2.66 g (5 mmol, 1 eq) of chlorobis(3-(tripropylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.0 g (4.75 mmol) of N-cyclohexyl-1,1-bis(3-(tripropylsilyl)phenyl)phosphanamine, which is Intermediate Compound 2, was obtained through column chromatography.

### <Intermediate Compound 2>

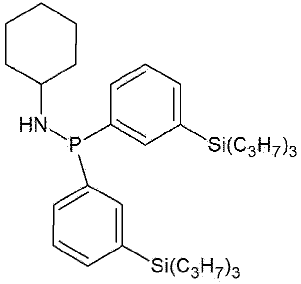

¹H NMR (500 MHz, DMSO): δ 7.50 (m, 2H), 7.40(m, 2H), 7.30(m, 2H), 7.10(m, 2H), 2.50(m, 1H), 1.70(m, 4H), 1.50(m, 1H), 1.40(m, 14H), 1.20(m, 16H), 0.90(m, 18H)

Subsequently, 3.0 g (5 mmol, 1 eq) of N-cyclohexyl-1,1-bis(3-(tripropylsilyl)phenyl)phosphanamine, which is Intermediate Compound 2 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.80 g (4.4 mmol, yield 88%) of N-(bis(3-(tripropylsilyl)phenyl)phosphaneyl)-N-cyclohexyl-1,1-bis(4-(tripropylsilyl)phenyl)phosphanamine, which is a compound represented by Formula 2-2, was obtained through column chromatography.

### <Ligand compound represented by Formula 2-2>

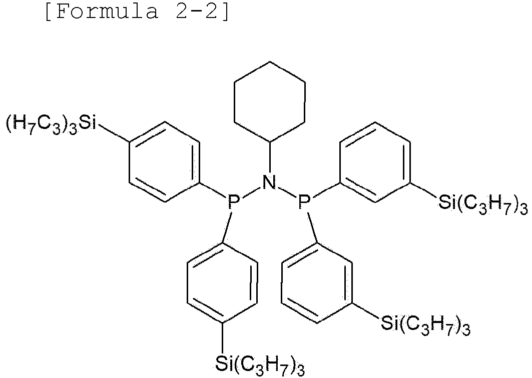

¹H NMR (500 MHz, DMSO): δ 7.55-7.13(m, 16H), 2.57 (m, 1H), 1.74(m, 4H), 1.46-1.43(m, 26H), 1.29-1.21(m, 28H), 0.94(m, 21H)

### Synthesis Example 3: Synthesis of ligand compound represented by Formula 2-3

1.24 g (11 mmol, 2.2 eq) of cycloheptylamine and 10 ml of dichloromethane were introduced into a dried flask under a nitrogen atmosphere and stirred. 2.66 g (5 mmol, 1 eq) of chlorobis(3-(tripropylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.0 g (5 mmol) of N-cycloheptyl-1,1-bis(3-(tripropylsilyl)phenyl)phosphanamine, which is Intermediate Compound 3, was obtained through column chromatography.

### <Intermediate Compound 3>

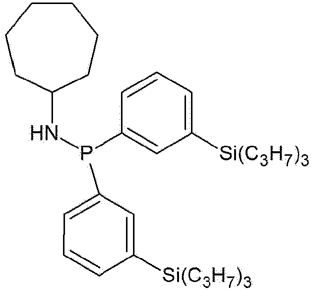

¹H NMR (500 MHz, C₆D₆) : δ 7.89(m, 2H), 7.54(m, 2H), 7.26(m, 2H), 3.25(m, 1H), 1.94-1.24(m, 24H), 0.99-0.88(m, 18H), 0.81-0.78(m, 12H)

Subsequently, 3.0 g (5 mmol, 1 eq) of N-cycloheptyl-1,1-bis(3-(tripropylsilyl)phenyl)phosphanamine, which is Intermediate Compound 3 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.4 g (4 mmol, yield 80%) of N-(bis(3-(tripropylsilyl)phenyl)phosphaneyl)-N-cycloheptyl-1,1-bis(4-(tripropylsilyl)phenyl)phosphanamine, which is a compound represented by Formula 2-3, was obtained through column chromatography.

### <Ligand compound represented by Formula 2-3>

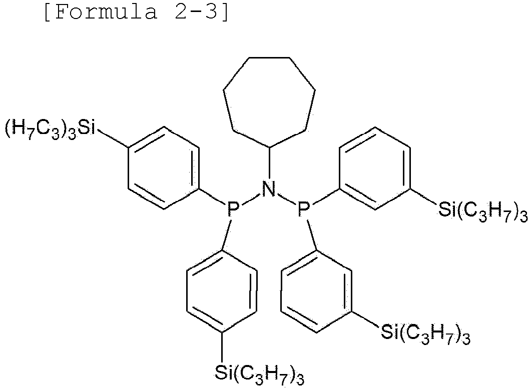

¹H NMR (500 MHz, C₆D₆): δ 7.90-7.10(m, 16H), 3.75(m, 1H), 1.64-1.24(m, 36H), 0.99-0.88(m, 36H), 0.81-0.78(m, 24H)

### Synthesis Example 4: Synthesis of ligand compound represented by Formula 2-4

1.40 g (11 mmol, 2.2 eq) of cyclooctylamine and 10 ml of dichloromethane were introduced into a dried flask under a nitrogen atmosphere and stirred. 2.66 g (5 mmol, 1 eq) of chlorobis(3-(tripropylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.12 g (5 mmol) of N-cyclooctyl-1,1-bis(3-(tripropylsilyl)phenyl)phosphanamine, which is Intermediate Compound 4, was obtained through column chromatography.

### <Intermediate Compound 4>

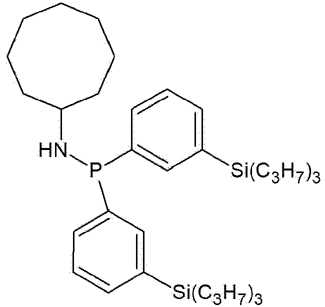

¹H NMR (500 MHz, DMSO): δ 7.55(m, 2H), 7.40(m, 2H), 7.33(m, 2H), 7.13(m, 2H), 2.43(m, 1H), 1.59(m, 4H), 1.50(m, 1H), 1.43(m, 12H), 1.32(m, 2H), 1.30(m, 4H), 1.29(m, 12H), 1.26(m, 4H), 0.94(m, 18H)

Subsequently, 3.12 g (5 mmol, 1 eq) of N-cyclooctyl-1,1-bis(3-(tripropylsilyl)phenyl)phosphanamine, which is Intermediate Compound 4 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.37 g (3.9 mmol, yield 78%) of N-(bis(3-(tripropylsilyl)phenyl)phosphaneyl)-N-cyclooctyl-1,1-bis(4-(tripropylsilyl)phenyl)phosphanamine, a compound represented by Formula 2-4, was obtained through column chromatography.

### <Ligand compound represented by Formula 2-4>

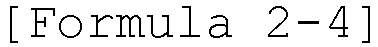

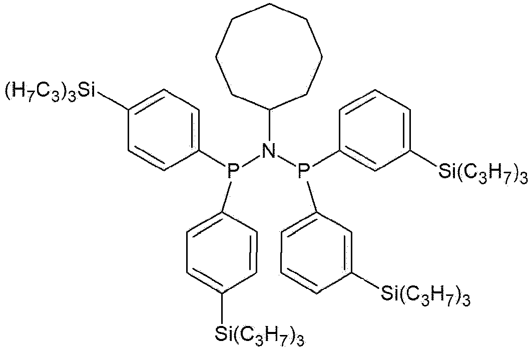

¹H NMR (500 MHz, DMSO): δ 7.55-7.13(m, 16H), 2.43(m, 1H), 1.60-1.43(m, 28H), 1.32-1.26(m, 34H), 0.94(m, 36H)

### Synthesis Example 5: Synthesis of ligand compound represented by Formula 2-5

1.46 g (11 mmol, 2.2 eq) of 2,3-dihydro-1H-inden-2-yl)amine and 10 ml of dichloromethane were introduced into a dried flask under a nitrogen atmosphere and stirred. 2.66 g (5 mmol, 1 eq) of chlorobis(3-(tripropylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.15 g (5 mmol) of N-(2,3-dihydro-1H-inden-2-yl)-1,1-bis(3-(tripropylsilyl)phenyl)phosphanamine, which is Intermediate Compound 5, was obtained through column chromatography.

### <Intermediate Compound 5>

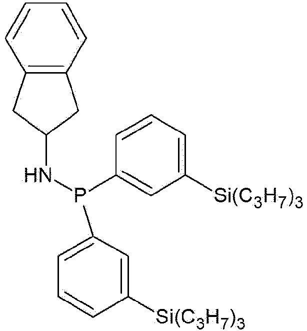

¹H NMR (500 MHz, DMSO) : δ 7.55 (m, 2H), 7.40(m, 2H), 7.33(m, 2H), 7.26(m, 2H), 7.13(m, 2H), 7.06 (m, 2H), 3.17(m, 1H), 3.13(m, 4H), 1.50(m, 1H), 1.43 (m, 12H), 1.29(m, 12H), 0.94(m, 18H)

Subsequently, 2.9 g (5 mmol, 1 eq) of N-(2,3-dihydro-1H-inden-2-yl)-1,1-bis(3-(tripropylsilyl)phenyl)phosphanamine, which is Intermediate Compound 5 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 5.01 g (4.45 mmol, yield 89%) of N-(bis(3-(tripropylsilyl)phenyl)phosphaneyl)-N-(2,3-dihydro-1H-inden-2-yl)-1,1-bis(4-(tripropylsilyl)phenyl)phosphanamine, a compound represented by Formula 2-5, was obtained through column chromatography.

### <Ligand compound represented by Formula 2-5>

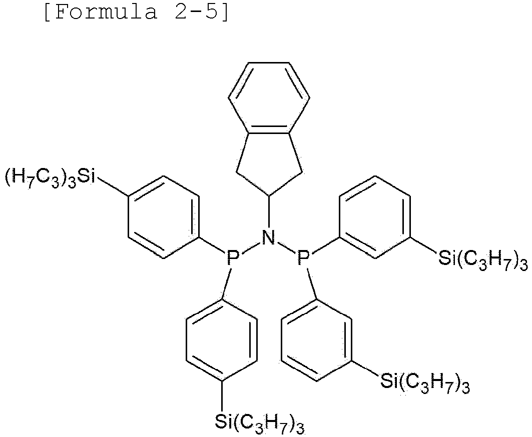

¹H NMR (500 MHz, DMSO): δ 7.65-7.06(m, 20H), 3.17-3.13(m, 5H), 1.43-1.29(m, 48H), 0.90(m, 36H)

### Synthesis Example 6: Synthesis of ligand compound represented by Formula 2-6

1.62 g (11 mmol, 2.2 eq) of 1,2,3,4-tetrahydronaphthalen-2-yl)amine and 10 ml of dichloromethane were introduced into a dried flask under a nitrogen atmosphere and stirred. 2.66 g (5 mmol, 1 eq) of chlorobis(3-(tripropylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.2 g (5 mmol) of N-(1,2,3,4-tetrahydronaphthalen-2-yl)-1,1-bis(3-(tripropylsilyl)phenyl)phosphanamine, which is Intermediate Compound 6, was obtained through column chromatography.

### <Intermediate Compound 6>

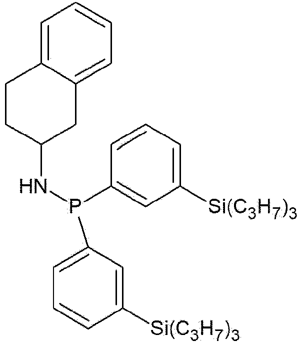

¹H NMR (500 MHz, DMSO): δ 7.55 (m, 2H), 7.40(m, 2H), 7.33(m, 2H), 7.13(m, 2H), 6.92 (m, 4H), 3.19 (m, 2H), 2.90(m, 2H), 2.73(m, 1H), 1.95(m, 2H), 1.50(m, 1H), 1.43 (m, 12H), 1.29(m, 12H), 0.94(m, 18H)

Subsequently, 3.2 g (5 mmol, 1 eq) of N-(1,2,3,4-tetrahydronaphthalen-2-yl)-1,1-bis(3-(tripropylsilyl)phenyl)phosphanamine, which is Intermediate Compound 6 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.3 g (3.75 mmol, yield 75%) of N-(bis(3-(tripropylsilyl)phenyl)phosphaneyl)-N-(1,2,3,4-tetrahydronaphthalen-2-yl)-1,1-bis(4-(tripropylsilyl)phenyl)phosphanamine, which is a compound represented by Formula 2-6, was obtained through column chromatography.

### <Ligand compound represented by Formula 2-6>

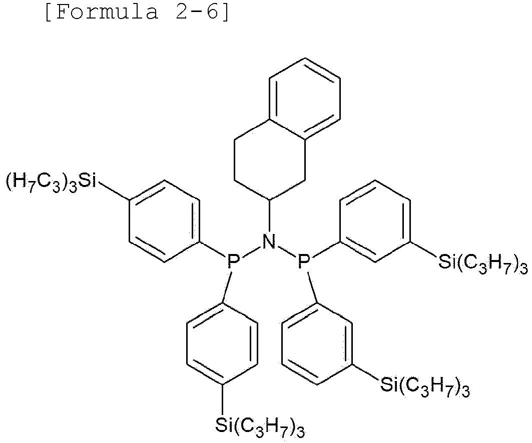

¹H NMR (500 MHz, DMSO): δ 7.70-6.92 (m, 20H), 3.19 (m, 2H), 2.90(m, 2H), 2.73(m, 1H), 1.95(m, 2H), 1.43 (m, 24H), 1.29(m, 24H), 0.89(m, 36H)

### Synthesis Example 7: Synthesis of ligand compound represented by Formula 2-7

0.65 g (11 mmol, 2.2 eq) of isopropylamine and 10 ml of dichloromethane were introduced into a dried flask under a nitrogen atmosphere and stirred. 2.66 g (5 mmol, 1 eq) of chlorobis(3-(tripropylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 2.78 g (5 mmol) of N-isopropyl-1,1-bis(3-(tripropylsilyl)phenyl)phosphanamine, which is Intermediate Compound 7, was obtained through column chromatography.

### <Intermediate Compound 7>

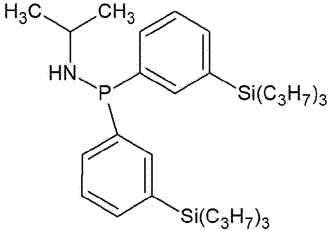

¹H NMR (500 MHz, DMSO): δ 7.55 (m, 2H), 7.40 (m, 2H), 7.33(m, 2H), 7.13(m, 2H), 2.83(m, 1H), 1.50(m, 1H), 1.43(m, 12H), 1.29(m, 12H), 1.06(m, 6H), 0.94(m, 18H)

Subsequently, 2.78 g (5 mmol, 1 eq) of N-isopropyl-1,1-bis(3-(tripropylsilyl)phenyl)phosphanamine, which is Intermediate Compound 7 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 3.26 g (3.1 mmol, yield 62%) of N-(bis(3-(tripropylsilyl)phenyl)phosphaneyl)-N-isopropyl-1,1-bis(4-(tripropylsilyl)phenyl)phosphanamine, which is a compound represented by Formula 2-7, was obtained through column chromatography.

### <Ligand compound represented by Formula 2-7>

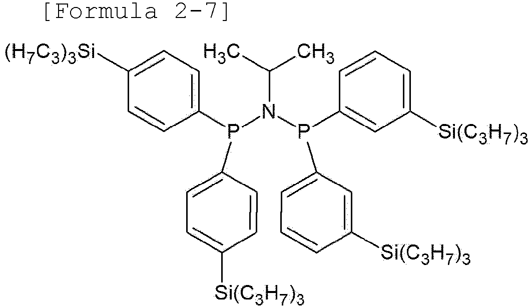

¹H NMR (500 MHz, DMSO): δ 7.70-7.13(m, 16H), 2.83 (m, 1H), 1.43-1.29(m, 48H), 1.06(m, 6H), 0.94(m, 36H)

### Synthesis Example 8: Synthesis of ligand compound represented by Formula 2-8

0.96 g (11 mmol, 2.2 eq) of 3-methylbutan-2-yl)amine and 10 ml of dichloromethane were introduced into a dried flask under a nitrogen atmosphere and stirred. 2.66 g (5 mmol, 1 eq) of chlorobis(3-(tripropylsilyl)phenyl)phosphane diluted in 10 ml of dichloromethane was slowly introduced into the flask. A reactant was stirred for 4 hours, and conversion was confirmed through ¹H NMR. After the reaction was completed solids were removed through a filter, the solvent was removed by decompression, and 2.9 g (5 mmol) of N-(3-methylbutan-2-yl)-1,1-bis(3-(tripropylsilyl)phenyl)phosphanamine, which is Intermediate Compound 8, was obtained through column chromatography.

### <Intermediate Compound 8>

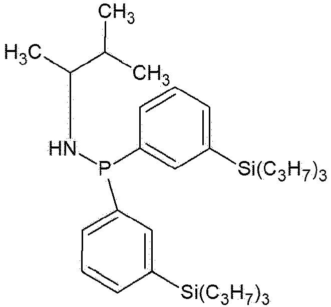

¹H NMR (500 MHz, DMSO): δ 7.55 (m, 2H), 7.40 (m, 2H), 7.33(m, 2H), 7.13(m, 2H), 2.53(m, 1H), 1.55(m, 1H), 1.50(m, 1H), 1.43(m, 12H), 1.29(m, 12H), 1.06(m, 3H), 0.94(m, 18H), 0.88(m, 6H)

Subsequently, 2.9 g (5 mmol, 1 eq) of N-(3-methylbutan-2-yl)-1,1-bis(3-(tripropylsilyl)phenyl)phosphanamine, which is Intermediate Compound 8 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 3.84 g (3.55 mmol, yield 71%) of N-(bis (3-(tripropylsilyl)phenyl)phosphaneyl)-N-(3-methylbutan-2-yl)-1,1-bis(4-(tripropylsilyl)phenyl)phosphanamine, which is a compound represented by Formula 2-8, was obtained through column chromatography.

### <Ligand compound represented by Formula 2-8>

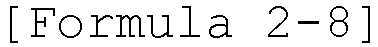

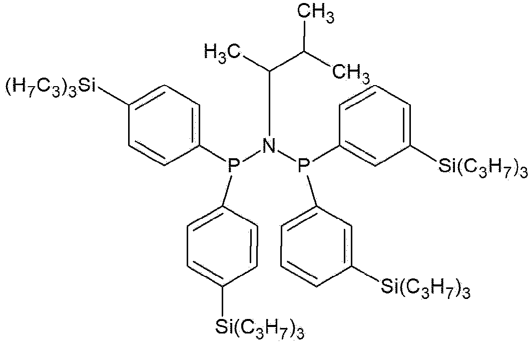

¹H NMR (500 MHz, DMSO): δ 7.51-7.02(m, 16H), 2.53(m, 1H), 1.55(m, 1H), 1.65-1.23(m, 48H), 1.06(m, 3H), 0.94(m, 36H), 0.88(m, 6H)

### Synthesis Example 9: Synthesis of ligand compound represented by Formula 2-9

1.18 g (11 mmol, 2.2 eq) of benzylamine and 10 ml of dichloromethane were introduced into a dried flask under a nitrogen atmosphere and stirred. 2.66 g (5 mmol, 1 eq) of chlorobis(3-(tripropylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.02 g (5 mmol) of N-benzyl-1,1-bis(3-(tripropylsilyl)phenyl)phosphanamine, which is Intermediate Compound 9, was obtained through column chromatography.

### <Intermediate Compound 9>

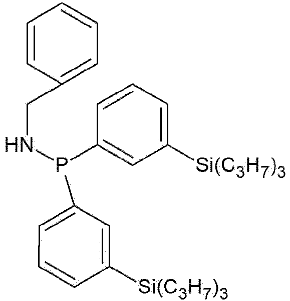

¹H NMR (500 MHz, DMSO): δ 7.55(m, 2H), 7.40 (m, 2H), 7.33(m, 2H), 7.31(m, 4H), 7.29(m, 1H), 7.13(m, 2H), 3.91(m, 2H), 2.30(m, 1H), 1.43(m, 12H), 1.29(m, 12H), 0.94(m, 18H)

Subsequently, 3.02 g (5 mmol, 1 eq) of N-benzyl-1,1-bis(3-(tripropylsilyl)phenyl)phosphanamine, which is Intermediate Compound 9 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.62 g (4.2 mmol, yield 84%) of N-(bis(3-(tripropylsilyl)phenyl)phosphaneyl)-N-benzyl-1,1-bis(4-(tripropylsilyl)phenyl)phosphanamine, which is a compound represented by Formula 2-9, was obtained through column chromatography.

### <Ligand compound represented by Formula 2-9>

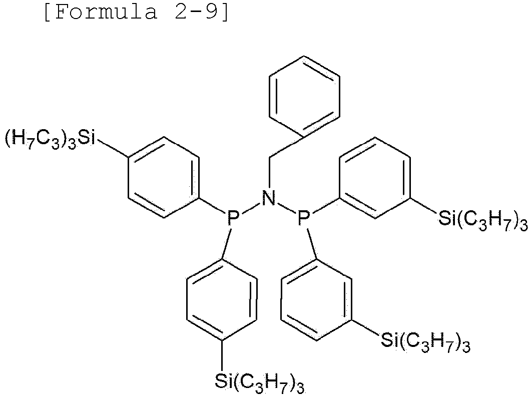

¹H NMR (500 MHz, DMSO): δ 7.72-7.00(m, 21H), 3.91 (m, 2H), 1.49-1.29(m, 48H), 0.94(m, 36H)

### Synthesis Example 10: Synthesis of ligand compound represented by Formula 2-10

1.33 g (11 mmol, 2.2 eq) of phenethylamine and 10 ml of dichloromethane were introduced into a dried flask under a nitrogen atmosphere and stirred. 2.66 g (5 mmol, 1 eq) of chlorobis(3-(tripropylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.09 g (5 mmol) of N-phenethyl-1,1-bis(3-(tripropylsilyl)phenyl)phosphanamine, which is Intermediate Compound 10, was obtained through column chromatography.

### <Intermediate Compound 10>

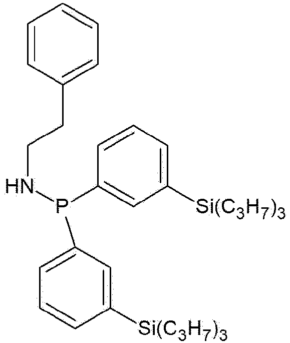

¹H NMR (500 MHz, DMSO): δ 7.55 (m, 2H), 7.40 (m, 2H), 7.33(m, 2H), 7.25(m, 2H), 7.24(m, 2H), 7.19(m, 1H), 7.13(m, 2H), 2.98(m, 2H), 2.83(m, 2H), 1.50(m, 1H), 1.43 (m, 12H), 1.29(m, 12H), 0.94(m, 18H)

Subsequently, 3.09 g (5 mmol, 1 eq) of N-phenethyl-1,1-bis(3-(tripropylsilyl)phenyl)phosphanamine, which is Intermediate Compound 10 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.63 g (4.15 mmol, yield 83%) of N-(bis(3-(tripropylsilyl)phenyl)phosphaneyl)-N-phenethyl-1,1-bis(4-(tripropylsilyl)phenyl)phosphanamine, which is a compound represented by Formula 2-10, was obtained through column chromatography.

### <Ligand compound represented by Formula 2-10>

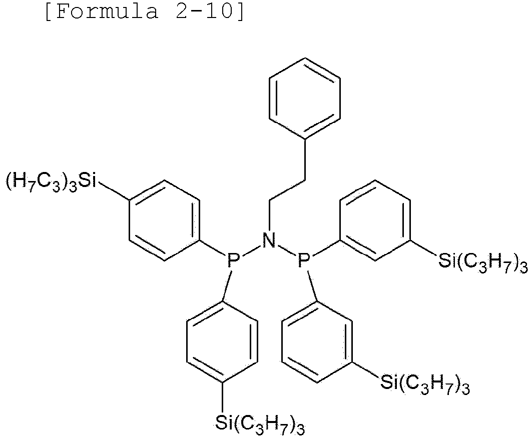

¹H NMR (500 MHz, DMSO): δ 7.75-7.13(m, 21H), 2.98(m, 2H), 2.83(m, 2H), 1.44-1.29(m, 48H), 0.94(m, 36H)

### Synthesis Example 11: Synthesis of ligand compound represented by Formula 3-1

0.94 g (11 mmol, 2.2 eq) of cyclopentylamine and 10 ml of dichloromethane were introduced into a dried flask under a nitrogen atmosphere and stirred. 3.09 g (5 mmol, 1 eq) of chlorobis(3-(tributylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.33 g (5 mmol) of N-cycloheptyl-1,1-bis(3-(tributylsilyl)phenyl)phosphanamine, which is Intermediate Compound 11, was obtained through column chromatography.

### <Intermediate Compound 11>

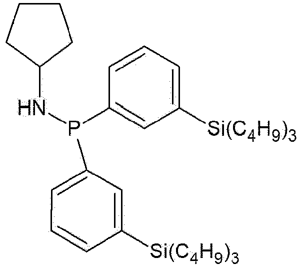

¹H NMR (500 MHz, DMSO): δ 7.55 (m, 2H), 7.40(m, 2H), 7.33(m, 2H), 7.13(m, 2H), 2.64(m, 1H), 1.85(m, 4H), 1.73(m, 4H), 1.50(m, 1H), 1.43 (m, 12H), 1.30(m, 12H), 1.23(m, 12H), 0.89(m, 18H)

Subsequently, 3.33 g (5 mmol, 1 eq) of N-cyclopentyl-1,1-bis(3-(tributylsilyl)phenyl)phosphanamine, which is Intermediate Compound 11 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 5.49 g (4 mmol, yield 88%) of N-(bis(3-(tributylsilyl)phenyl)phosphaneyl)-N-cyclopentyl-1,1-bis(4-(tributylsilyl)phenyl)phosphanamine, which is a compound represented by Formula 3-1, was obtained through column chromatography.

### <Ligand compound represented by Formula 3-1>

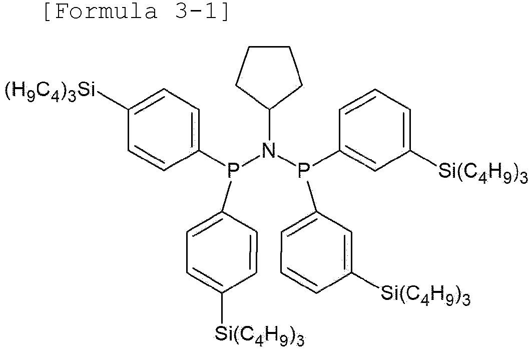

¹H NMR (500 MHz, DMSO): δ 7.60-7.13(m, 16H), 2.68 (m, 1H), 1.86(m, 4H), 1.77(m, 4H), 1.43-1.23(m, 72H), 0.91(m, 36H)

### Synthesis Example 12: Synthesis of ligand compound represented by Formula 3-2

1.09 g (11 mmol, 2.2 eq) of cyclohexylamine and 10 ml of dichloromethane were introduced into a dried flask under a nitrogen atmosphere and stirred. 3.09 g (5 mmol, 1 eq) of chlorobis(3-(tributylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.4 g (5 mmol) of N-cyclohexyl-1,1-bis(3-(tributylsilyl)phenyl)phosphanamine, which is Intermediate Compound 12, was obtained through column chromatography.

### <Intermediate Compound 12>

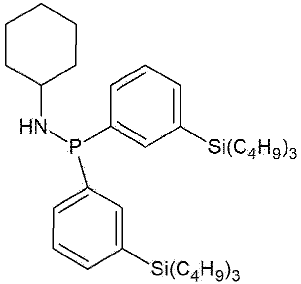

¹H NMR (500 MHz, DMSO) : δ 7.57 (m, 2H), 7.47 (m, 2H), 7.38(m, 2H), 7.15(m, 2H), 2.58(m, 1H), 1.75(m, 4H), 1.55(m, 1H), 1.51(m, 2H), 1.45(m, 12H), 1.33(m, 12H), 1.28(m, 12H), 1.21(m, 4H), 0.94(m, 18H)

Subsequently, 3.4 g (5 mmol, 1 eq) of N-cyclohexyl-1,1-bis(3-(tributylsilyl)phenyl)phosphanamine, which is Intermediate Compound 12 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 5.05 g (4 mmol, yield 80%) of N-(bis(3-(tributylsilyl)phenyl)phosphaneyl)-N-cyclohexyl-1,1-bis(4-(tributylsilyl)phenyl)phosphanamine, which is a compound represented by Formula 3-2, was obtained through column chromatography.

### <Ligand compound represented by Formula 3-2>

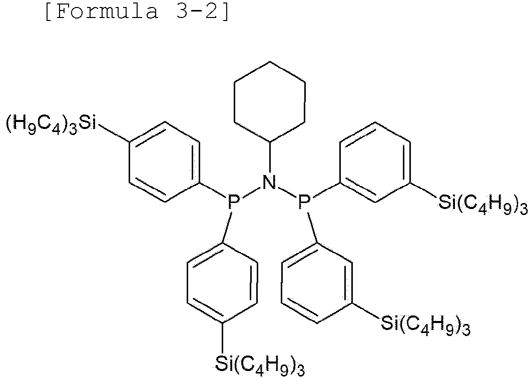

¹H NMR (500 MHz, DMSO): δ 7.60-7.19(m, 16H), 2.62(m, 1H), 1.78(m, 4H), 1.50(m, 26H), 1.30-1.22(m, 52H), 0.98(m, 36H)

### Synthesis Example 13: Synthesis of ligand compound represented by Formula 3-3

1.24 g (11 mmol, 2.2 eq) of cycloheptylamine and 10 ml of dichloromethane were introduced into a dried flask under a nitrogen atmosphere and stirred. 3.09 g (5 mmol, 1 eq) of chlorobis(3-(tributylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.47 g (5 mmol) of N-cycloheptyl-1,1-bis(3-(tributylsilyl)phenyl)phosphanamine, which is Intermediate Compound 13, was obtained through column chromatography.

### <Intermediate Compound 13>

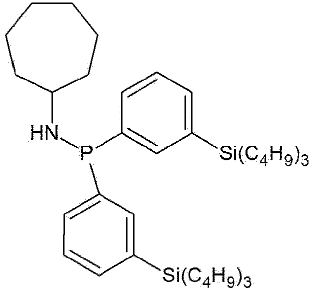

¹H NMR (500 MHz, DMSO): δ 7.64(m, 2H), 7.47(m, 2H), 7.38(m, 2H), 7.15(m, 2H), 2.45(m, 1H), 1.67(m, 4H), 1.58(m, 9H), 1.45(m, 12H), 1.35(m, 12H), 1.31(m, 12H), 0.92(m, 18H)

Subsequently, 3.47 g (5 mmol, 1 eq) of N-cycloheptyl-1,1-bis(3-(tributylsilyl)phenyl)phosphanamine, which is Intermediate Compound 13 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 5.2 g (4.09 mmol, yield 82%) of N-(bis(3-(tributylsilyl)phenyl)phosphaneyl)-N-cycloheptyl-1,1-bis(4-(tributylsilyl)phenyl)phosphanamine, which is a compound represented by Formula 3-3, was obtained through column chromatography.

### <Ligand compound represented by Formula 3-3>

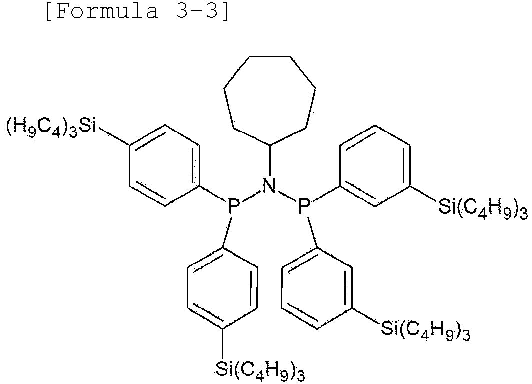

¹H NMR (500 MHz, DMSO) : δ 7.56-7.17(m, 16H), 2.47 (m, 1H), 1.64-1.59(m, 12H), 1.45(m, 24H), 1.33-1.31(m, 48H), 0.96(m, 36H)

### Synthesis Example 14: Synthesis of ligand compound represented by Formula 3-4

1.40 g (11 mmol, 2.2 eq) of cyclooctylamine and 10 ml of dichloromethane were introduced into a dried flask under a nitrogen atmosphere and stirred. 3.09 g (5 mmol, 1 eq) of chlorobis(3-(tributylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.54 g (5 mmol) of N-cyclooctyl-1,1-bis(3-(tributylsilyl)phenyl)phosphanamine, which is Intermediate Compound 14, was obtained through column chromatography.

### <Intermediate Compound 14>

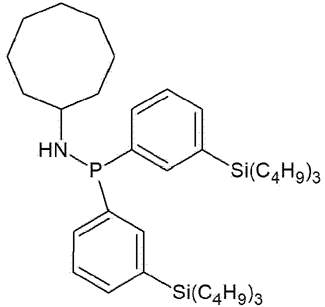

¹H NMR (500 MHz, DMSO) : δ 7.55 (m, 2H), 7.43 (m, 2H), 7.37(m, 2H), 7.20(m, 2H), 2.48(m, 1H), 1.66(m, 4H), 1.55(m, 1H), 1.50(m, 12H), 1.39(m, 18H), 1.26(m, 16H), 0.91(m, 18H)

Subsequently, 3.54 g (5 mmol, 1 eq) of N-cyclooctyl-1,1-bis(3-(tributylsilyl)phenyl)phosphanamine, which is Intermediate Compound 14 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.44 g (3.45 mmol, yield 69%) of N-(bis(3-(tributylsilyl)phenyl)phosphaneyl)-N-cyclooctyl-1,1-bis(4-(tributylsilyl)phenyl)phosphanamine, which is a compound represented by Formula 3-4, was obtained through column chromatography.

### <Ligand compound represented by Formula 3-4>

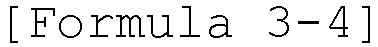

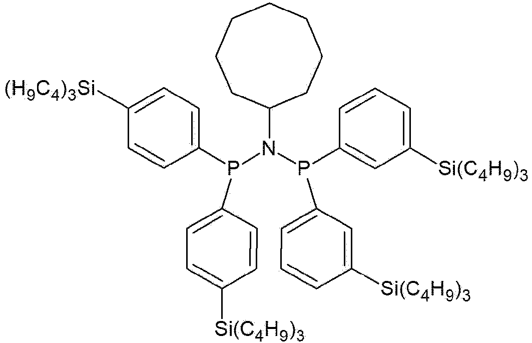

¹H NMR (500 MHz, DMSO): δ 7.56-7.17(m, 16H), 2.50 (m, 1H), 1.64 (m, 4H), 1.47(m, 24H), 1.39-1.31(m, 58H), 0.90(m, 36H)

### Synthesis Example 15: Synthesis of ligand compound represented by Formula 3-5

1.47 g (11 mmol, 2.2 eq) of 2,3-dihydro-1H-inden-2-yl)amine and 10 ml of dichloromethane were introduced into a dried flask under a nitrogen atmosphere and stirred. 3.09 g (5 mmol, 1 eq) of chlorobis(3-(tributylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.57 g (5 mmol) of N-(2,3-dihydro-1H-inden-2-yl)-1,1-bis(3-(tributylsilyl)phenyl)phosphanamine, which is Intermediate Compound 15, was obtained through column chromatography.

### <Intermediate Compound 15>

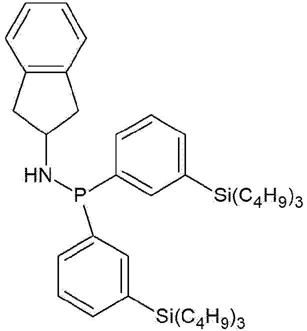

¹H NMR (500 MHz, DMSO) : δ 7.57 (m, 2H), 7.47 (m, 2H), 7.37(m, 2H), 7.34(m, 2H), 7.18(m, 2H), 7.11(m, 2H), 3.18(m, 5H), 1.59(m, 1H), 1.52(m, 12H), 1.39(m, 12H), 1.26(m, 12H), 0.90(m, 18H)

Subsequently, 3.57 g (5 mmol, 1 eq) of N-(2,3-dihydro-1H-inden-2-yl)-1,1-bis(3-(tributylsilyl)phenyl)phosphanamine, which is Intermediate Compound 15 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 6.40 g (4.94 mmol, yield 99%) of N-(bis(3-(tributylsilyl)phenyl)phosphaneyl)-N-(2,3-dihydro-1H-inden-2-yl)-1,1-bis(4-(tributylsilyl)phenyl)phosphanamine, a compound represented by Formula 3-5, was obtained through column chromatography.

### <Ligand compound represented by Formula 3-5>

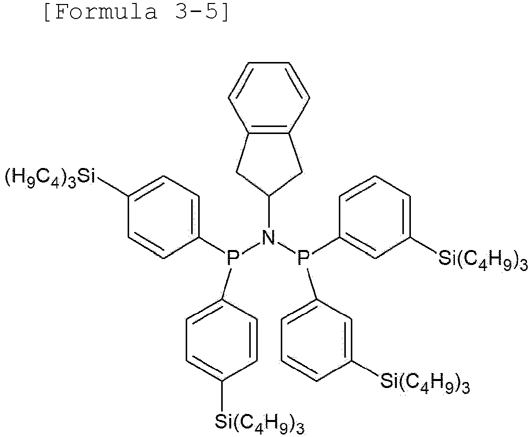

¹H NMR (500 MHz, DMSO): δ 7.58-7.15(m, 20H), 3.19 (m, 1H), 3.13(m, 4H), 1.43 (m, 24H), 1.30-1.26(m, 48H), 0.91(m, 36H)

### Synthesis Example 16: Synthesis of ligand compound represented by Formula 3-6

1.62 g (11 mmol, 2.2 eq) of 1,2,3,4-tetrahydronaphthalen-2-yl)amine and 10 ml of dichloromethane were introduced into a dried flask under a nitrogen atmosphere and stirred. 3.09 g (5 mmol, 1 eq) of chlorobis(3-(tributylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.64 g (5 mmol) of N-(1,2,3,4-tetrahydronaphthalen-2-yl)-1,1-bis(3-(tributylsilyl)phenyl)phosphanamine, which is Intermediate Compound 16, was obtained through column chromatography.

### <Intermediate Compound 16>

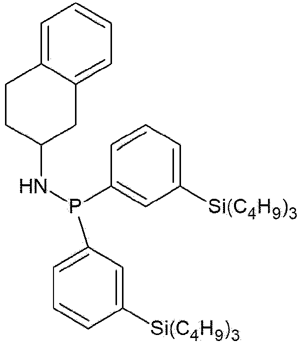

¹H NMR (500 MHz, DMSO) : δ 7.59(m, 2H), 7.49 (m, 2H), 7.41(m, 2H), 7.21(m, 2H), 6.94 (m, 4H), 3.20 (m, 2H), 2.93 (m, 2H), 2.78(m, 1H), 1.96(m, 2H), 1.54(m, 1H), 1.48(m, 12H), 1.35(m, 12H), 1.24(m, 12H), 0.93(m, 18H)

Subsequently, 3.64 g (5 mmol, 1 eq) of N-(1,2,3,4-tetrahydronaphthalen-2-yl)-1,1-bis(3-(tributylsilyl)phenyl)phosphanamine, which is Intermediate Compound 16 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 5.02 g (3.83 mmol, yield 77%) of N-(bis(3-(tributylsilyl)phenyl)phosphaneyl)-N-(1,2,3,4-tetrahydronaphthalen-2-yl)-1,1-bis(4-(tributylsilyl)phenyl)phosphanamine, which is a compound represented by Formula 3-6, was obtained through column chromatography.

### <Ligand compound represented by Formula 3-6>

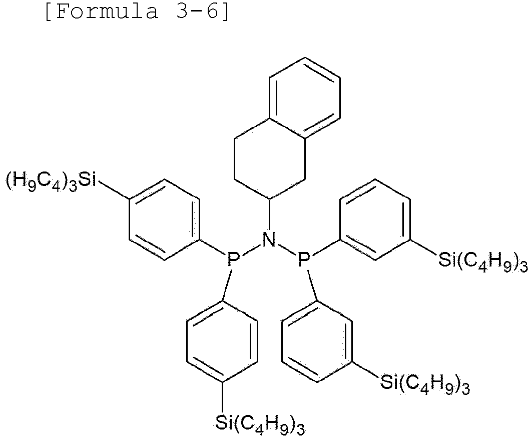

¹H NMR (500 MHz, DMSO) : δ 7.61-6.93(m, 20H), 3.28 (m, 2H), 2.93 (m, 2H), 2.80(m, 1H), 1.99(m, 2H), 1.43(m, 24H), 1.39-1.24(m, 48H), 0.90(m, 36H)

### Synthesis Example 17: Synthesis of ligand compound represented by Formula 3-7

0.65 g (11 mmol, 2.2 eq) of isopropylamine and 10 ml of dichloromethane were introduced into a dried flask under a nitrogen atmosphere and stirred. 3.09 g (5 mmol, 1 eq) of chlorobis(3-(tributylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.2 g (5 mmol) of N-isopropyl-1,1-bis(3-(tributylsilyl)phenyl)phosphanamine, which is Intermediate Compound 17, was obtained through column chromatography.

### <Intermediate Compound 17>

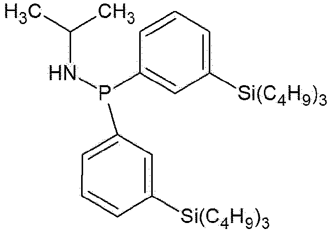

¹H NMR (500 MHz, DMSO) : δ 7.62(m, 2H), 7.40(m, 4H), 7.19(m, 2H), 2.89(m, 1H), 1.53 (m, 1H), 1.51(m, 12H), 1.37 (m, 12H), 1.32(m, 12H), 1.13(m, 6H), 0.94(m, 18H)

Subsequently, 3.2 g (5 mmol, 1 eq) of N-isopropyl-1,1-bis(3-(tributylsilyl)phenyl)phosphanamine, which is Intermediate Compound 17 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 3.76 g (3.08 mmol, yield 62%) of N-(bis(3-(tributylsilyl)phenyl)phosphaneyl)-N-isopropyl-1,1-bis(4-(tributylsilyl)phenyl)phosphanamine, which is a compound represented by Formula 3-7, was obtained through column chromatography.

### <Ligand compound represented by Formula 3-7>

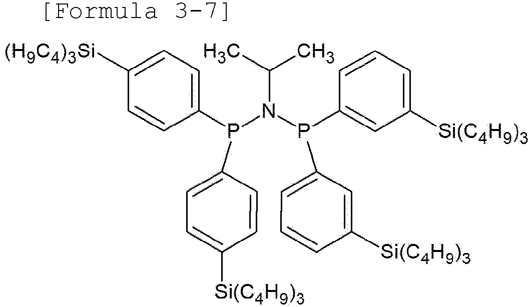

¹H NMR (500 MHz, DMSO): δ 7.61-7.17(m, 16H), 2.90(m, 1H), 1.52(m, 24H), 1.35-1.27(m, 48H), 1.09(m, 6H), 0.91(m, 36H)

### Synthesis Example 18: Synthesis of ligand compound represented by Formula 3-8

0.96 g (11 mmol, 2.2 eq) of 3-methylbutan-2-yl)amine and 10 ml of dichloromethane were introduced into a dried flask under a nitrogen atmosphere and stirred. 3.09 g (5 mmol, 1 eq) of chlorobis(3-(tributylsilyl)phenyl)phosphane diluted in 10 ml of dichloromethane was slowly introduced into the flask. A reactant was stirred for 4 hours, and conversion was confirmed through ¹H NMR. After the reaction was completed solids were removed through a filter, the solvent was removed by decompression, and 3.3 g (5 mmol) of N-(3-methylbutan-2-yl)-1,1-bis(3-(tributylsilyl)phenyl)phosphanamine, which is Intermediate Compound 18, was obtained through column chromatography.

### <Intermediate Compound 18>

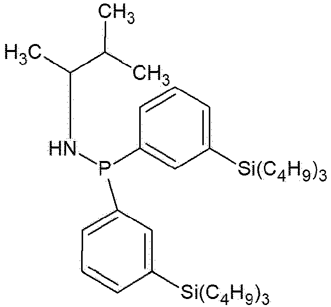

¹H NMR (500 MHz, DMSO): δ 7.62(m, 2H), 7.47 (m, 2H), 7.38(m, 2H), 7.19(m, 2H), 2.56(m, 1H), 1.59(m, 1H), 1.56(m, 1H), 1.47(m, 12H), 1.33(m, 12H), 1.29(m, 12H), 1.08(m, 3H), 0.97(m, 18H), 0.94(m, 6H)

Subsequently, 3.3 g (5 mmol, 1 eq) of N-(3-methylbutan-2-yl)-1,1-bis(3-(tributylsilyl)phenyl)phosphanamine, which is Intermediate Compound 18 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.0 g (3.2 mmol, yield 64%) of N-(bis(3-(tributylsilyl)phenyl)phosphaneyl)-N-(3-methylbutan-2-yl)-1,1-bis(4-(tributylsilyl)phenyl)phosphanamine, which is a compound represented by Formula 3-8, was obtained through column chromatography.

### <Ligand compound represented by Formula 3-8>

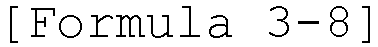

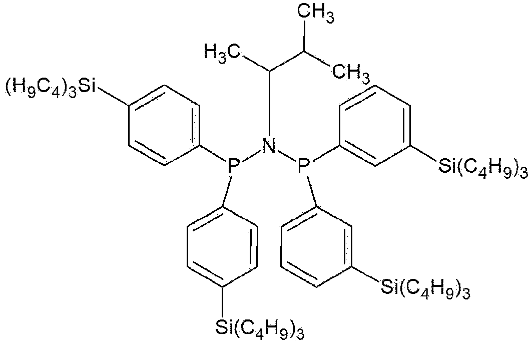

¹H NMR (500 MHz, DMSO): δ 7.64-7.20(m, 16H), 2.55 (m, 1H), 1.58(m, 1H), 1.48(m, 24H), 1.36-1.26(m, 48H), 1.11(m, 3H), 0.98(m, 36H), 0.94(m, 6H)

### Synthesis Example 19: Synthesis of ligand compound represented by Formula 3-9

1.18 g (11 mmol, 2.2 eq) of benzylamine and 10 ml of dichloromethane were introduced into a dried flask under a nitrogen atmosphere and stirred. 3.09 g (5 mmol, 1 eq) of chlorobis(3-(tributylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.4 g (5 mmol) of N-benzyl-1,1-bis(3-(tributylsilyl)phenyl)phosphanamine, which is Intermediate Compound 19, was obtained through column chromatography.

### <Intermediate Compound 19>

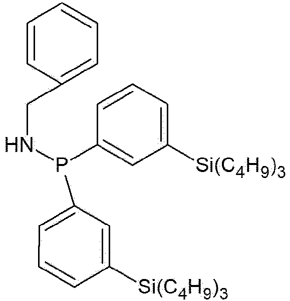

¹H NMR (500 MHz, DMSO): δ 7.60(m, 2H), 7.48(m, 2H), 7.37(m, 2H), 7.35(m, 4H), 7.28(m, 1H), 7.13(m, 2H), 3.95(m, 2H), 2.32(m, 1H), 1.48(m, 12H), 1.35(m, 12H), 1.28(m, 12H), 0.91(m, 18H)

Subsequently, 3.4 g (5 mmol, 1 eq) of N-benzyl-1,1-bis(3-(tributylsilyl)phenyl)phosphanamine, which is Intermediate Compound 19 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 3.36 g (4.22 mmol, yield 84%) of N-(bis(3-(tributylsilyl)phenyl)phosphaneyl)-N-benzyl-1,1-bis(4-(tributylsilyl)phenyl)phosphanamine, which is a compound represented by Formula 3-9, was obtained through column chromatography.

### <Ligand compound represented by Formula 3-9>

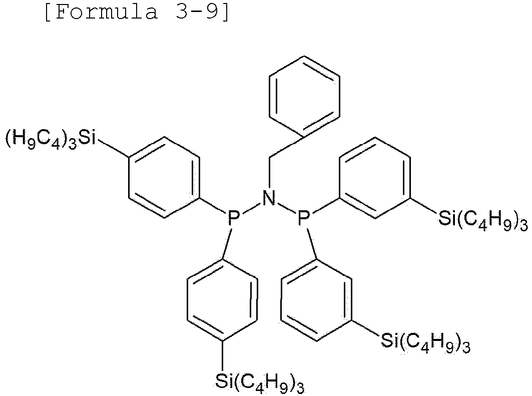

¹H NMR (500 MHz, DMSO) : δ 7.64(m, 21H), 3.96 (m, 2H), 1.51(m, 24H), 1.35-1.31(m, 48H), 0.93(m, 36H)

### Synthesis Example 20: Synthesis of ligand compound represented by Formula 3-10

1.33 g (11 mmol, 2.2 eq) of phenethylamine and 10 ml of dichloromethane were introduced into a dried flask under a nitrogen atmosphere and stirred. 3.09 g (5 mmol, 1 eq) of chlorobis(3-(tributylsilyl)phenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.5 g (5 mmol) of N-phenethyl-1,1-bis(3-(tributylsilyl)phenyl)phosphanamine, which is Intermediate Compound 20, was obtained through column chromatography.

### <Intermediate Compound 20>

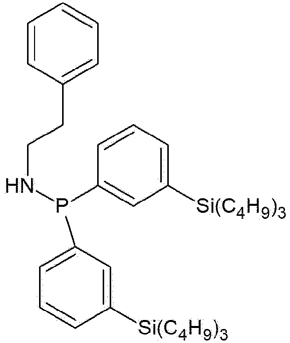

¹H NMR (500 MHz, DMSO): δ 7.62(m, 2H), 7.44(m, 2H), 7.37(m, 2H), 7.31(m, 2H), 7.27(m, 2H), 7.24(m, 1H), 7.14(m, 2H), 2.99(m, 2H), 2.85(m, 2H), 1.52(m, 1H), 1.45(m, 12H), 1.35(m, 12H), 1.30(m, 12H), 0.94 (m, 18H)

Subsequently, 3.4 g (5 mmol, 1 eq) of N-phenethyl-1,1-bis(3-(tributylsilyl)phenyl)phosphanamine, which is Intermediate Compound 20 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 5.54 g (4.32 mmol, yield 86%) of N-(bis(3-(tributylsilyl)phenyl)phosphaneyl)-N-phenethyl-1,1-bis(4-(tributylsilyl)phenyl)phosphanamine, which is a compound represented by Formula 3-10, was obtained through column chromatography.

### <Ligand compound represented by Formula 3-10>

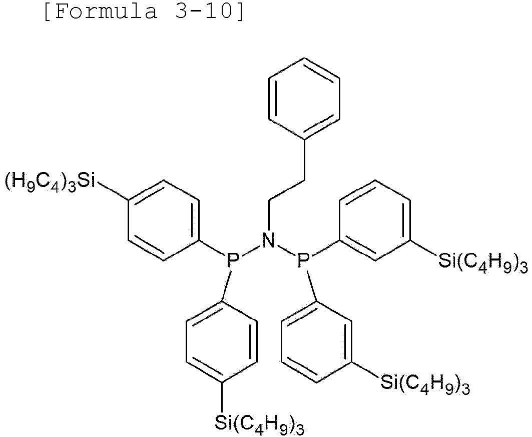

¹H NMR (500 MHz, DMSO): δ 7.62-7.20(m, 21H), 3.06 (m, 2H), 2.92 (m, 2H), 1.50(m, 24H), 1.37-1.30(m, 48H), 0.91 (m, 36H)

### Synthesis Example 21: Synthesis of ligand compound represented by Formula 4-1

2.9 g (5 mmol, 1 eq) of N-cyclopentyl-1,1-bis(3-(tripropylsilyl)phenyl)phosphanamine, which is Intermediate Compound 1 obtained in Synthesis Example 1 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 3.85 g (3.44 mmol, yield 69%) of N-(bis(3-(tripropylsilyl)phenyl)phosphaneyl)-N-cycloheptyl-1,1-bis(4-(tributylsilyl)phenyl)phosphanamine, which is a compound represented by Formula 4-1, was obtained through column chromatography.

### <Ligand compound represented by Formula 4-1>

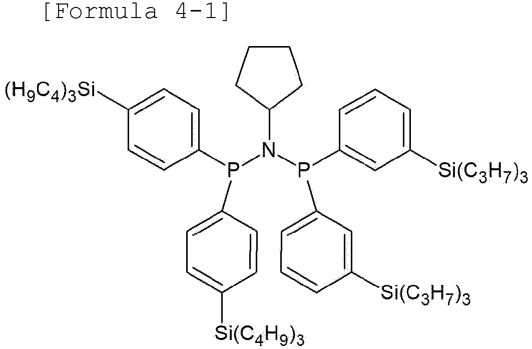

¹H NMR (500 MHz, DMSO) : δ 7.61-7.13 (m, 16H), 2.67 (m, 1H), 1.85(m, 4H), 1.75(m, 4H), 1.46(m, 24H), 1.35-1.31(m, 36H), 0.99-0.96(m, 36H)

### Synthesis Example 22: Synthesis of ligand compound represented by Formula 4-2

2.98 g (5 mmol, 1 eq) of N-cyclohexyl-1,1-bis(3-(tripropylsilyl)phenyl)phosphanamine, which is Intermediate Compound 2 obtained in Synthesis Example 2 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 5.12 g (4.6 mmol, yield 92%) of N-(bis(3-(tripropylsilyl)phenyl)phosphaneyl)-N-cyclohexyl-1,1-bis(4-(tributylsilyl)phenyl)phosphanamine, which is a compound represented by Formula 4-2, was obtained through column chromatography.

### <Ligand compound represented by Formula 4-2>

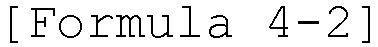

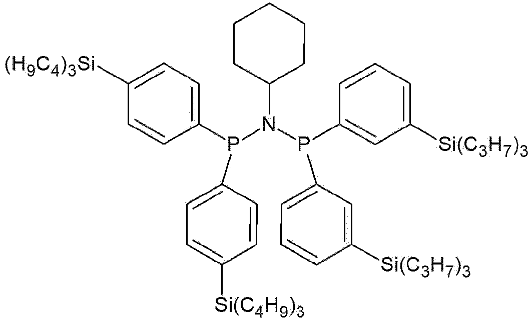

¹H NMR (500 MHz, DMSO): δ 7.59-7.19(m, 16H), 2.64-1.30(m, 71H), 0.96-0.94(m, 36H)

### Synthesis Example 23: Synthesis of ligand compound represented by Formula 4-3

3.5 g (5 mmol, 1 eq) of N-cycloheptyl-1,1-bis(3-(tripropylsilyl)phenyl)phosphanamine, which is Intermediate Compound 3 obtained in Synthesis Example 3 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 5.04 g (4.39 mmol, yield 88%) of N-(bis(3-(tripropylsilyl)phenyl)phosphaneyl)-N-cycloheptyl-1,1-bis(4-(tributylsilyl)phenyl)phosphanamine, which is a compound represented by Formula 4-3, was obtained through column chromatography.

### <Ligand compound represented by Formula 4-3>

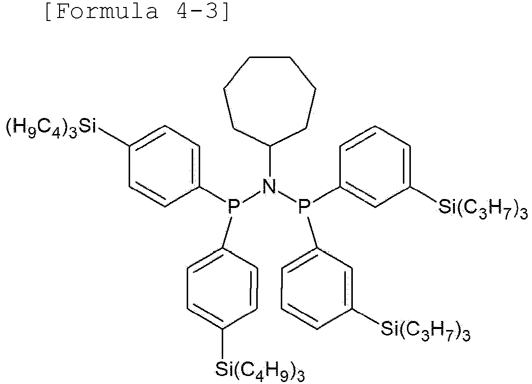

¹H NMR (500 MHz, DMSO): δ 7.59-7.16(m, 16H), 2.49 (m, 1H), 1.65-1.25(m, 72H), 0.95-0.91(m, 36H)

### Synthesis Example 24: Synthesis of ligand compound represented by Formula 4-4

3.1 g (5 mmol, 1 eq) of N-cyclooctyl-1,1-bis(3-(tripropylsilyl)phenyl)phosphanamine, which is Intermediate Compound 4 obtained in Synthesis Example 4 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.26 g (3.67 mmol, yield 73%) of N-(bis(3-(tripropylsilyl)phenyl)phosphaneyl)-N-cyclooctyl-1,1-bis(4-(tributylsilyl)phenyl)phosphanamine, which is a compound represented by Formula 4-4, was obtained through column chromatography.

### <Ligand compound represented by Formula 4-4>

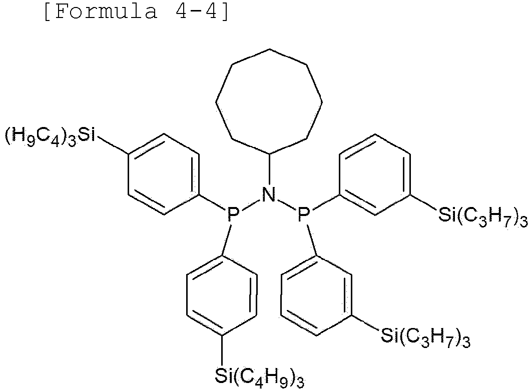

¹H NMR (500 MHz, DMSO): δ 7.55-7.20(m, 16H), 2.44 (m, 1H), 1.65-1.26(m, 74H), 1.02-0.96(m, 36H)

### Synthesis Example 25: Synthesis of ligand compound represented by Formula 4-5

3.2 g (5 mmol, 1 eq) of N-(2,3-dihydro-1H-inden-2-yl)-1,1-bis(3-(tripropylsilyl)phenyl)phosphanamine, which is Intermediate Compound 5 obtained in Synthesis Example 5 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis (4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 5.70 g (4.88 mmol, yield 98%) of N-(bis(3-(tripropylsilyl)phenyl)phosphaneyl)-N-(2,3-dihydro-1H-inden-2-yl)-1,1-bis(4-(tributylsilyl)phenyl)phosphanamine, a compound represented by Formula 4-5, was obtained through column chromatography.

### <Ligand compound represented by Formula 4-5>

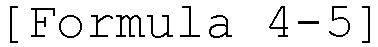

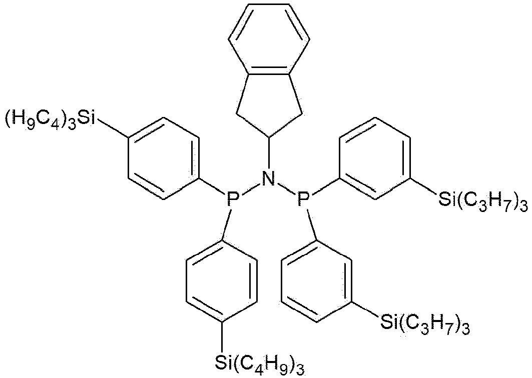

¹H NMR (500 MHz, DMSO): δ 7.64-7.12(m, 20H), 3.20(m, 5H), 1.48-1.26(m, 60H), 1.01-0.96(m, 36H)

### Synthesis Example 26: Synthesis of ligand compound represented by Formula 4-6

3.2 g (5 mmol, 1 eq) of N-(1,2,3,4-tetrahydronaphthalen-2-yl)-1,1-bis(3-(tripropylsilyl)phenyl)phosphanamine, which is Intermediate Compound 6 obtained in Synthesis Example 6 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 3.92 g (4 mmol, yield 66%) of N-(bis(3-(tripropylsilyl)phenyl)phosphaneyl)-N-(1,2,3,4-tetrahydronaphthalen-2-yl)-1,1-bis(4-(tributylsilyl)phenyl)phosphanamine, which is a compound represented by Formula 4-6, was obtained through column chromatography.

### <Ligand compound represented by Formula 4-6>

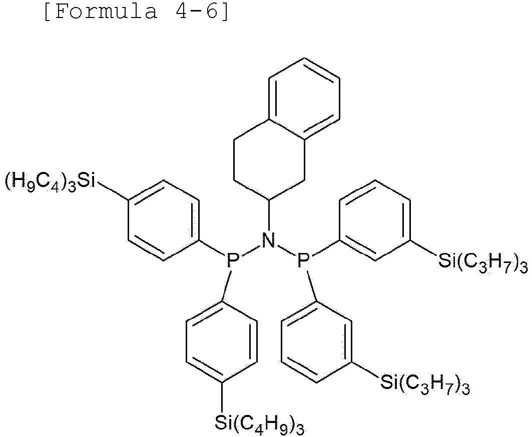

¹H NMR (500 MHz, DMSO) : δ 7.56-6.92 (m, 20H), 3.22 (m, 2H), 2.97(m, 2H), 2.76(m, 1H), 1.99(m, 2H), 1.45-1.23(m, 60H), 1.00-0.90 (m, 36H)

### Synthesis Example 27: Synthesis of ligand compound represented by Formula 4-7

2.7 g (5 mmol, 1 eq) of N-isopropyl-1,1-bis(3-(tripropylsilyl)phenyl)phosphanamine, which is Compound 7 obtained in Synthesis Example 7 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 3.5 g (3.2 mmol, yield 64%) of N-(bis(3-(tripropylsilyl)phenyl)phosphaneyl)-N-isopropyl-1,1-bis(4-(tributylsilyl)phenyl)phosphanamine, which is a compound represented by Formula 4-7, was obtained through column chromatography.

### <Ligand compound represented by Formula 4-7>

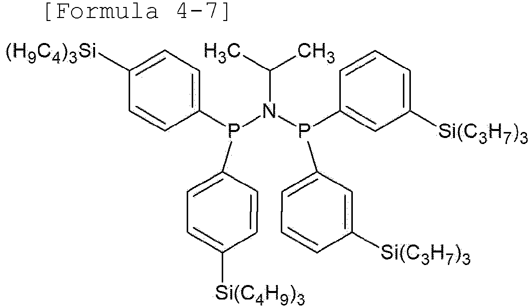

¹H NMR (500 MHz, DMSO): δ 7.59-7.13(m, 16H), 2.84 (m, 1H), 1.45-1.15(m, 66H), 0.94-0.92(m, 36H)

### Synthesis Example 28: Synthesis of ligand compound represented by Formula 4-8

2.9 g (5 mmol, 1 eq) of N-(3-methylbutan-2-yl)-1,1-bis(3-(tripropylsilyl)phenyl)phosphanamine, which is Intermediate Compound 8 obtained in Synthesis Example 8 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis (4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.05 g (3.61 mmol, yield 72%) of N-(bis(3-(tripropylsilyl)phenyl)phosphaneyl)-N-(3-methylbutan-2-yl)-1,1-bis(4-(tributylsilyl)phenyl)phosphanamine, which is a compound represented by Formula 4-8, was obtained through column chromatography.

### <Ligand compound represented by Formula 4-8>

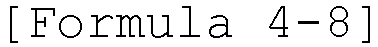

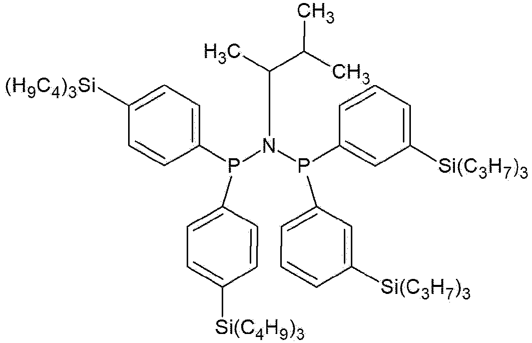

¹H NMR (500 MHz, DMSO): δ 7.61-7.15(m, 16H), 2.55(m, 1H), 1.55-1.31(m, 61H), 1.09-0.91(m, 45H)

### Synthesis Example 29: Synthesis of ligand compound represented by Formula 4-9

3.0 g (5 mmol, 1 eq) of N-benzyl-1,1-bis(3-(tripropylsilyl)phenyl)phosphanamine, which is Intermediate Compound 9 obtained in Synthesis Example 9 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 5.05 g (4.42 mmol, yield 88%) of N-(bis(3-(tripropylsilyl)phenyl)phosphaneyl)-N-benzyl-1,1-bis(4-(tributylsilyl)phenyl)phosphanamine, which is a compound represented by Formula 4-9, was obtained through column chromatography.

### <Ligand compound represented by Formula 4-9>

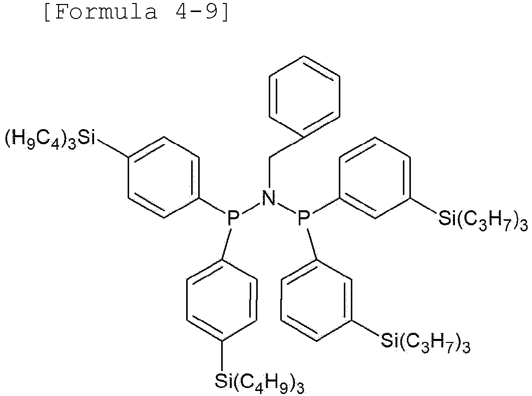

¹H NMR (500 MHz, DMSO): δ 7.58-7.16 (m, 21H), 3.95 (m, 2H), 1.50-1.32(m, 60H), 1.03-0.98(m, 36H)

### Synthesis Example 30: Synthesis of ligand compound represented by Formula 4-10

3.09 g (5 mmol, 1 eq) of N-phenethyl-1,1-bis(3-(tripropylsilyl)phenyl)phosphanamine, which is Intermediate Compound 10 obtained in Synthesis Example 10 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 3.09 g (5 mmol, 1 eq) of chlorobis(4-(tributylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.41 g (3.82 mmol, yield 76%) of N-(bis(3-(tripropylsilyl)phenyl)phosphaneyl)-N-phenethyl-1,1-bis(4-(tributylsilyl)phenyl)phosphanamine, which is a compound represented by Formula 4-10, was obtained through column chromatography.

### <Ligand compound represented by Formula 4-10>

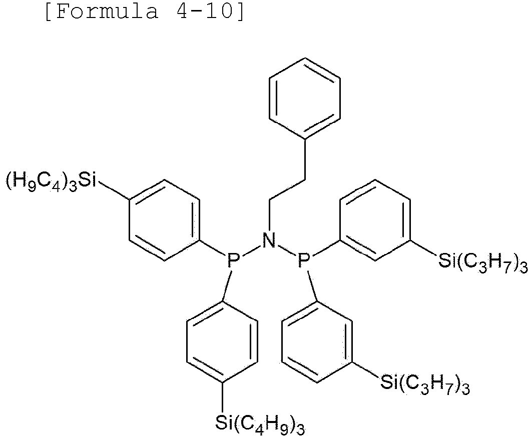

¹H NMR (500 MHz, DMSO) : δ 7.61-7.16 (m, 21H), 3.03 (m, 2H), 2.92(m, 2H), 1.49-1.28(m, 60H), 0.95-0.93(m, 36H)

### Synthesis Example 31: Synthesis of ligand compound represented by Formula 5-1

3.3 g (5 mmol, 1 eq) of N-cyclopentyl-1,1-bis(3-(tripropylsilyl)phenyl)phosphanamine, which is Intermediate Compound 1 obtained in Synthesis Example 1 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.27 g (4 mmol, yield 76%) of N-(bis(3-(tributylsilyl)phenyl)phosphaneyl)-N-cycloheptyl-1,1-bis(4-(tripropylsilyl)phenyl)phosphanamine, which is a compound represented by Formula 5-1, was obtained through column chromatography.

### <Ligand compound represented by Formula 5-1>

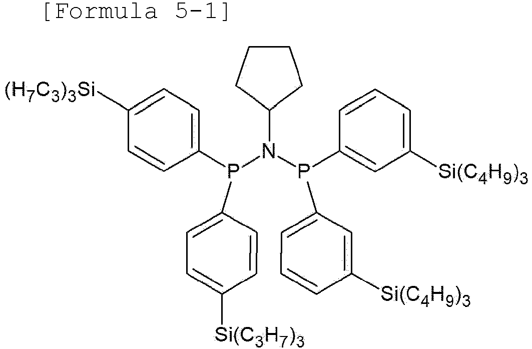

¹H NMR (500 MHz, DMSO): δ 7.61-7.10 (m, 16H), 2.66 (m, 1H), 1.90(m, 4H), 1.71 (m, 4H), 1.51(m, 24H), 1.39-1.31(m, 36H), 0.98-0.93(m, 36H)

### Synthesis Example 32: Synthesis of ligand compound represented by Formula 5-2

3.40 g (5 mmol, 1 eq) of N-cyclohexyl-1,1-bis(3-(tributylsilyl)phenyl)phosphanamine, which is Intermediate Compound 12 obtained in Synthesis Example 12 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 5.38 g (4 mmol, yield 95%) of N-(bis(3-(tributylsilyl)phenyl)phosphaneyl)-N-cyclohexyl-1,1-bis(4-(tripropylsilyl)phenyl)phosphanamine, which is a compound represented by Formula 5-2, was obtained through column chromatography.

### <Ligand compound represented by Formula 5-2>

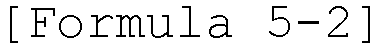

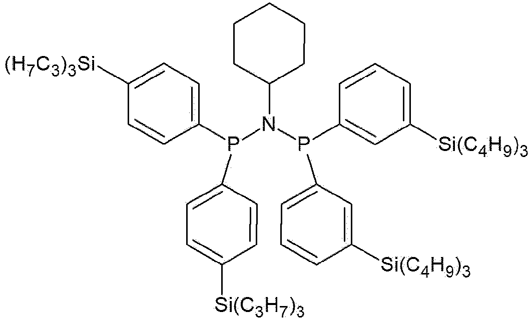

¹H NMR (500 MHz, DMSO): δ 7.59-7.11(m, 16H), 2.64-1.35(m, 71H), 0.94-0.91(m, 36H)

### Synthesis Example 33: Synthesis of ligand compound represented by Formula 5-3

3.47 g (5 mmol, 1 eq) of N-cycloheptyl-1,1-bis(3-(tributylsilyl)phenyl)phosphanamine, which is Intermediate Compound 13 obtained in Synthesis Example 13 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.69 g (4.08 mmol, yield 82%) of N-(bis(3-(tributylsilyl)phenyl)phosphaneyl)-N-cycloheptyl-1,1-bis(4-(tripropylsilyl)phenyl)phosphanamine, which is a compound represented by Formula 5-3, was obtained through column chromatography.

### <Ligand compound represented by Formula 5-3>

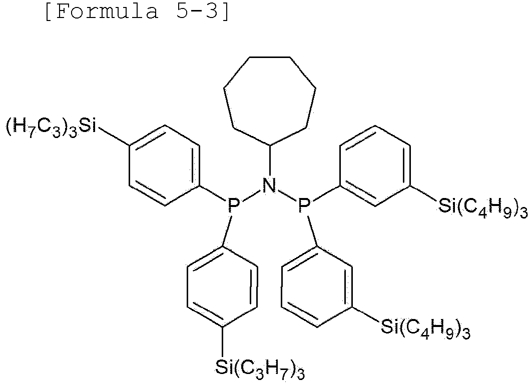

¹H NMR (500 MHz, DMSO): δ 7.69-7.12 (m, 16H), 2.56 (m, 1H), 1.68-1.25(m, 72H), 0.95-0.89(m, 36H)

### Synthesis Example 34: Synthesis of ligand compound represented by Formula 5-4

3.54 g (5 mmol, 1 eq) of N-cyclooctyl-1,1-bis(3-(tributylsilyl)phenyl)phosphanamine, which is Intermediate Compound 14 obtained in Synthesis Example 14 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.91 g (4.22 mmol, yield 84%) of N-(bis(3-(tributylsilyl)phenyl)phosphaneyl)-N-cyclooctyl-1,1-bis(4-(tripropylsilyl)phenyl)phosphanamine, which is a compound represented by Formula 5-4, was obtained through column chromatography.

### <Ligand compound represented by Formula 5-4>

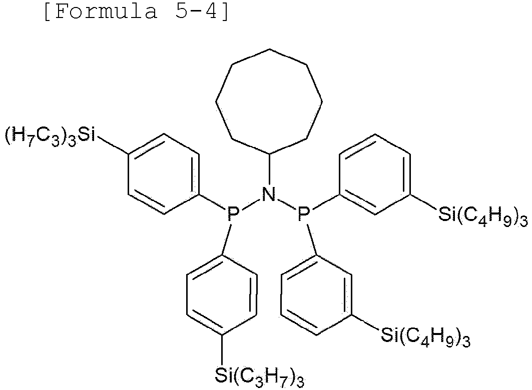

¹H NMR (500 MHz, DMSO) : δ 7.55-7.02 (m, 16H), 2.49 (m, 1H), 1.65-1.30(m, 74H), 1.02-0.92(m, 36H)

### Synthesis Example 35: Synthesis of ligand compound represented by Formula 3-5

3.54 g (5 mmol, 1 eq) of N-(2,3-dihydro-1H-inden-2-yl)-1,1-bis(3-(tributylsilyl)phenyl)phosphanamine, which is Intermediate Compound 15 obtained in Synthesis Example 15 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis (4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 5.55 g (4.75 mmol, yield 95%) of N-(bis(3-(tributylsilyl)phenyl)phosphaneyl)-N-(2,3-dihydro-1H-inden-2-yl)-1,1-bis(4-(tripropylsilyl)phenyl)phosphanamine, a compound represented by Formula 5-5, was obtained through column chromatography.

### <Ligand compound represented by Formula 5-5>

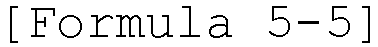

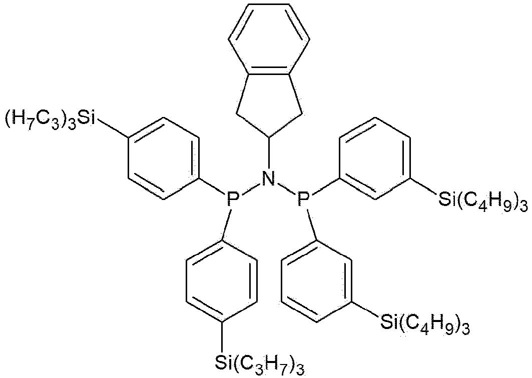

¹H NMR (500 MHz, DMSO) : δ 7.45-7.21 (m, 20H), 3.22 (m, 5H), 1.48-1.31(m, 60H), 1.01-0.90(m, 36H)

### Synthesis Example 36: Synthesis of ligand compound represented by Formula 5-6

3.64 g (5 mmol, 1 eq) of N-(1,2,3,4-tetrahydronaphthalen-2-yl)-1,1-bis(3-(tributylsilyl)phenyl)phosphanamine, which is Intermediate Compound 16 obtained in Synthesis Example 16 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.91 g (4.15 mmol, yield 83%) of N-(bis(3-(tributylsilyl)phenyl)phosphaneyl)-N-(1,2,3,4-tetrahydronaphthalen-2-yl)-1,1-bis(4-(tripropylsilyl)phenyl)phosphanamine, which is a compound represented by Formula 5-6, was obtained through column chromatography.

### <Ligand compound represented by Formula 5-6>

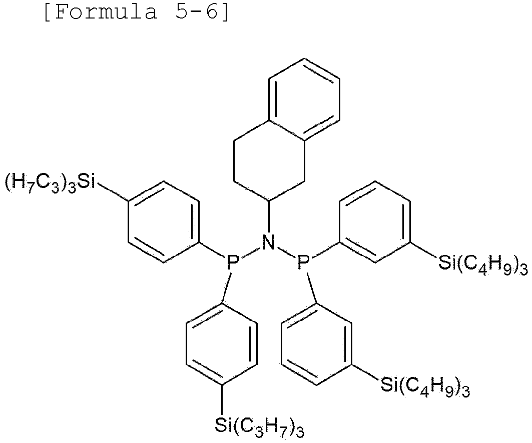

¹H NMR (500 MHz, DMSO) : δ 7. 65-6.90 (m, 20H), 3.37 (m, 2H), 2.97(m, 2H), 2.81 (m, 1H), 2.04 (m, 2H), 1.44-1.25(m, 60H), 1.04-0.94 (m, 36H)

### Synthesis Example 37: Synthesis of ligand compound represented by Formula 5-7

3.2 g (5 mmol, 1 eq) of N-isopropyl-1,1-bis(3-(tributylsilyl)phenyl)phosphanamine, which is Intermediate Compound 17 obtained in Synthesis Example 17 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 3.26 g (2.98 mmol, yield 60%) of N-(bis(3-(tributylsilyl)phenyl)phosphaneyl)-N-isopropyl-1,1-bis(4-(tripropylsilyl)phenyl)phosphanamine, which is a compound represented by Formula 5-7, was obtained through column chromatography.

### <Ligand compound represented by Formula 5-7>

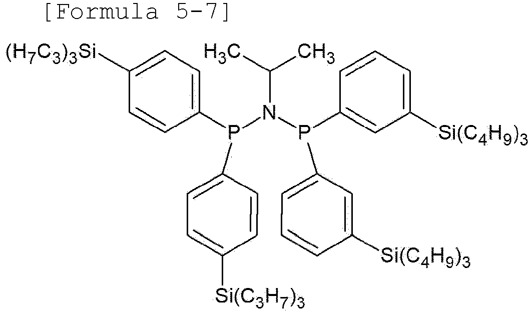

¹H NMR (500 MHz, DMSO): δ 7.72-7.11 (m, 16H), 2.48 (m, 1H), 1.54-1.21(m, 66H), 0.94-0.90(m, 36H)

### Synthesis Example 38: Synthesis of ligand compound represented by Formula 5-8

3.34 g (5 mmol, 1 eq) of N-(3-methylbutan-2-yl)-1,1-bis(3-(tributylsilyl)phenyl)phosphanamine, which is Intermediate Compound 18 obtained in Synthesis Example 18 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis (4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 3.71 g (3.31 mmol, yield 66%) of N-(bis(3-(tributylsilyl)phenyl)phosphaneyl)-N-(3-methylbutan-2-yl)-1,1-bis(4-(tripropylsilyl)phenyl)phosphanamine, which is a compound represented by Formula 5-8, was obtained through column chromatography.

### <Ligand compound represented by Formula 5-8>

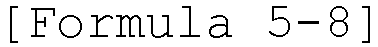

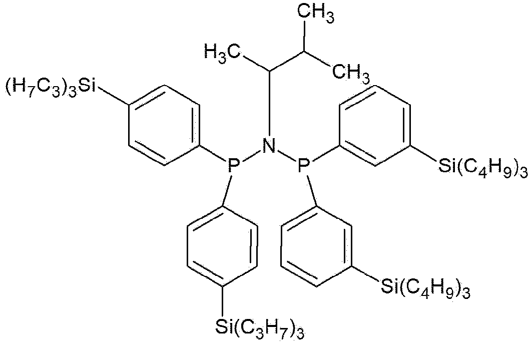

¹H NMR (500 MHz, DMSO): δ 7.67-7.21(m, 16H), 2.87 (m, 1H), 1.54-1.28(m, 61H), 1.09-0.88(m, 45H)

### Synthesis Example 39: Synthesis of ligand compound represented by Formula 5-9

3.44 g (5 mmol, 1 eq) of N-benzyl-1,1-bis(3-(tributylsilyl)phenyl)phosphanamine, which is Intermediate Compound 19 obtained in Synthesis Example 19 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.79 g (4.2 mmol, yield 84%) of N-(bis(3-(tributylsilyl)phenyl)phosphaneyl)-N-benzyl-1,1-bis(4-(tripropylsilyl)phenyl)phosphanamine, which is a compound represented by Formula 5-9, was obtained through column chromatography.

### <Ligand compound represented by Formula 5-9>

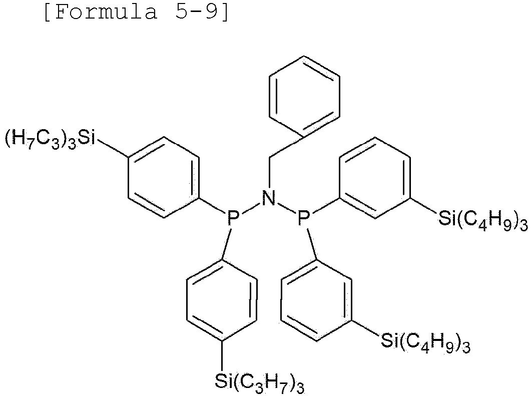

¹H NMR (500 MHz, DMSO) : δ 7.63-7.19 (m, 21H), 3.92 (m, 2H), 1.51-1.23(m, 60H), 1.03-0.91(m, 36H)

### Synthesis Example 40: Synthesis of ligand compound represented by Formula 5-10

3.51 g (5 mmol, 1 eq) of N-phenethyl-1,1-bis(3-(tributylsilyl)phenyl)phosphanamine, which is Intermediate Compound 20 obtained in Synthesis Example 20 above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.66 g (5 mmol, 1 eq) of chlorobis(4-(tripropylsilyl)phenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 5.16 g (4.46 mmol, yield 89%) of N-(bis(3-(tributylsilyl)phenyl)phosphaneyl)-N-phenethyl-1,1-bis(4-(tripropylsilyl)phenyl)phosphanamine, which is a compound represented by Formula 5-10, was obtained through column chromatography.

### <Ligand compound represented by Formula 5-10>

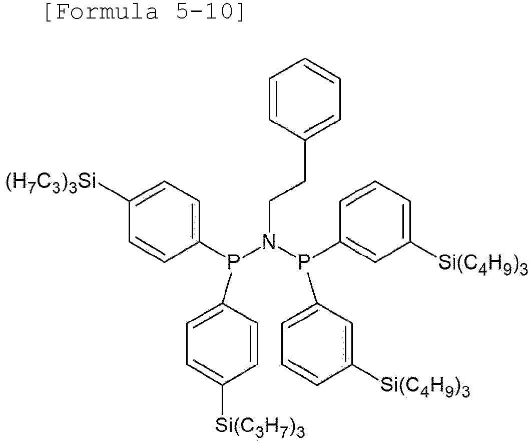

¹H NMR (500 MHz, DMSO): δ 7.65-7.15 (m, 21H), 3.08 (m, 2H), 2.90(m, 2H), 1.56-1.22(m, 60H), 0.95-0.87(m, 36H)

### Synthesis Example 41: Synthesis of ligand compound represented by Formula 6-1

0.94 g (11 mmol, 2.2 eq) of cyclopentylamine and 10 ml of dichloromethane were introduced into a dried flask under a nitrogen atmosphere and stirred. 2.51 g (5 mmol, 1 eq) of chlorobis(3-decylphenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 2.75 g (5 mmol) of N-cycloheptyl-1,1-bis(3-decylphenyl)phosphanamine, which is Intermediate Compound 21, was obtained through column chromatography.

### <Intermediate Compound 21>

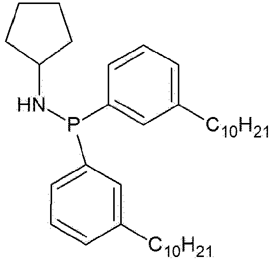

¹H NMR (500 MHz, DMSO) : δ 7.37 (m, 2H), 7.27 (m, 2H), 7.25(m, 2H), 7.13(m, 2H), 2.72 (m, 1H), 1.92(m, 4H) , 1.76 (m, 6H), 1.62(m, 6H), 1.52(m, 1H), 1.29(m, 28H), 0.93 (m, 6H)

Subsequently, 2.75 g (5 mmol, 1 eq) of N-cyclopentyl-1,1-bis(3-decylphenyl)phosphanamine, which is Intermediate Compound 21 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.51 g (5 mmol, 1 eq) of chlorobis(4-decylphenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 3.98 g (3.92 mmol, yield 78%) of N-(bis (3-decylphenyl)phosphaneyl)-N-cycloheptyl-1,1-bis(4-decylphenyl)phosphanamine, which is a compound represented by Formula 6-1, was obtained through column chromatography.

### <Ligand compound represented by Formula 6-1>

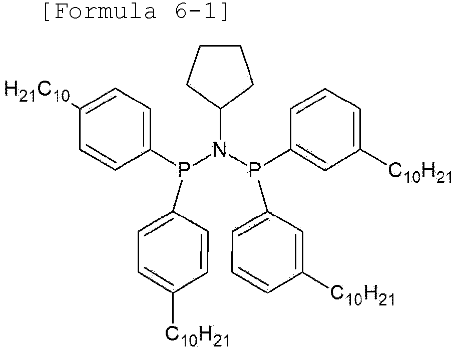

¹H NMR (500 MHz, DMSO): δ 7.35-7.12 (m, 16H), 2.65 (m, 1H), 1.89-1.27(m, 78H), 0.97(m, 12H)

### Synthesis Example 42: Synthesis of ligand compound represented by Formula 6-2

1.09 g (11 mmol, 2.2 eq) of cyclohexylamine and 10 ml of dichloromethane were introduced into a dried flask under a nitrogen atmosphere and stirred. 2.51 g (5 mmol, 1 eq) of chlorobis(3-decylphenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 2.82 g (5 mmol) of N-(bis(3-decylphenyl)phosphaneyl)-N-cyclohexyl-1,1-bis(4-decylphenyl)phosphanamine, which is Intermediate Compound 22, was obtained through column chromatography.

### <Intermediate Compound 22>

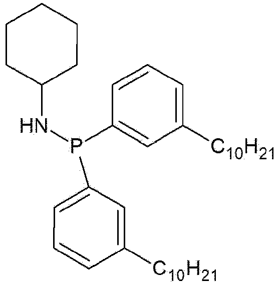

¹H NMR (500 MHz, DMSO) : δ 7.42 (m, 2H), 7.26 (m, 2H), 7.21(m, 2H), 7.06(m, 2H), 2.73(m, 4H), 2.65(m, 1H), 1.80(m, 4H), 1.69(m, 4H), 1.52(m, 3H), 1.27(m, 28H), 1.30(m, 4H), 0.93 (m, 6H)

Subsequently, 2.82 g (5 mmol, 1 eq) of N-cyclohexyl-1,1-bis(3-decylphenyl)phosphanamine, which is Intermediate Compound 22 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.51 g (5 mmol, 1 eq) of chlorobis(4-decylphenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.43 g (4.31 mmol, yield 86%) of N-(bis(3-decylphenyl)phosphaneyl)-N-cyclohexyl-1,1-bis(4-decylphenyl)phosphanamine, which is a compound represented by Formula 6-2, was obtained through column chromatography.

### <Ligand compound represented by Formula 6-2>

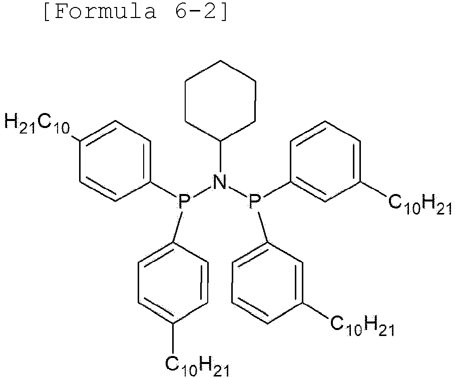

¹H NMR (500 MHz, DMSO) : δ 7.44 (m, 16H), 7.14 (m, 2H), 2.64 (m, 9H), 1.81-1.28(m, 74H), 0.91(m, 12H)

### Synthesis Example 43: Synthesis of ligand compound represented by Formula 6-3

1.24 g (11 mmol, 2.2 eq) of cycloheptylamine and 10 ml of dichloromethane were introduced into a dried flask under a nitrogen atmosphere and stirred. 2.51 g (5 mmol, 1 eq) of chlorobis(3-decylphenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 2.89 g (5 mmol) of N-cycloheptyl-1,1-bis(3-decylphenyl)phosphanamine, which is Intermediate Compound 23, was obtained through column chromatography.

### <Intermediate Compound 23>

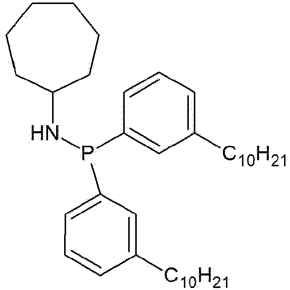

¹H NMR (500 MHz, DMSO): δ 7.38 (m, 2H), 7.29 (m, 2H), 7.20(m, 2H), 7.08(m, 2H), 2.65(m, 4H), 2.46(m, 1H), 1.66(m, 4H), 1.60(m, 9H), 1.57(m, 4H), 1.34(m, 28H), 0.93 (m, 6H)

Subsequently, 2.89 g (5 mmol, 1 eq) of N-cycloheptyl-1,1-bis(3-decylphenyl)phosphanamine, which is Intermediate Compound 23 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.51 g (5 mmol, 1 eq) of chlorobis(4-decylphenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.30 g (4.13 mmol, yield 83%) of N-(bis(3-decylphenyl)phosphaneyl)-N-cycloheptyl-1,1-bis(4-decylphenyl)phosphanamine, which is a compound represented by Formula 6-3, was obtained through column chromatography.

### <Ligand compound represented by Formula 6-3>

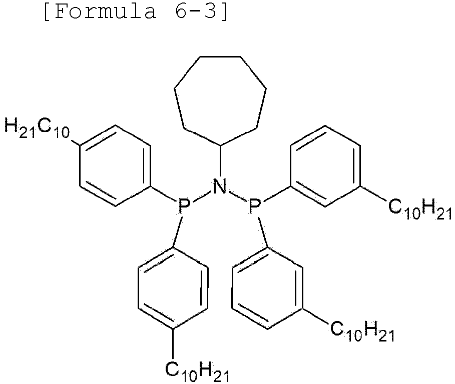

¹H NMR (500 MHz, DMSO) : δ 7.38-7.05 (m, 16H), 2.67-2.46(m, 9H), 1.70-1.28 (m, 76H), 0.89(m, 12H)

### Synthesis Example 44: Synthesis of ligand compound represented by Formula 6-4

1.40 g (11 mmol, 2.2 eq) of cyclooctylamine and 10 ml of dichloromethane were introduced into a dried flask under a nitrogen atmosphere and stirred. 2.51 g (5 mmol, 1 eq) of chlorobis(3-decylphenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 2.96 g (5 mmol) of N-cyclooctyl-1,1-bis(3-decylphenyl)phosphanamine, which is Intermediate Compound 24, was obtained through column chromatography.

### <Intermediate Compound 24>

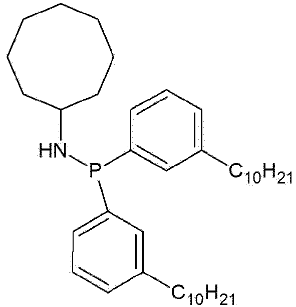

¹H NMR (500 MHz, DMSO): δ 7.40 (m, 2H), 7.32 (m, 2H), 7.24(m, 2H), 7.07(m, 2H), 2.73 (m, 4H), 2.50 (m, 1H), 1.67(m, 4H), 1.61(m, 4H), 1.58(m, 1H), 1.39(m, 2H), 1.34(m, 32H), 1.30(m, 4H), 0.93(m, 6H)

Subsequently, 2.96 g (5 mmol, 1 eq) of N-cyclooctyl-1,1-bis(3-decylphenyl)phosphanamine, which is Intermediate Compound 24 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.51 g (5 mmol, 1 eq) of chlorobis(4-decylphenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.05 g (3.84 mmol, yield 77%) of N-(bis(3-decylphenyl)phosphaneyl)-N-cyclooctyl-1,1-bis(4-decylphenyl)phosphanamine, which is a compound represented by Formula 6-4, was obtained through column chromatography.

### <Ligand compound represented by Formula 6-4>

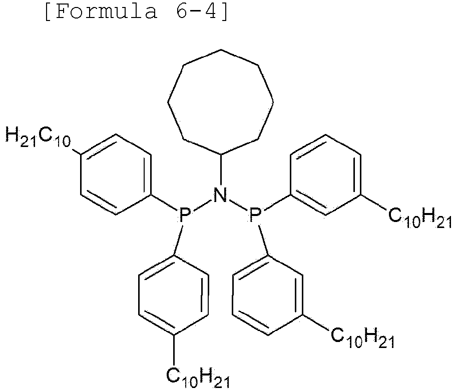

¹H NMR (500 MHz, DMSO) : δ 7.35 (m, 2H), 7.32 (m, 2H), 7.28(m, 2H), 7.24(m, 4H), 7.17(m, 4H), 7.13 (m, 2H), 2.65(m, 8H), 2.46(m, 1H), 1.69-1.35(m, 78H), 0.97 (m, 12H)

### Synthesis Example 45: Synthesis of ligand compound represented by Formula 6-5

1.47 g (11 mmol, 2.2 eq) of 2,3-dihydro-1H-inden-2-yl)amine and 10 ml of dichloromethane were introduced into a dried flask under a nitrogen atmosphere and stirred. 2.51 g (5 mmol, 1 eq) of chlorobis(3-decylphenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 2.99 g (5 mmol) of N-(2,3-dihydro-1H-inden-2-yl)-1,1-bis(3-decylphenyl)phosphanamine, which is Intermediate Compound 25, was obtained through column chromatography.

### <Intermediate Compound 25>

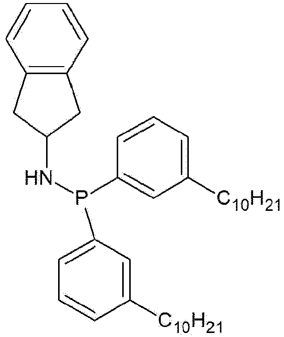

¹H NMR (500 MHz, DMSO) : δ 7.43 (m, 2H), 7.29(m, 2H), 7.26(m, 4H), 7.09 (m, 4H), 3.21(m, 1H), 3.17(m, 4H), 2.64(m, 4H), 1.61(m, 4H), 1.53(m, 1H), 1.35(m, 28H), 0.91(m, 6H)

Subsequently, 2.99 g (5 mmol, 1 eq) of N-(2,3-dihydro-1H-inden-2-yl)-1,1-bis(3-decylphenyl)phosphanamine, which is Intermediate Compound 25 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.51 g (5 mmol, 1 eq) of chlorobis(4-decylphenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.32 g (4.07 mmol, yield 81%) of N-(bis(3-decylphenyl)phosphaneyl)-N-(2,3-dihydro-1H-inden-2-yl)-1,1-bis(4-decylphenyl)phosphanamine, a compound represented by Formula 6-5, was obtained through column chromatography.

### <Ligand compound represented by Formula 6-5>

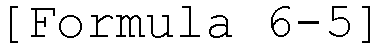

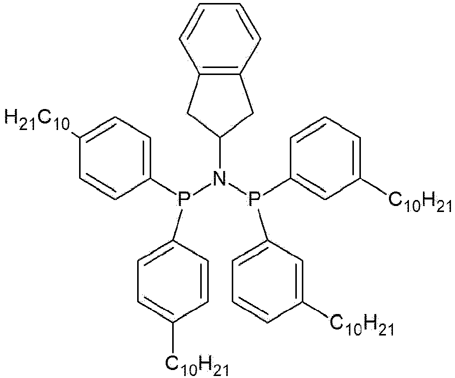

¹H NMR (500 MHz, DMSO) : δ 7.35-7.09 (m, 20H), 3.21(m, 1H), 3.14(m, 4H), 2.64(m, 8H), 1.72-1.28(m, 64H), 0.93 (m, 12H)

### Synthesis Example 46: Synthesis of ligand compound represented by Formula 6-6

1.62 g (11 mmol, 2.2 eq) of 1,2,3,4-tetrahydronaphthalen-2-yl)amine and 10 ml of dichloromethane were introduced into a dried flask under a nitrogen atmosphere and stirred. 2.51 g (5 mmol, 1 eq) of chlorobis(3-decylphenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 3.06 g (5 mmol) of N-(1,2,3,4-tetrahydronaphthalen-2-yl)-1,1-bis(3-decylphenyl)phosphanamine, which is Intermediate Compound 26, was obtained through column chromatography.

### <Intermediate Compound 26>

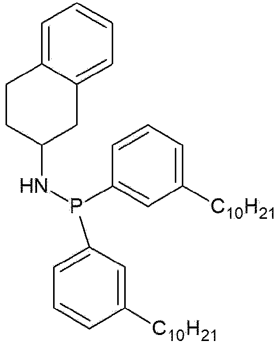

¹H NMR (500 MHz, DMSO) : δ 7.35 (m, 2H), 7.32 (m, 2H), 7.27 (m, 2H), 7.09 (m, 2H), 6.92(m, 4H), 3.28 (m, 2H), 2.90(m, 2H), 2.74(m, 1H), 2.72(m, 4H), 2.02(m, 2H), 1.68 (m, 4H), 1.56 (m, 1H), 1.33(m, 28H), 0.93(m, 6H)

Subsequently, 3.06 g (5 mmol, 1 eq) of N-(1,2,3,4-tetrahydronaphthalen-2-yl)-1,1-bis(3-decylphenyl)phosphanamine, which is Intermediate Compound 26 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.51 g (5 mmol, 1 eq) of chlorobis(4-decylphenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 3.95 g (3.67 mmol, yield 73%) of N-(bis (3-decylphenyl)phosphaneyl)-N-(1,2,3,4-tetrahydronaphthalen-2-yl)-1,1-bis(4-decylphenyl)phosphanamine, which is a compound represented by Formula 6-6, was obtained through column chromatography.

### <Ligand compound represented by Formula 6-6>

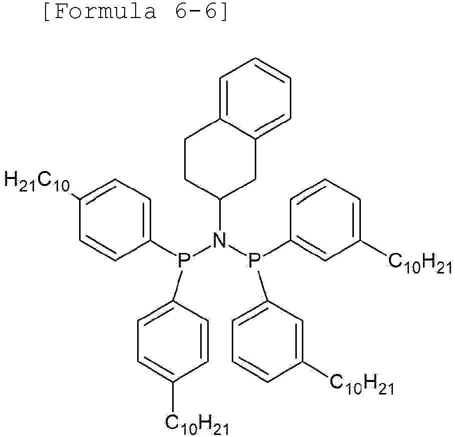

¹H NMR (500 MHz, DMSO) : δ 7.39-6.96 (m, 20H), 3.26 (m, 2H), 2.96(m, 2H), 2.80(m, 1H), 2.69(m, 8H), 2.03 (m, 2H), 1.70-1.29 (m, 64H), 0.95 (m, 12H)

### Synthesis Example 47: Synthesis of ligand compound represented by Formula 6-7

0.65 g (11 mmol, 2.2 eq) of isopropylamine and 10 ml of dichloromethane were introduced into a dried flask under a nitrogen atmosphere and stirred. 2.51 g (5 mmol, 1 eq) of chlorobis(3-decylphenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 2.62 g (5 mmol) of N-isopropyl-1,1-bis(3-decylphenyl)phosphanamine, which is Intermediate Compound 27, was obtained through column chromatography.

### <Intermediate Compound 27>

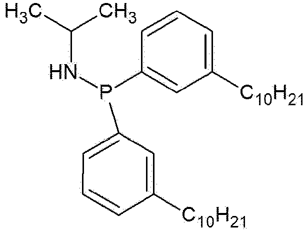

¹H NMR (500 MHz, DMSO) : δ 7.41 (m, 2H), 7.28 (m, 2H), 7.26(m, 2H), 7.08 (m, 2H), 2.87(m, 1H), 2.72 (m, 4H), 1.63(m, 4H), 1.57(m, 1H), 1.29(m, 28H), 1.11(m, 6H), 0.91(m, 6H)

Subsequently, 2.62 g (5 mmol, 1 eq) of N-isopropyl-1,1-bis(3-decylphenyl)phosphanamine, which is Intermediate Compound 27 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.51 g (5 mmol, 1 eq) of chlorobis(4-decylphenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 2.67 g (2.70 mmol, yield 54%) of N-(bis (3-decylphenyl)phosphaneyl)-N-isopropyl-1,1-bis(4-decylphenyl)phosphanamine, which is a compound represented by Formula 6-7, was obtained through column chromatography.

### <Ligand compound represented by Formula 6-7>

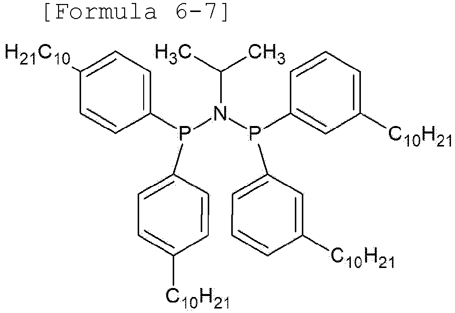

¹H NMR (500 MHz, DMSO): δ 7.43-7.11(m, 16H), 2.91(m, 1H), 2.65(m, 8H), 1.67-1.08(m, 70H), 0.92(m, 12H)

### Synthesis Example 48: Synthesis of ligand compound represented by Formula 6-8

0.96 g (11 mmol, 2.2 eq) of 3-methylbutan-2-yl)amine and 10 ml of dichloromethane were introduced into a dried flask under a nitrogen atmosphere and stirred. 2.51 g (5 mmol, 1 eq) of chlorobis(3-decylphenyl)phosphane diluted in 10 ml of dichloromethane was slowly introduced into the flask. A reactant was stirred for 4 hours, and conversion was confirmed through ¹H NMR. After the reaction was completed solids were removed through a filter, the solvent was removed by decompression, and 2.76 g (5 mmol) of N-(3-methylbutan-2-yl)-1,1-bis(3-decylphenyl)phosphanamine, which is Intermediate Compound 28, was obtained through column chromatography.

### <Intermediate Compound 28>

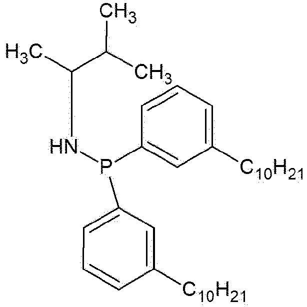

¹H NMR (500 MHz, DMSO) : δ 7.39 (m, 2H), 7.31(m, 2H), 7.29(m, 2H), 7.08(m, 2H), 2.66(m, 4H), 2.61(m, 1H), 1.61(m, 4H), 1.60(m, 1H), 1.57(m, 1H), 1.27(m, 28H), 1.14(m, 3H), 0.92(m, 12H)

Subsequently, 2.76 g (5 mmol, 1 eq) of N-(3-methylbutan-2-yl)-1,1-bis(3-decylphenyl)phosphanamine, which is Intermediate Compound 28 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.51 g (5 mmol, 1 eq) of chlorobis(4-decylphenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 3.62 g (3.56 mmol, yield 71%) of N-(bis(3-decylphenyl)phosphaneyl)-N-(3-methylbutan-2-yl)-1,1-bis(4-decylphenyl)phosphanamine, which is a compound represented by Formula 6-8, was obtained through column chromatography.

### <Ligand compound represented by Formula 6-8>

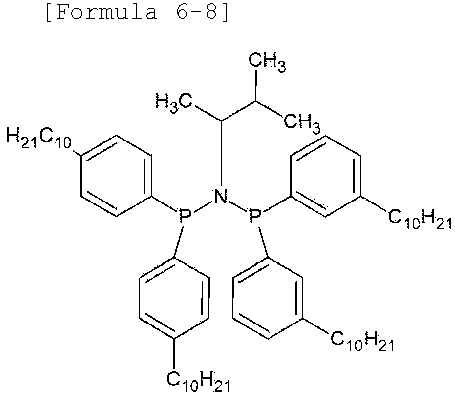

¹H NMR (500 MHz, DMSO): δ 7.42-7.12(m, 16H), 2.73 (m, 8H), 2.56(m, 1H), 1.66-1.09(m, 68H), 0.89(m, 18H)

### Synthesis Example 49: Synthesis of ligand compound represented by Formula 6-9

1.18 g (11 mmol, 2.2 eq) of benzylamine and 10 ml of dichloromethane were introduced into a dried flask under a nitrogen atmosphere and stirred. 2.51 g (5 mmol, 1 eq) of chlorobis(3-decylphenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 2.86 g (5 mmol) of N-benzyl-1,1-bis(3-decylphenyl)phosphanamine, which is Intermediate Compound 29, was obtained through column chromatography.

### <Intermediate Compound 29>

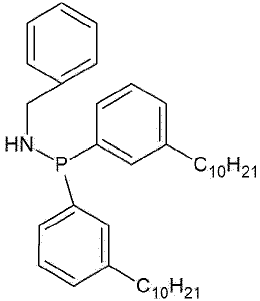

¹H NMR (500 MHz, DMSO) : δ 7.42 (m, 2H), 7.38 (m, 5H), 7.26(m, 4H), 7.14 (m, 2H), 3.94 (m, 2H), 2.67 (m, 4H), 2.39(m, 1H), 1.67(m, 4H), 1.35(m, 28H), 0.95 (m, 6H)

Subsequently, 2.86 g (5 mmol, 1 eq) of N-benzyl-1,1-bis(3-decylphenyl)phosphanamine, which is Intermediate Compound 29 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.51 g (5 mmol, 1 eq) of chlorobis(4-decylphenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.19 g (4.05 mmol, yield 81%) of N-(bis(3-decylphenyl)phosphaneyl)-N-benzyl-1,1-bis(4-decylphenyl)phosphanamine, which is a compound represented by Formula 6-9, was obtained through column chromatography.

### <Ligand compound represented by Formula 6-9>

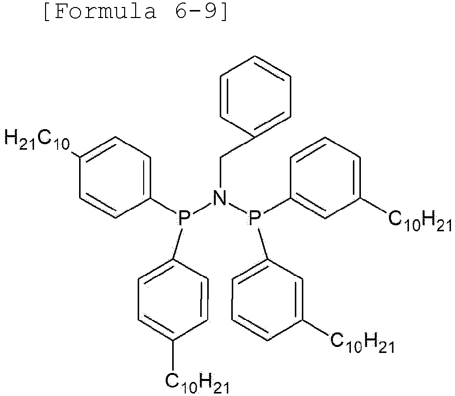

¹H NMR (500 MHz, DMSO) : δ 7.37-7.06 (m, 21H), 3.95 (m, 2H), 2.67 (m, 8H), 1.69-1.34 (m, 64H), 0.93 (m, 12H)

### Synthesis Example 50: Synthesis of ligand compound represented by Formula 6-10

1.33 g (11 mmol, 2.2 eq) of phenethylamine and 10 ml of dichloromethane were introduced into a dried flask under a nitrogen atmosphere and stirred. 2.51 g (5 mmol, 1 eq) of chlorobis(3-decylphenyl)phosphane diluted in 10 ml of dichloromethane was slowly introduced into the flask. A reactant was stirred for 4 hours, and conversion was confirmed through ¹H NMR. After the reaction was completed, precipitated solids were removed through a filter, the solvent was removed by decompression, and 2.92 g (5 mmol) of N-phenethyl-1,1-bis(3-decylphenyl)phosphanamine, which is Intermediate Compound 30, was obtained through column chromatography.

### <Intermediate Compound 30>

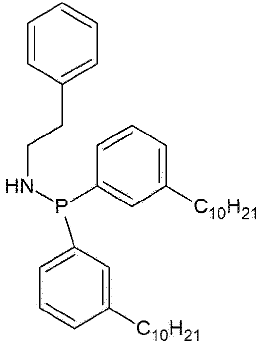

¹H NMR (500 MHz, DMSO) : δ 7.36 (m, 2H), 7.33 (m, 4H), 7.28(m, 2H), 7.23(m, 2H), 7.18(m, 1H), 7.08(m, 2H), 3.04 (m, 2H), 2.83 (m, 2H), 2.72 (m, 4H), 1.66(m, 4H), 1.50 (m, 1H), 1.29 (m, 28H), 0.96 (m, 6H)

Subsequently, 2.92 g (5 mmol, 1 eq) of N-phenethyl-1,1-bis(3-decylphenyl)phosphanamine, which is Intermediate Compound 30 obtained above, and 13 ml of methyl t-butyl ether were introduced into a dried flask under a nitrogen atmosphere, cooled to -78 °C, and then stirred. 2.1 ml (5.25 mmol, 1.05 eq) of a solution in which n-butyl lithium was dissolved in hexane at 2.5 M was slowly introduced into the flask, stirred for 1 hour, and then 2.51 g (5 mmol, 1 eq) of chlorobis(4-decylphenyl)phosphane diluted in 10 ml of methyl t-butyl ether was slowly introduced thereto. A reactant was stirred for 4 hours, and conversion was confirmed through ¹H NMR. After the reaction was completed, precipitated solids were removed through a filter, the solvent was removed by decompression, and 4.40 g (4.19 mmol, yield 84%) of N-(bis(3-decylphenyl)phosphaneyl)-N-phenethyl-1,1-bis(4-decylphenyl)phosphanamine, which is a compound represented by Formula 6-10, was obtained through column chromatography.

### <Ligand compound represented by Formula 6-10>

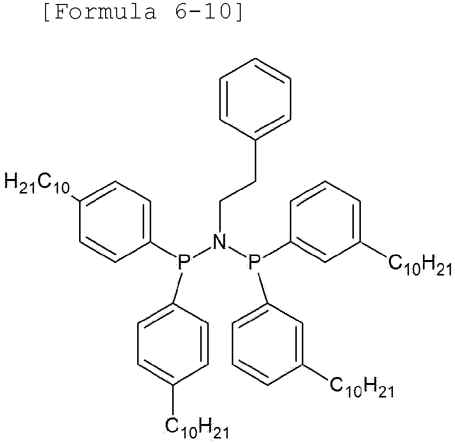

¹H NMR (500 MHz, DMSO) : δ 7.35-7.09 (m, 21H), 3.06 (m, 2H), 2.87(m, 2H), 2.65(m, 8H), 1.66-1.31(m, 64H), 0.8 (m, 12H)

### Comparative Synthesis Example 1: Synthesis of ligand compound represented by Formula 11

0.55 g(5.5 mmol, 0.5 eq) of cyclohexylamine, 2.23 g (22 mmol, 2 eq) of triethylamine, and 10 ml of dichloromethane were introduced into a dried flask under a nitrogen atmosphere and stirred. Subsequently, the mixture was cooled to 0 °C, and then 4.29 g (11 mmol, 1 eq) of chlorobis(4-cyclohexylphenyl)phosphane was slowly introduced thereto. After the reaction was completed, precipitated solids were removed through a filter, the solvent was removed by decompression, and 2.50 g (3.25 mmol, yield 65%) of N-(bis(4-cyclohexylphenyl)phosphaneyl)-N-cyclohexyl-1,1-bis(4-cyclohexlyphenyl)phosphanamine, which is a compound represented by Formula 11, was obtained through column chromatography.

### <Ligand compound represented by Formula 11>

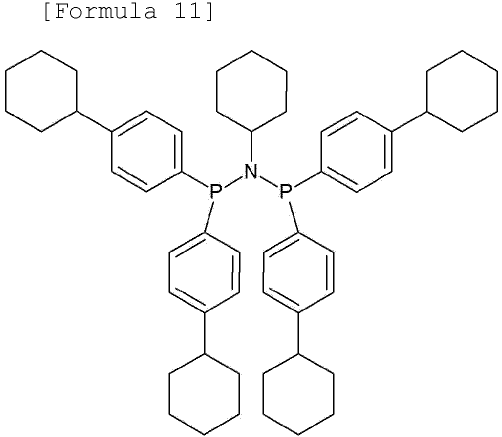

¹H NMR (500 MHz, C₆D₆) : δ 7.58(br. s, 8H), 7.08 (d, 8H), 3.47 (pent, 1H), 2.35(t, 4H), 2.21-2.11(m, 2H), 1.80 (d, 8H), 1.71-1.56(m, 16H), 1.44-1.11(m, 24H)

### Comparative Synthesis Example 2: Synthesis of ligand compound represented by Formula 12

0.55 g(5.5 mmol, 0.5 eq) of cyclohexylamine, 2.23 g (22 mmol, 2 eq) of triethylamine, and 10 ml of dichloromethane were introduced into a dried flask under a nitrogen atmosphere and stirred. Subsequently, the mixture was cooled to 0 °C, and then 4.56 g (11 mmol, 1 eq) of di([1,1'-biphenyl]-4-yl)chlorophosphane was slowly introduced thereto. After the reaction was completed, precipitated solids were removed through a filter, the solvent was removed by decompression, and 2.78 g (3.60 mmol, yield 72%) of di([1,1'-biphenyl]-4-yl)-N-cyclohexyl-N-(di([1,1'-biphenyl]-4-yl)phosphaneyl)phosphanamine, which is a compound represented by Formula 12, was obtained through column chromatography.

### <Ligand compound represented by Formula 12>

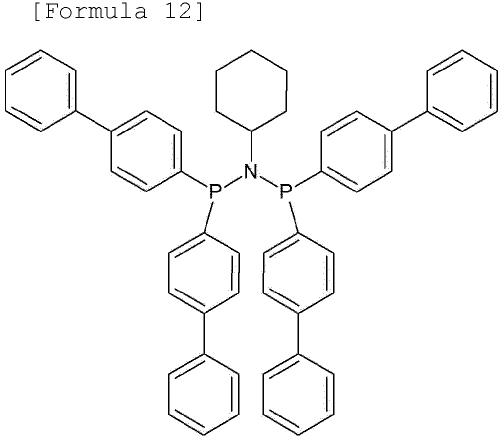

¹H NMR (500 MHz, C₆D₆) : δ 7.92-7.42 (m, 24H), 7.17 (t, 8H), 7.10(t, 4H), 3.58 (pent, 1H), 2.28-2.17(m, 2H), 1.84-1.77 (m, 2H), 1.67-1.60(m, 2H), 1.48-1.42(m, 1H), 1.18-1.07 (m, 3H)

### Comparative Synthesis Example 3: Synthesis of ligand compound represented by Formula 13

0.55 g (5.5 mmol, 0.5 eq) of cyclohexylamine, 2.23 g (22 mmol, 2 eq) of triethylamine, and 10 ml of dichloromethane were introduced into a dried flask under a nitrogen atmosphere and stirred. Subsequently, the mixture was cooled to 0 °C, and then 3.38 g (11 mmol, 1 eq) of chlorobis(3,5-dimethylphenyl)phosphane was slowly introduced thereto. After the reaction was completed, precipitated solids were removed through a filter, the solvent was removed by decompression, and 2.32 g (4 mmol, yield 80%) of N-(bis(3,5-dimethylphenyl)phosphaneyl)-N-cyclohexyl-1,1-bis(3,5-dimethylphenyl)phosphanamine, which is a compound represented by Formula 12, was obtained through column chromatography.

### <Ligand compound represented by Formula 13>

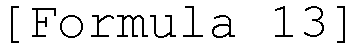

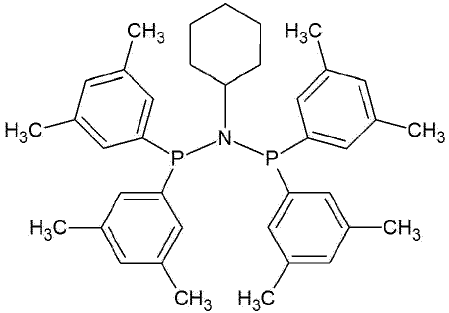

¹H NMR (500 MHz, C₆D₆) : δ 7.38(br.s, 8H), 6.79 (s, 4H), 3.63 (pent, 1H), 2.24-2.13(m, 2H), 2.10(s, 24H), 1.77(d, 2H), 1.60(d, 2H), 1.45-1.39(m, 1H), 1.19-1.00(m, 3H)

### Examples and Comparative Examples

### Example 1

Under an argon gas atmosphere, 17.5 mg (0.05 mmol) of chromium(III) acetylacetonate (Cr(acac)₃) and 0.5 mmol of the ligand compound represented by Formula 2-1 according to Synthesis Example 1 were put into a flask, and then 100 ml of methylcyclohexane was introduced thereto and stirred to prepare a 5 mM catalyst solution (based on Cr).

A Parr reactor with a capacity of 600 ml was prepared and vacuumed at 120 °C for 2 hours, and then the inside of the reactor was substituted with argon and the temperature was lowered to 80 °C. Subsequently, 180 ml of methylcyclohexane and 2 ml of an isoheptane solution (MMAO) (Al/Cr=600) were injected thereto, and 2 ml of the catalyst solution (1.0 µmol Cr) was injected thereto. After two minutes of stirring at 1,000 rpm, the valve of an ethylene line which was set to 30 bar was opened to fill the reactor with ethylene, and then stirring was performed for 60 minutes at 1,000 rpm. The ethylene line valve was closed, and the reactor was cooled to 0 °C using a dry ice/acetone bath, and then unreacted ethylene was slowly vented, and 0.5 ml of nonane (GC internal standard) was added thereto. After stirring was performed for 10 seconds, 2 ml of a liquid portion of the reactor was taken and quenched with water, and an obtained organic portion was filtered with a PTFE syringe filter to prepare a GC-FID sample. Then, the distribution of a liquid product was analyzed by GC (Agilent Co., 6890N, Alltech AT-5 (30 m X 0.32 mm ID X 0.25 µm; series no. 12446)). In addition, 400 ml of ethanol/HCl (10 vol% of aqueous 12 M HCl solution) was added to the remaining reaction solution, stirred, and filtered to analyze the amount of a solid product. The obtained polymer was dried in a 80 °C vacuum oven overnight.

### Examples 2 to 50 and Comparative Examples 1 to 3

Examples 2 to 50 and Comparative Examples 1 to 3 were executed in the same manner as in Example 1 above, except that the catalyst type was changed as shown in Table 1 below.

**[Table 1]**

| Classification | Ligand compound | Co-catalyst | Chromium source |
|---|---|---|---|
| Example 1 | Synthesis Example 1 (Formula 2-1) | MMAO | Cr(acac)₃ |
| Example 2 | Synthesis Example 2 (Formula 2-2) | MMAO | Cr(acac)₃ |
| Example 3 | Synthesis Example 3 (Formula 2-3) | MMAO | Cr(acac)₃ |
| Example 4 | Synthesis Example 4 (Formula 2-4) | MMAO | Cr(acac)₃ |
| Example 5 | Synthesis Example 5 (Formula 2-5) | MMAO | Cr(acac)₃ |
| Example 6 | Synthesis Example 6 (Formula 2-6) | MMAO | Cr(acac)₃ |
| Example 7 | Synthesis Example 7 (Formula 2-7) | MMAO | Cr(acac)₃ |
| Example 8 | Synthesis Example 8 (Formula 2-8) | MMAO | Cr(acac)₃ |
| Example 9 | Synthesis Example 9 (Formula 2-9) | MMAO | Cr(acac)₃ |
| Example 10 | Synthesis Example 10 (Formula 2-10) | MMAO | Cr(acac)₃ |
| Example 11 | Synthesis Example 11 (Formula 3-1) | MMAO | Cr(acac)₃ |
| Example 12 | Synthesis Example 12 (Formula 3-2) | MMAO | Cr(acac)₃ |
| Example 13 | Synthesis Example 13 (Formula 3-3) | MMAO | Cr(acac)₃ |
| Example 14 | Synthesis Example 14 (Formula 3-4) | MMAO | Cr(acac)₃ |
| Example 15 | Synthesis Example 15 (Formula 3-5) | MMAO | Cr(acac)₃ |
| Example 16 | Synthesis Example 16 (Formula 3-6) | MMAO | Cr(acac)₃ |
| Example 17 | Synthesis Example 17 (Formula 3-7) | MMAO | Cr(acac)₃ |
| Example 18 | Synthesis Example 18 (Formula 3-8) | MMAO | Cr(acac)₃ |
| Example 19 | Synthesis Example 19 (Formula 3-9) | MMAO | Cr(acac)₃ |
| Example 20 | Synthesis Example 20 (Formula 3-10) | MMAO | Cr(acac)₃ |
| Example 21 | Synthesis Example 21 (Formula 4-1) | MMAO | Cr(acac)₃ |
| Example 22 | Synthesis Example 22 (Formula 4-2) | MMAO | Cr(acac)₃ |
| Example 23 | Synthesis Example 23 (Formula 4-3) | MMAO | Cr(acac)₃ |
| Example 24 | Synthesis Example 24 (Formula 4-4) | MMAO | Cr(acac)₃ |
| Example 25 | Synthesis Example 25 (Formula 4-5) | MMAO | Cr(acac)₃ |
| Example 26 | Synthesis Example 26 (Formula 4-6) | MMAO | Cr(acac)₃ |
| Example 27 | Synthesis Example 27 (Formula 4-7) | MMAO | Cr(acac)₃ |
| Example 28 | Synthesis Example 28 (Formula 4-8) | MMAO | Cr(acac)₃ |
| Example 29 | Synthesis Example 29 (Formula 4-9) | MMAO | Cr(acac)₃ |
| Example 30 | Synthesis Example 30 (Formula 4-10) | MMAO | Cr(acac)₃ |
| Example 31 | Synthesis Example 31 (Formula 5-1) | MMAO | Cr(acac)₃ |
| Example 32 | Synthesis Example 32 (Formula 5-2) | MMAO | Cr(acac)₃ |
| Example 33 | Synthesis Example 33 (Formula 5-3) | MMAO | Cr(acac)₃ |
| Example 34 | Synthesis Example 34 (Formula 5-4) | MMAO | Cr(acac)₃ |
| Example 35 | Synthesis Example 35 (Formula 5-5) | MMAO | Cr(acac)₃ |
| Example 36 | Synthesis Example 36 (Formula 5-6) | MMAO | Cr(acac)₃ |
| Example 37 | Synthesis Example 37 (Formula 5-7) | MMAO | Cr(acac)₃ |
| Example 38 | Synthesis Example 38 (Formula 5-8) | MMAO | Cr(acac)₃ |
| Example 39 | Synthesis Example 39 (Formula 5-9) | MMAO | Cr(acac)₃ |
| Example 40 | Synthesis Example 40 (Formula 5-10) | MMAO | Cr(acac)₃ |
| Example 41 | Synthesis Example 41 (Formula 6-1) | MMAO | Cr(acac)₃ |
| Example 42 | Synthesis Example 42 (Formula 6-2) | MMAO | Cr(acac)₃ |
| Example 43 | Synthesis Example 43 (Formula 6-3) | MMAO | Cr(acac)₃ |
| Example 44 | Synthesis Example 44 (Formula 6-4) | MMAO | Cr(acac)₃ |
| Example 45 | Synthesis Example 45 (Formula 6-5) | MMAO | Cr(acac)₃ |
| Example 46 | Synthesis Example 46 (Formula 6-6) | MMAO | Cr(acac)₃ |
| Example 47 | Synthesis Example 47 (Formula 6-7) | MMAO | Cr(acac)₃ |
| Example 48 | Synthesis Example 48 (Formula 6-8) | MMAO | Cr(acac)₃ |
| Example 49 | Synthesis Example 49 (Formula 6-9) | MMAO | Cr(acac)₃ |
| Example 50 | Synthesis Example 50 (Formula 6-10) | MMAO | Cr(acac)₃ |
| Comparative Example 1 | Comparative Synthesis Example 1 (Formula 11) | MMAO | Cr(acac)₃ |
| Comparative Example 2 | Comparative Synthesis Example 2 (Formula 12) | MMAO | Cr(acac)₃ |
| Comparative Example 3 | Comparative Synthesis Example 3 (Formula 13) | MMAO | Cr(acac)₃ |

### Experimental Examples

The results of the ethylene oligomerization reaction according to Examples and Comparative examples above are shown in Table 2 below.
* Catalytic activity (ton/mol· Cr/hr) : From a total product weight (ton) value, which is the sum of the weight (ton) of the obtained liquid product and the weight of the obtained solid product, the catalytic activity was calculated.
* 1-C6 and 1-C8 selectivity (wt%): From the result of analyzing the distribution of the liquid product by GC, the contents of 1-hexene (1-C6) and 1-octene (1-C8) were calculated to calculate the wt% of 1-hexene or 1-octene with respect to the total weight of the product.
* Solid (wt%): The wt% of the solid product with respect to the total weight of the product was calculated. This represents the degree to which polyethylene having a carbon number of about 40 or more was generated as an insoluble solid which was not dissolved in the solvent.

**[Table 2]**

| Classif ication | Catalytic activity (ton/mol ·Cr/hr) | 1-C6 and 1-C8 selectivity | | | Solid (wt%) |
|---|---|---|---|---|---|
| | | 1-C6 (wt%) | 1-C8 (wt%) | Total (wt%) | |
| Example 1 | 158 | 26.2 | 57.6 | 83.8 | 0.69 |
| Example 2 | 196 | 25.4 | 62.1 | 87.5 | 0.41 |
| Example 3 | 221 | 27.1 | 63.0 | 90.1 | 0.49 |
| Example 4 | 132 | 30.2 | 58.4 | 88.6 | 0.45 |
| Example 5 | 172 | 25.9 | 58.3 | 84.2 | 0.49 |
| Example 6 | 203 | 26.0 | 61.2 | 87.2 | 0.48 |
| Example 7 | 179 | 26.3 | 61.7 | 88.0 | 0.55 |
| Example 8 | 161 | 29.9 | 58.1 | 88.0 | 0.64 |
| Example 9 | 126 | 19.8 | 66.0 | 85.8 | 0.63 |
| Example 10 | 105 | 17.3 | 65.8 | 83.1 | 0.32 |
| Example 11 | 173 | 25.9 | 58.5 | 84.4 | 0.49 |
| Example 12 | 205 | 25.6 | 63.0 | 88.6 | 0.37 |
| Example 13 | 241 | 26.9 | 62.7 | 89.6 | 0.57 |
| Example 14 | 154 | 30.0 | 58.6 | 88.6 | 0.45 |
| Example 15 | 197 | 26.1 | 58.1 | 84.2 | 0.51 |
| Example 16 | 221 | 25.8 | 60.7 | 86.5 | 0.30 |
| Example 17 | 189 | 26.1 | 61.5 | 87.6 | 0.50 |
| Example 18 | 181 | 30.0 | 57.8 | 87.8 | 0.60 |
| Example 19 | 151 | 20.6 | 65.6 | 86.2 | 0.64 |
| Example 20 | 120 | 18.1 | 66.4 | 84.5 | 0.60 |
| Example 21 | 166 | 25.9 | 56.4 | 82.3 | 0.42 |
| Example 22 | 203 | 24.9 | 61.0 | 85.9 | 0.35 |
| Example 23 | 226 | 26.6 | 62.8 | 89.4 | 0.34 |
| Example 24 | 141 | 31.0 | 58.1 | 89.1 | 0.42 |
| Example 25 | 187 | 26.3 | 58.5 | 84.8 | 0.62 |
| Example 26 | 222 | 25.7 | 60.9 | 86.6 | 0.63 |
| Example 27 | 194 | 25.4 | 61.5 | 86.9 | 0.47 |
| Example 28 | 169 | 30.2 | 57.8 | 88.8 | 0.36 |
| Example 29 | 128 | 20.2 | 65.7 | 85.9 | 0.61 |
| Example 30 | 112 | 16.8 | 66.3 | 83.1 | 0.67 |
| Example 31 | 167 | 26.1 | 56.8 | 82.9 | 0.61 |
| Example 32 | 201 | 25.3 | 60.7 | 86.0 | 0.47 |
| Example 33 | 225 | 26.3 | 62.1 | 88.4 | 0.67 |
| Example 34 | 143 | 30.7 | 57.8 | 88.5 | 0.68 |
| Example 35 | 187 | 25.8 | 58.3 | 84.1 | 0.31 |
| Example 36 | 220 | 25.8 | 60.6 | 86.4 | 0.48 |
| Example 37 | 197 | 25.3 | 61.2 | 86.5 | 0.48 |
| Example 38 | 172 | 30.1 | 57.7 | 87.8 | 0.39 |
| Example 39 | 126 | 20.4 | 65.4 | 85.8 | 0.38 |
| Example 40 | 104 | 17.1 | 66.0 | 83.1 | 0.58 |
| Example 41 | 121 | 25.9 | 58.4 | 84.3 | 0.55 |
| Example 42 | 147 | 25.1 | 61.8 | 86.9 | 0.68 |
| Example 43 | 176 | 26.8 | 62.3 | 89.1 | 0.33 |
| Example 44 | 108 | 29.7 | 57.9 | 87.6 | 0.60 |
| Example 45 | 143 | 26.2 | 58.4 | 84.6 | 0.33 |
| Example 46 | 177 | 25.4 | 60.9 | 86.3 | 0.54 |
| Example 47 | 146 | 26.1 | 61.5 | 87.6 | 0.64 |
| Example 48 | 131 | 30.2 | 57.8 | 88.0 | 0.40 |
| Example 49 | 108 | 20.3 | 65.5 | 85.8 | 0.69 |
| Example 50 | 81 | 18.4 | 65.1 | 83.5 | 0.53 |
| Compara tive Example 1 | 42 | 45.7 | 43.0 | 88.7 | 1.2 |
| Compara tive Example 2 | 0 | - | - | - | - |
| Compara tive Example 3 | 0 | - | - | - | - |

As shown in Table 2 above, when an ethylene oligomerization reaction is performed using a catalyst composition including the ligand compound according to the present invention, it can be confirmed that phenyl positioned at the end of the diphosphino aminyl moiety had, as a substituent, an alkyl group of a specific carbon number, or a silyl group substituted with an alkyl group of a specific carbon number, at a meta position and a para position, in an asymmetric form, and that catalytic activity, selectivity, and stability were all improved by controlling the steric strain around a P-N-P functional group from a substituent substituted on the nitrogen atom.

On the other hand, in the case of Comparative Examples 2 and 3, the catalyst exhibited no activity, so that it was impossible to perform ethylene oligomerization.

From the above-described results, it can be confirmed that when ethylene oligomerization is performed using an organic chromium compound including the ligand compound of the present invention and the catalyst composition, it is possible to manufacture linear alpha-olefins with high 1-hexene and 1-octene selectivity while having excellent productivity due to high catalytic activity.

## Claims

1. A ligand compound represented by Formula 1 below: 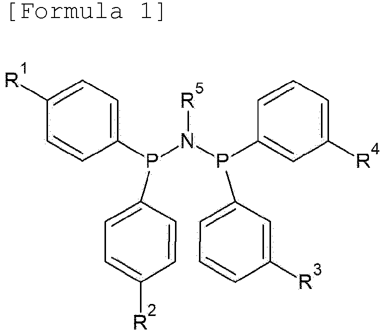 wherein in Formula 1 above,
R¹ to R⁴ are each independently an alkyl group having 5 to 20 carbon atoms, a cycloalkyl group having 5 to 20 carbon atoms, an alkoxyalkyl group having 6 to 20 carbon atoms, an arylalkoxyalkyl group having 10 to 30 carbon atoms, or a trialkylsilyl group, wherein the alkyl groups of the trialkylsilyl group are each independently an alkyl group having 1 to 10 carbon atoms, and
R⁵ is an alkyl group having 1 to 20 carbon atoms, an alkyl group having 1 to 20 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.

2. The ligand compound of claim 1, wherein R¹ to R⁴ are each independently an alkyl group having 8 to 12 carbon atoms, a tripropylsilyl group, or a tributylsilyl group.

3. The ligand compound of claim 1, wherein R⁵ is an alkyl group having 3 to 5 carbon atoms, an alkyl group having 1 to 5 carbon atoms substituted with an aryl group having 6 to 10 carbon atoms, a cycloalkyl group having 5 to 8 carbon atoms, or a cycloalkyl group having 5 to 8 carbon atoms fused with an aryl group having 6 to 10 carbon atoms.

4. The ligand compound of claim 1, wherein the ligand compound represented by Formula 1 above is represented by Formula 2 below: 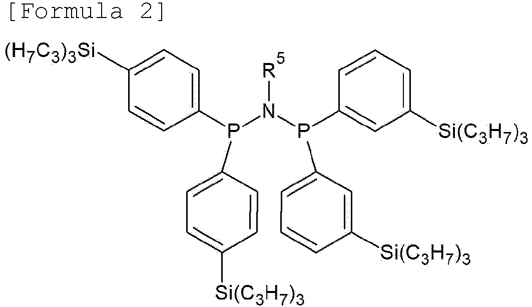 wherein in Formula 2 above,
R⁵ is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.

5. The ligand compound of claim 4, wherein the ligand compound represented by Formula 2 above is one selected from the group consisting of ligand compounds represented by Formula 2-1 to Formula 2-10 below:

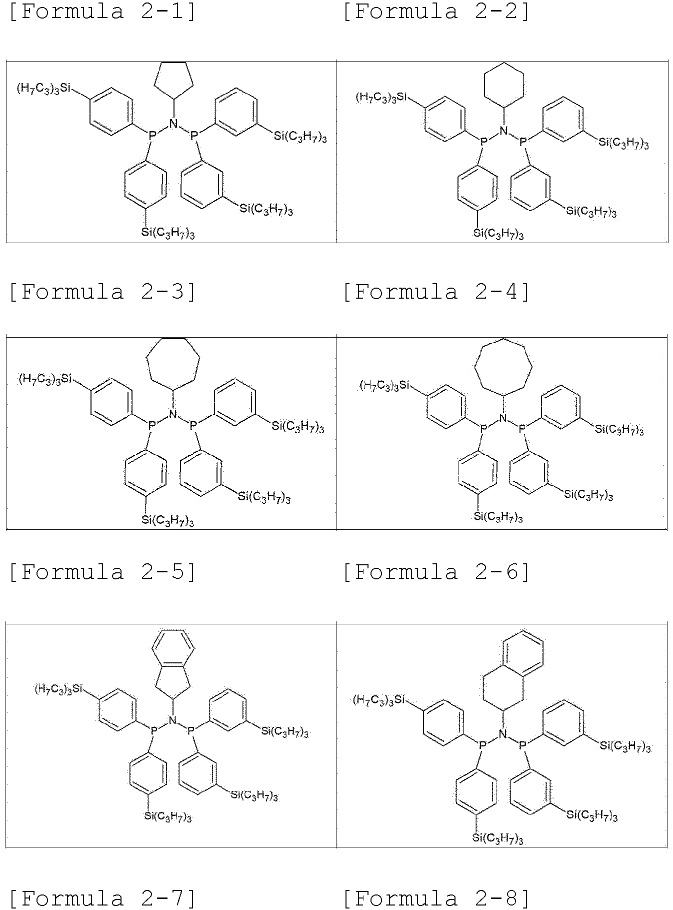

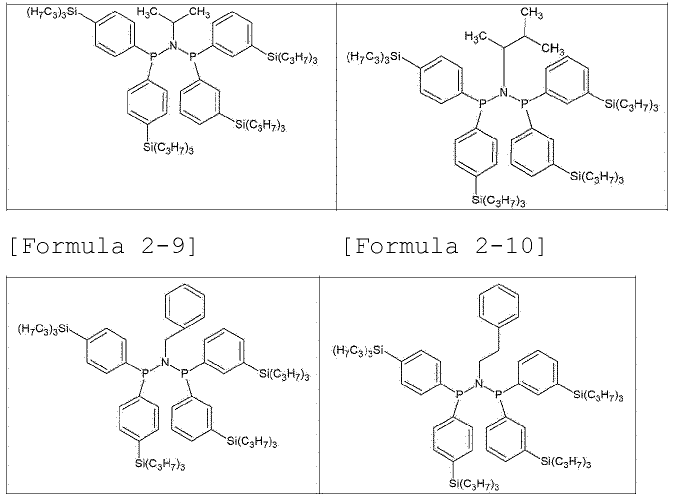

6. The ligand compound of claim 1, wherein the ligand compound represented by Formula 1 above is represented by Formula 3 below: 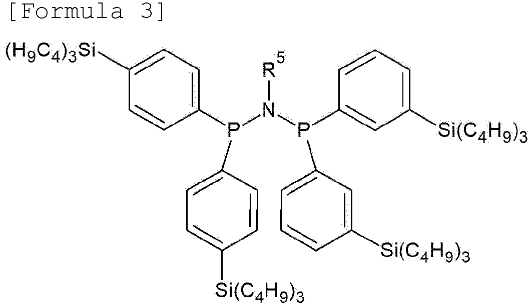 wherein in Formula 3 above,
R⁵ is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.

7. The ligand compound of claim 6, wherein the ligand compound represented by Formula 3 above is one selected from the group consisting of ligand compounds represented by Formula 3-1 to Formula 3-10 below:

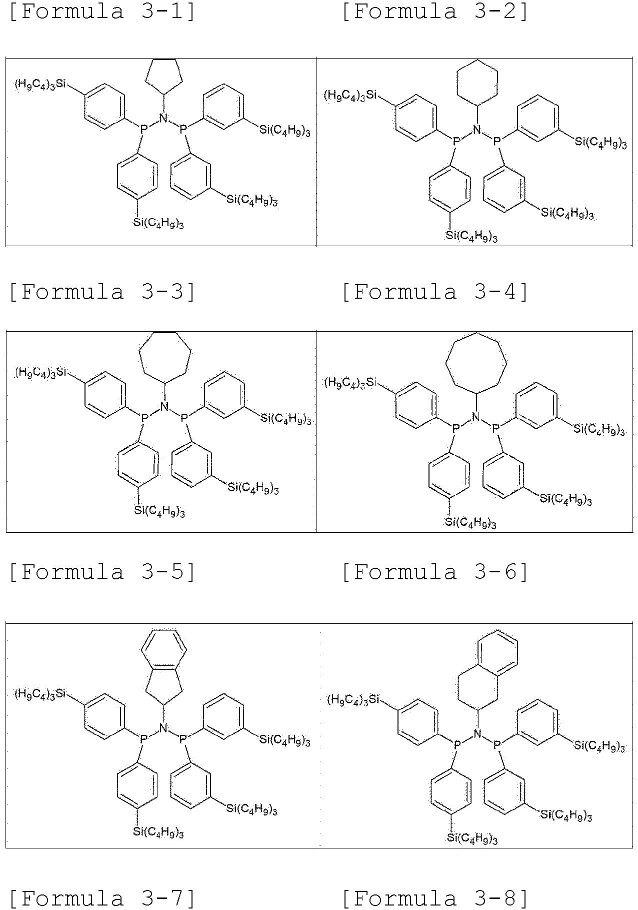

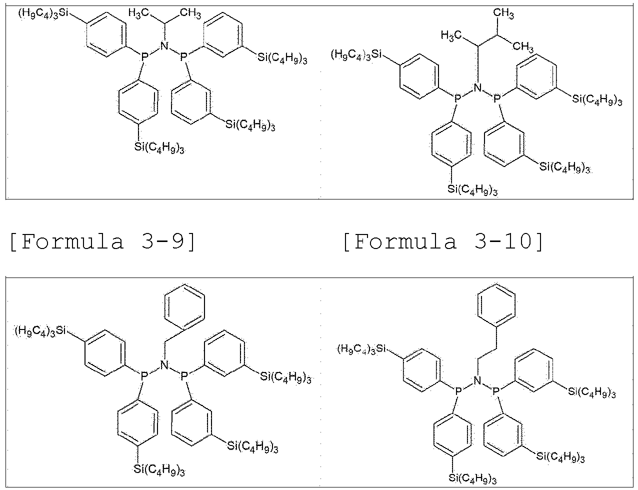

8. The ligand compound of claim 1, wherein the ligand compound represented by Formula 1 above is represented by Formula 4 below: 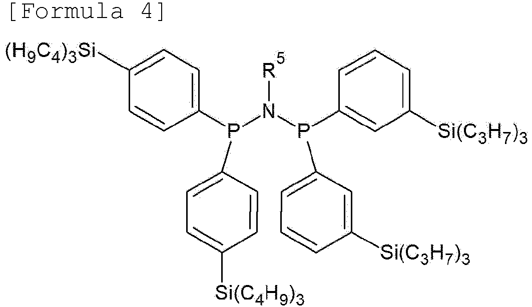 wherein in Formula 4 above,
R⁵ is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.

9. The ligand compound of claim 8, wherein the ligand compound represented by Formula 4 above is one selected from the group consisting of ligand compounds represented by Formula 4-1 to Formula 4-10 below:

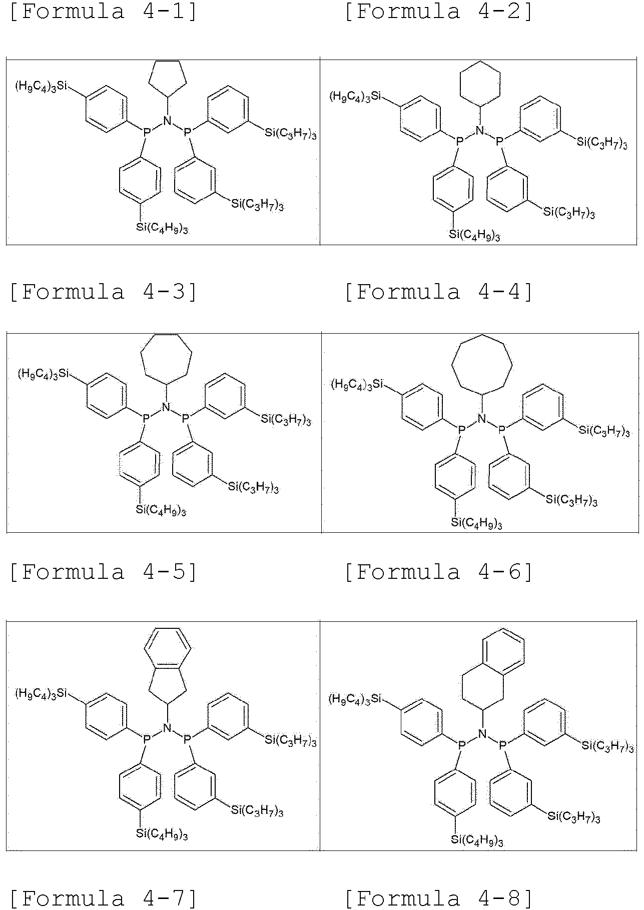

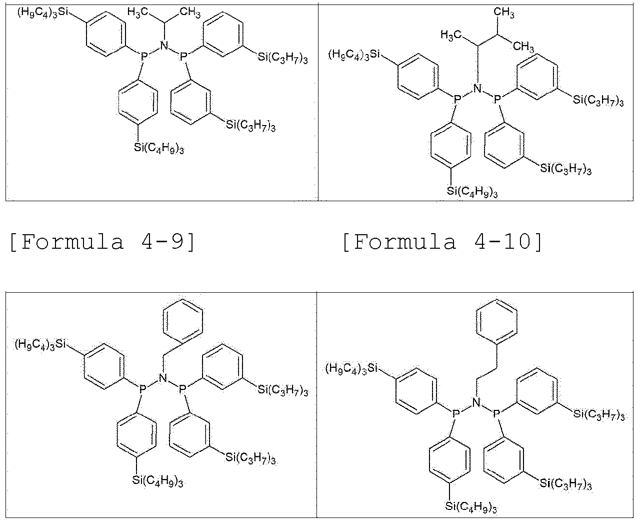

10. The ligand compound of claim 1, wherein the ligand compound represented by Formula 1 above is represented by Formula 5 below: 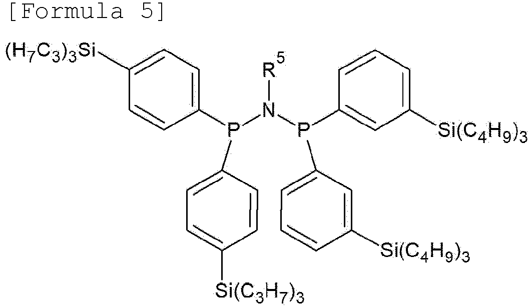 wherein in Formula 5 above,
R⁵ is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.

11. The ligand compound of claim 10, wherein the ligand compound represented by Formula 5 above is one selected from the group consisting of ligand compounds represented by Formula 5-1 to Formula 5-10 below:

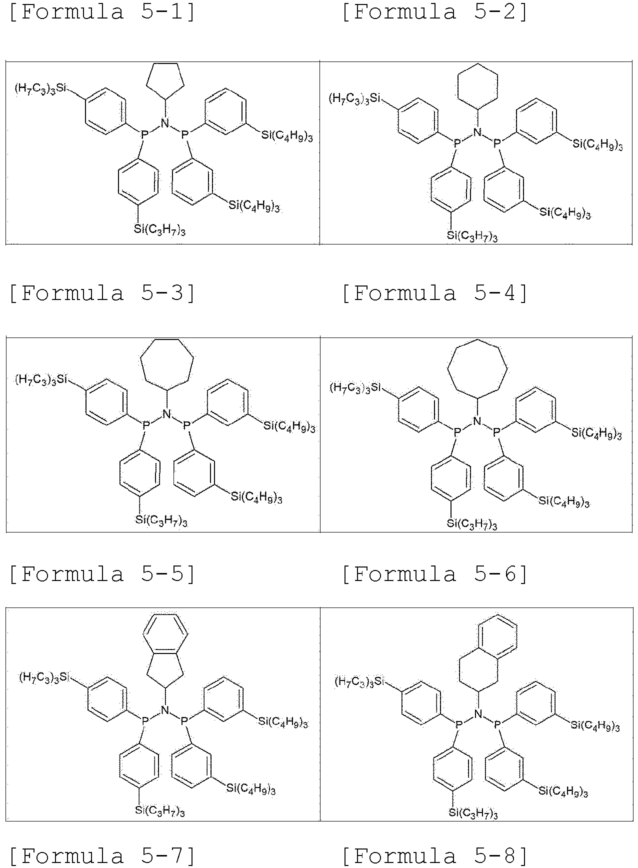

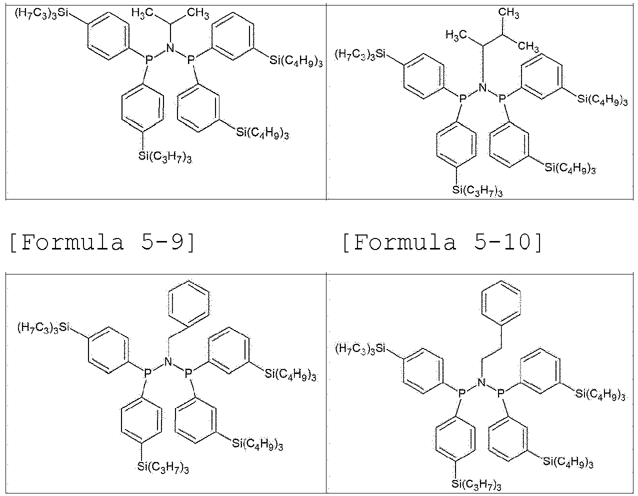

12. The ligand compound of claim 1, wherein the ligand compound represented by Formula 1 above is represented by Formula 6 below: 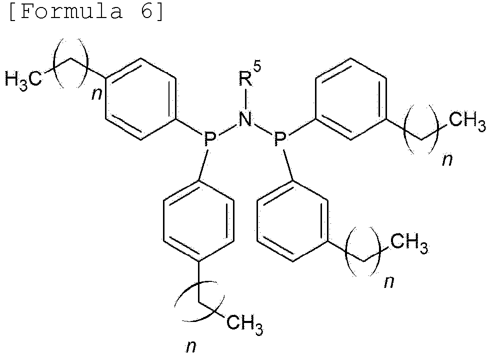 wherein in Formula 6 above,
R⁵ is an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms, and
n is an integer selected from 4 to 14.

13. The ligand compound of claim 8, wherein the ligand compound represented by Formula 6 above is one selected from the group consisting of ligand compounds represented by Formula 6-1 to Formula 6-10 below:

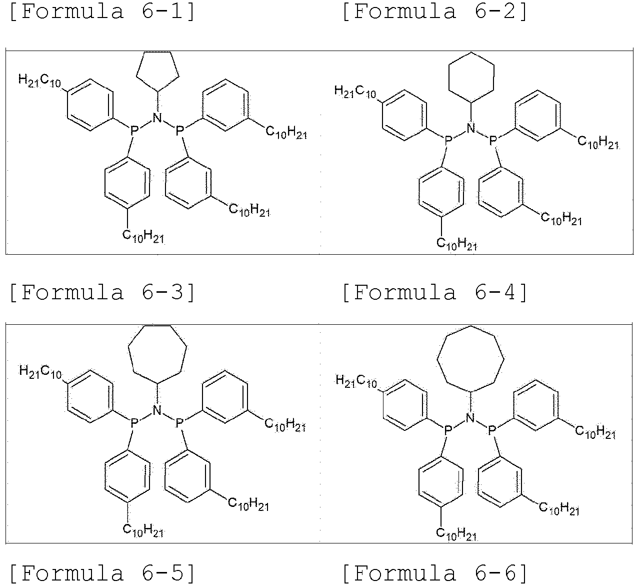

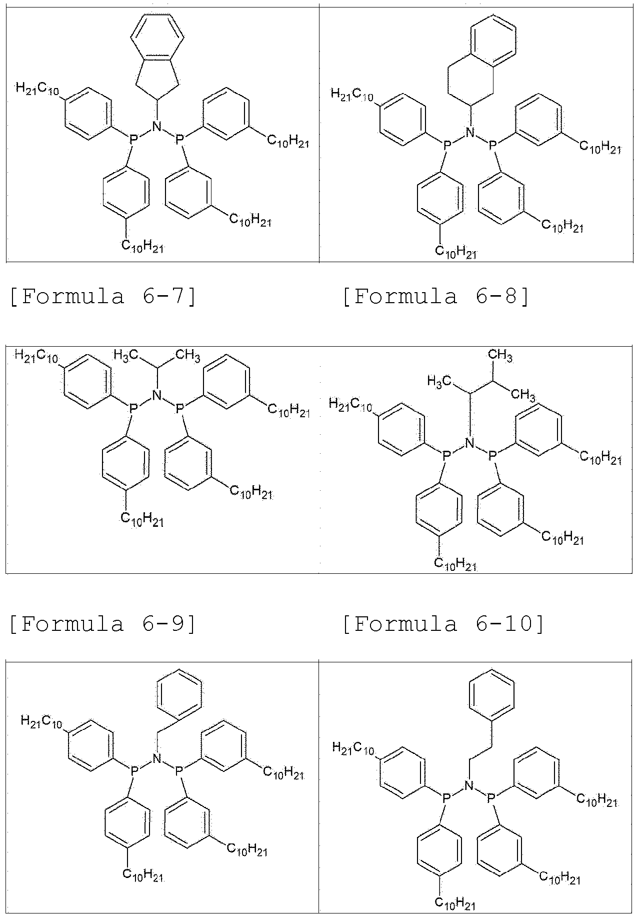

14. An organic chromium compound comprising the ligand compound according to claim 1 and chromium coordinated to the ligand compound.

15. A catalyst composition comprising the ligand compound according to claim 1, chromium, and a co-catalyst.

## Patentansprüche

1. Ligandenverbindung, dargestellt durch die nachfolgende Formel 1: 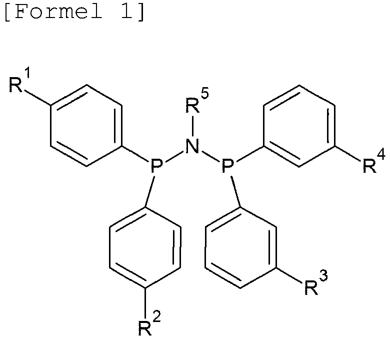 worin in der vorstehenden Formel 1
R¹ bis R⁴ jeweils unabhängig eine Alkylgruppe mit 5 bis 20 Kohlenstoffatomen, eine Cycloalkylgruppe mit 5 bis 20 Kohlenstoffatomen, eine Alkoxyalkylgruppe mit 6 bis 20 Kohlenstoffatomen, eine Arylalkoxyalkylgruppe mit 10 bis 30 Kohlenstoffatomen oder eine Trialkylsilylgruppe sind, worin die Alkylgruppen der Trialkylsilylgruppe jeweils unabhängig eine Alkylgruppe mit 1 bis 10 Kohlenstoffatomen sind, und
R⁵ eine Alkylgruppe mit 1 bis 20 Kohlenstoffatomen, eine Alkylgruppe mit 1 bis 20 Kohlenstoffatomen, die mit einer Arylgruppe mit 6 bis 10 Kohlenstoffatomen substituiert ist, eine Cycloakylgruppe mit 5 bis 10 Kohlenstoffatomen oder eine Cycloalkylgruppe mit 5 bis 10 Kohlenstoffatomen, die mit einer Arylgruppe mit 6 bis 10 Kohlenstoffatomen kondensiert ist, ist.

2. Ligandenverbindung gemäß Anspruch 1, worin R¹ bis R⁴ jeweils unabhängig eine Alkylgruppe mit 8 bis 12 Kohlenstoffatomen, eine Tripropylsilylgruppe oder eine Tributylsilylgruppe sind.

3. Ligandenverbindung gemäß Anspruch 1, worin R⁵ eine Alkylgruppe mit 3 bis 5 Kohlenstoffatomen, eine Alkylgruppe mit 1 bis 5 Kohlenstoffatomen, die mit einer Arylgruppe mit 6 bis 10 Kohlenstoffatomen substituiert ist, eine Cycloalkylgruppe mit 5 bis 8 Kohlenstoffatomen oder eine Cycloalkylgruppe mit 5 bis 8 Kohlenstoffatomen, die mit einer Arylgruppe mit 6 bis 10 Kohlenstoffatomen kondensiert ist, ist.

4. Ligandenverbindung gemäß Anspruch 1, worin die durch die vorstehende Formel 1 dargestellte Ligandenverbindung durch die nachstehende Formel 2 dargestellt ist: 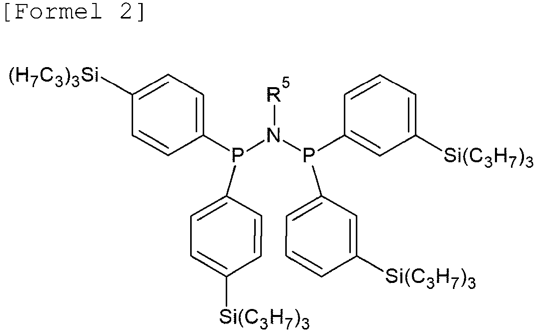 worin in der vorstehenden Formel 2
R⁵ eine Alkylgruppe mit 1 bis 10 Kohlenstoffatomen, eine Alkylgruppe mit 1 bis 10 Kohlenstoffatomen, die mit einer Arylgruppe mit 6 bis 10 Kohlenstoffatomen substituiert ist, eine Cycloalkylgruppe mit 5 bis 10 Kohlenstoffatomen oder eine Cycloalkylgruppe mit 5 bis 10 Kohlenstoffatomen, die mit einer Arylgruppe mit 6 bis 10 Kohlenstoffatomen kondensiert ist, ist.

5. Ligandenverbindung gemäß Anspruch 4, worin die durch die vorstehende Formel 2 dargestellte Ligandenverbindung irgendeine ist, die ausgewählt ist aus den durch die nachstehenden Formeln 2-1 bis 2-10 dargestellten Ligandenverbindungen:

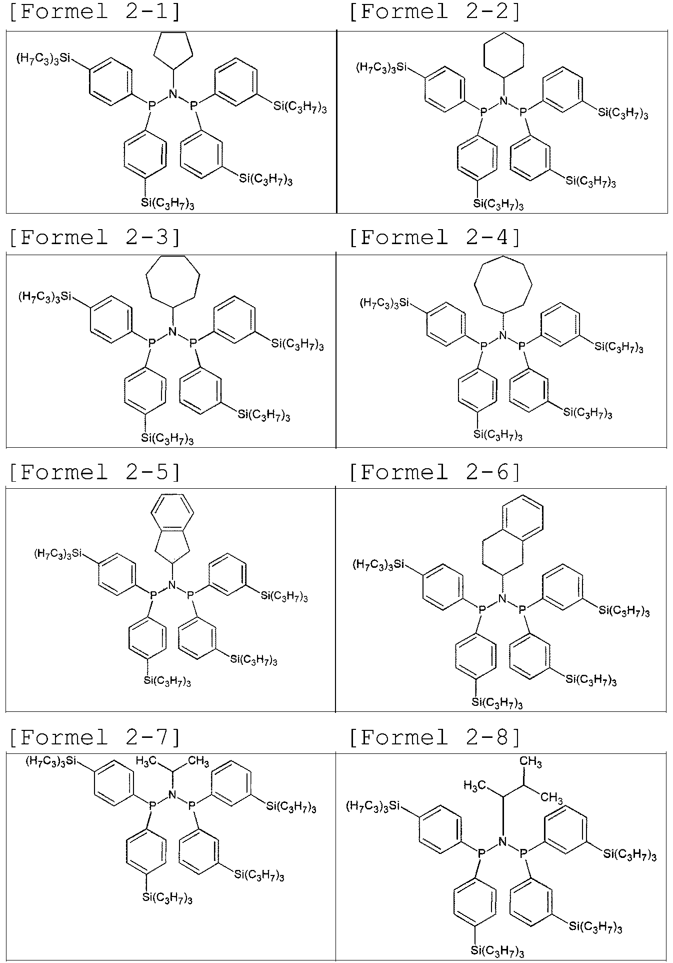

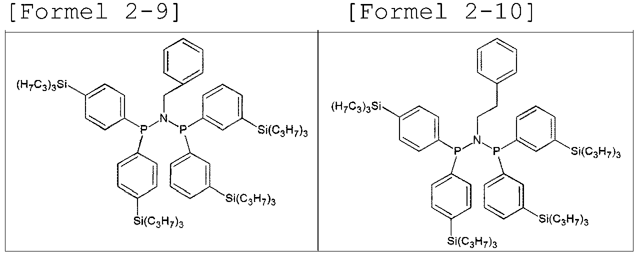

6. Ligandenverbindung gemäß Anspruch 1, worin die durch die vorstehende Formel 1 dargestellte Ligandenverbindung durch die nachstehende Formel 3 dargestellt ist: 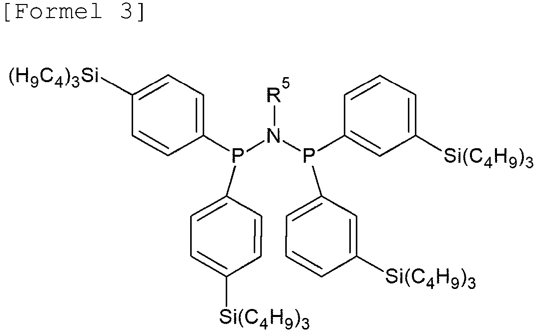 worin in der vorstehenden Formel 3
R⁵ eine Alkylgruppe mit 1 bis 10 Kohlenstoffatomen, eine Alkylgruppe mit 1 bis 10 Kohlenstoffatomen, die mit einer Arylgruppe mit 6 bis 10 Kohlenstoffatomen substituiert ist, eine Cycloalkylgruppe mit 5 bis 10 Kohlenstoffatomen oder eine Cycloalkylgruppe mit 5 bis 10 Kohlenstoffatomen, die mit einer Arylgruppe mit 6 bis 10 Kohlenstoffatomen kondensiert ist, ist.

7. Ligandenverbindung gemäß Anspruch 6, worin die durch die vorstehende Formel 3 dargestellte Ligandenverbindung eine ist, die ausgewählt ist aus den durch die nachstehenden Formeln 3-1 bis 3-10 dargestellten Ligandenverbindungen:

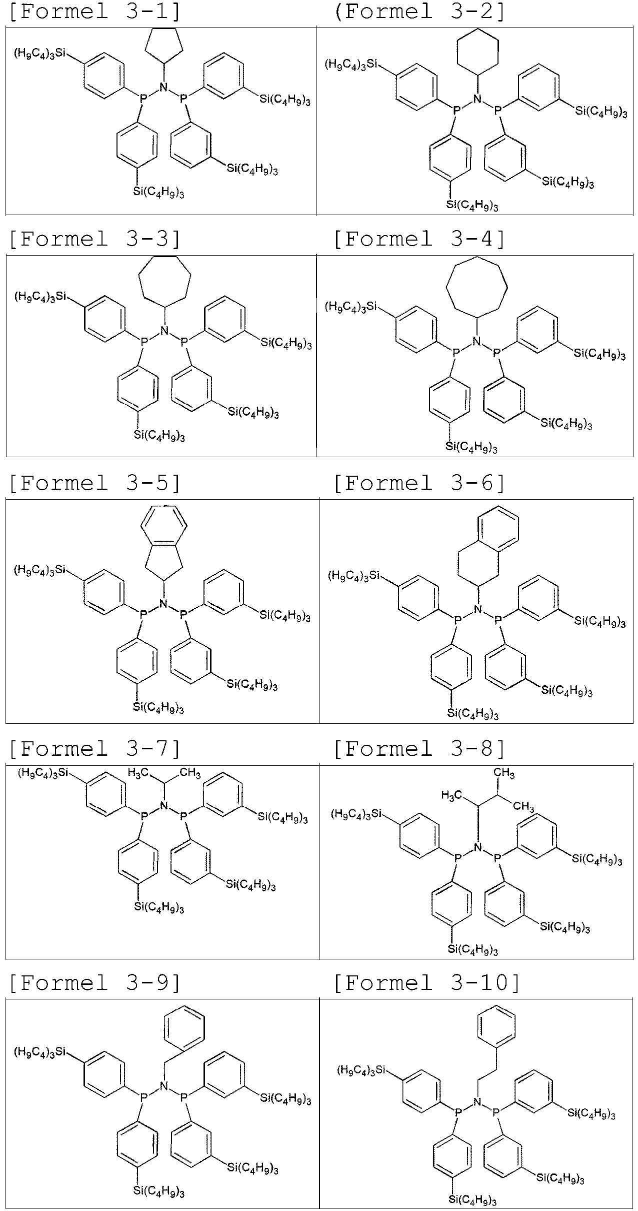

8. Ligandenverbindung gemäß Anspruch 1, worin die durch die vorstehende Formel 1 dargestellte Ligandenverbindung durch die nachstehende Formel 4 dargestellt ist: 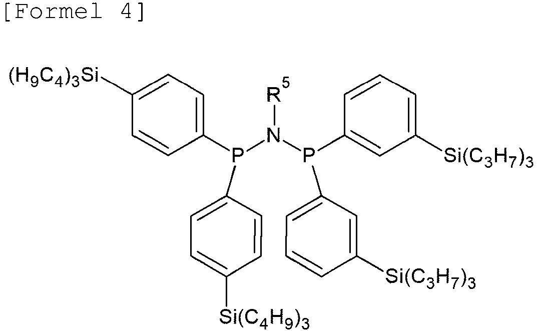 worin in der vorstehenden Formel 4
R⁵ eine Alkylgruppe mit 1 bis 10 Kohlenstoffatomen, eine Alkylgruppe mit 1 bis 10 Kohlenstoffatomen, die mit einer Arylgruppe mit 6 bis 10 Kohlenstoffatomen substituiert ist, eine Cycloalkylgruppe mit 5 bis 10 Kohlenstoffatomen oder eine Cycloalkylgruppe mit 5 bis 10 Kohlenstoffatomen, die mit einer Arylgruppe mit 6 bis 10 Kohlenstoffatomen kondensiert ist, ist.

9. Ligandenverbindung gemäß Anspruch 8, worin die durch die vorstehende Formel 4 dargestellte Ligandenverbindung eine ist, die ausgewählt ist aus den Ligandenverbindungen, die durch die nachstehenden Formeln 4-1 bis 4-10 dargestellt sind:

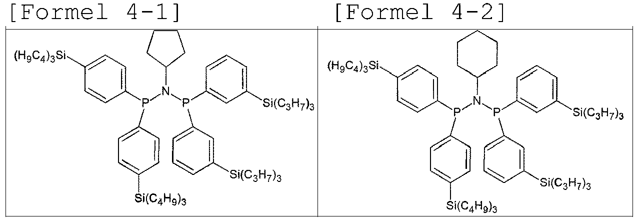

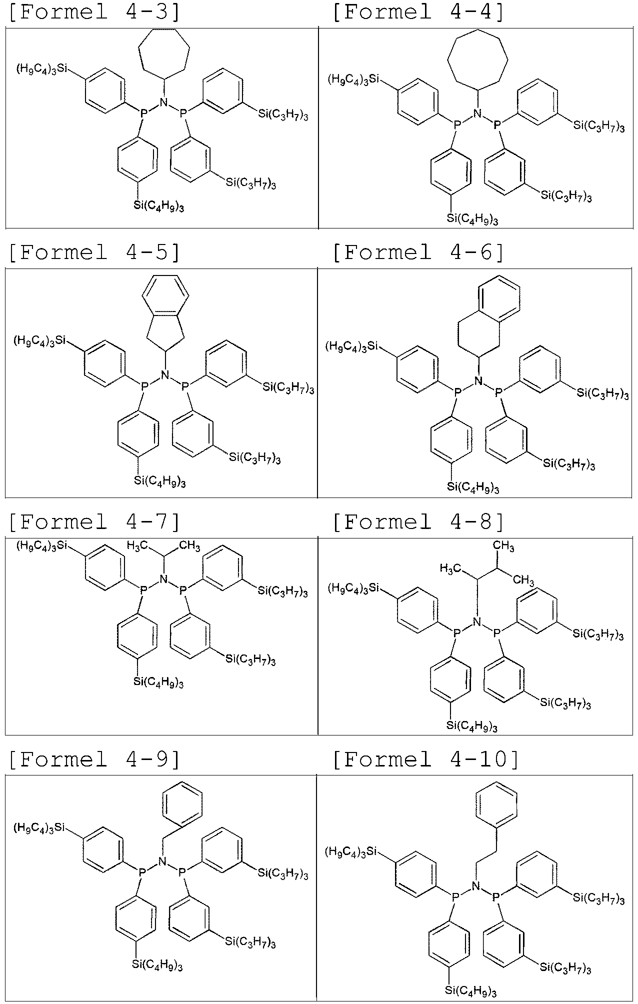

10. Ligandenverbindung gemäß Anspruch 1, worin die durch die vorstehende Formel 1 dargestellte Ligandenverbindung durch die nachstehende Formel 5 dargestellt ist: 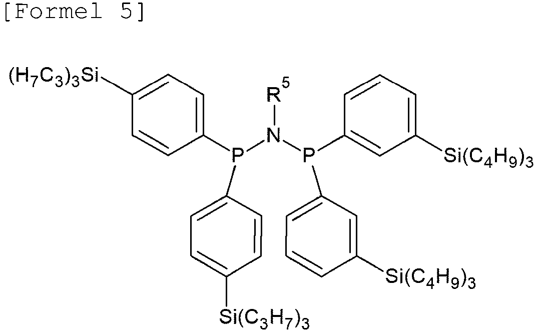 worin in der vorstehenden Formel 5
R⁵ eine Alkylgruppe mit 1 bis 10 Kohlenstoffatomen, eine Alkylgruppe mit 1 bis 10 Kohlenstoffatomen, die mit einer Arylgruppe mit 6 bis 10 Kohlenstoffatomen substituiert ist, eine Cycloalkylgruppe mit 5 bis 10 Kohlenstoffatomen oder eine Cycloalkylgruppe mit 5 bis 10 Kohlenstoffatomen, die mit einer Arylgruppe mit 6 bis 10 Kohlenstoffatomen kondensiert ist, ist.

11. Ligandenverbindung gemäß Anspruch 10, worin die durch die vorstehende Formel 5 dargestellte Ligandenverbindung eine ist, die ausgewählt ist aus der Gruppe, bestehend aus den durch die nachstehenden Formeln 5-1 bis 5-10 dargestellten Ligandenverbindungen:

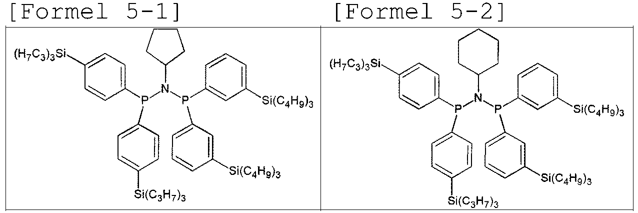

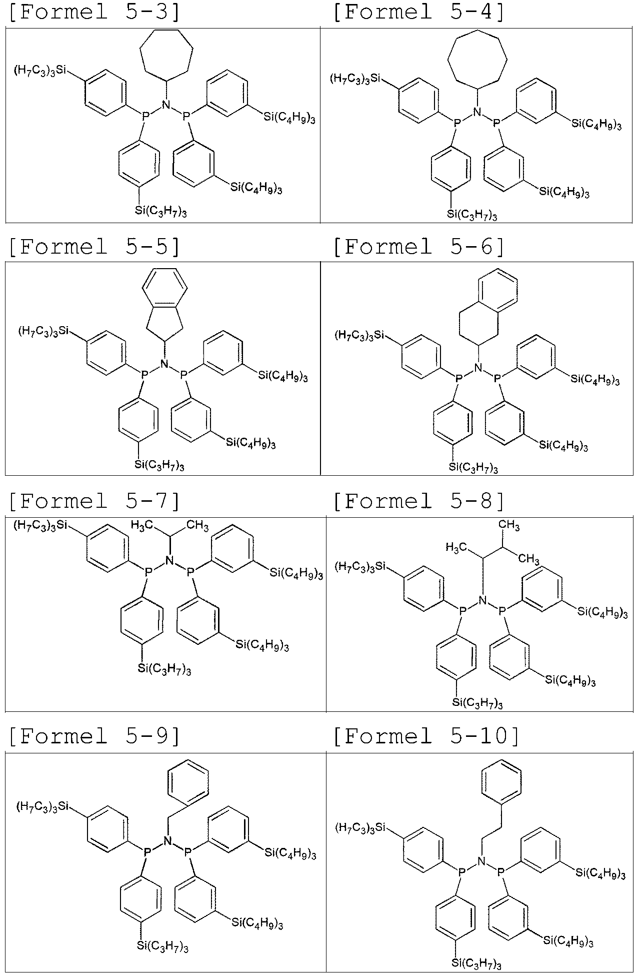

12. Ligandenverbindung gemäß Anspruch 1, worin die durch die vorstehende Formel 1 dargestellte Ligandenverbindung durch die nachstehende Formel 6 dargestellt ist: 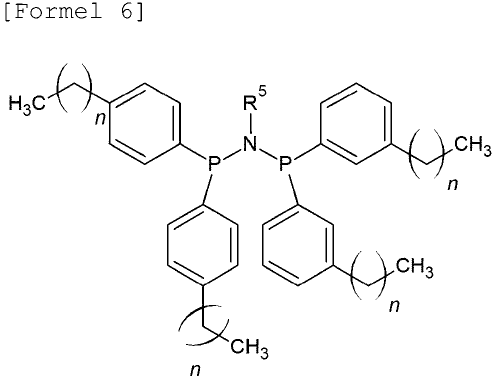 worin in der vorstehenden Formel 6
R⁵ eine Alkylgruppe mit 1 bis 10 Kohlenstoffatomen, eine Alkylgruppe mit 1 bis 10 Kohlenstoffatomen, die mit einer Arylgruppe mit 6 bis 10 Kohlenstoffatomen substituiert ist, eine Cycloalkylgruppe mit 5 bis 10 Kohlenstoffatomen oder eine Cycloalkylgruppe mit 5 bis 10 Kohlenstoffatomen, die mit einer Arylgruppe mit 6 bis 10 Kohlenstoffatomen kondensiert ist, ist, und
n eine ganze Zahl ist, ausgewählt aus 4 bis 14.

13. Ligandenverbindung gemäß Anspruch 8, worin die durch die vorstehende Formel 6 dargestellte Ligandenverbindung eine ist, die ausgewählt ist aus der Gruppe, bestehend aus den durch die nachstehenden Formeln 6-1 bis 6-10 dargestellten Ligandenverbindungen:

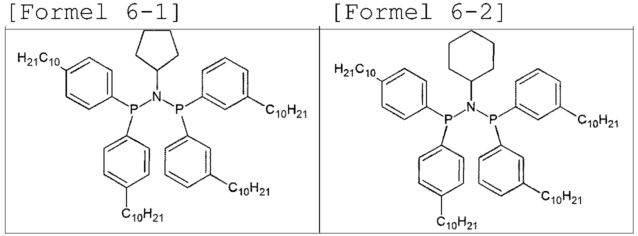

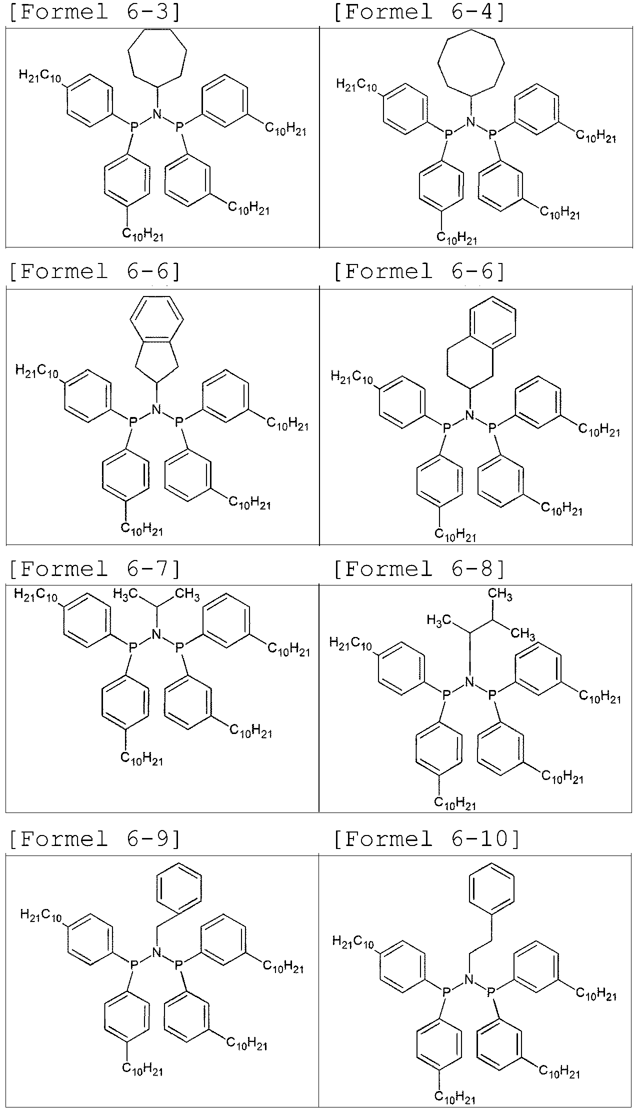

14. Organische Chromverbindung, umfassend die Ligandenverbindung gemäß Anspruch 1 und Chrom, das an die Ligandenverbindung koordiniert ist.

15. Katalysatorzusammensetzung, umfassend die Ligandenverbindung gemäß Anspruch 1, Chrom und einen CoKatalysator.

## Revendications

1. Composé ligand représenté par la Formule 1 ci-dessous : 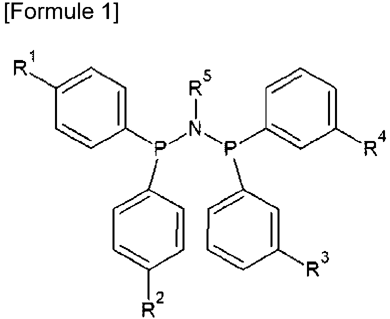 dans lequel, dans la Formule 1 ci-dessus,
R¹ à R⁴ sont chacun indépendamment un groupe alkyle présentant 5 à 20 atomes de carbone, un groupe cycloalkyle présentant 5 à 20 atomes de carbone, un groupe alkoxyalkyle présentant 6 à 20 atomes de carbone, un groupe arylalkoxyalkyle présentant 10 à 30 atomes de carbone, ou un groupe trialkylsilyle, les groupes alkyle du groupe trialkylsilyle étant chacun indépendamment un groupe alkyle présentant 1 à 10 atomes de carbone, et
R⁵ étant un groupe alkyle présentant 1 à 20 atomes de carbone, un groupe alkyle présentant 1 à 20 atomes de carbone substitué par un groupe aryle présentant 6 à 10 atomes de carbone, un groupe cycloalkyle présentant 5 à 10 atomes de carbone, ou un groupe cycloalkyle présentant 5 à 10 atomes de carbone fusionné avec un groupe aryle présentant 6 à 10 atomes de carbone.

2. Composé ligand selon la revendication 1, dans lequel R¹ à R⁴ sont chacun indépendamment un groupe alkyle présentant de 8 à 12 atomes de carbone, un groupe tripropylsilyle ou un groupe tributylsilyle.

3. Composé ligand selon la revendication 1, dans lequel R⁵ est un groupe alkyle présentant de 3 à 5 atomes de carbone, un groupe alkyle présentant de 1 à 5 atomes de carbone substitué par un groupe aryle présentant de 6 à 10 atomes de carbone, un groupe cycloalkyle présentant de 5 à 8 atomes de carbone, ou un groupe cycloalkyle présentant de 5 à 8 atomes de carbone fusionné avec un groupe aryle présentant de 6 à 10 atomes de carbone.

4. Composé ligand selon la revendication 1, dans lequel le composé ligand représenté par la Formule 1 ci-dessus est représenté par la Formule 2 ci-dessous : 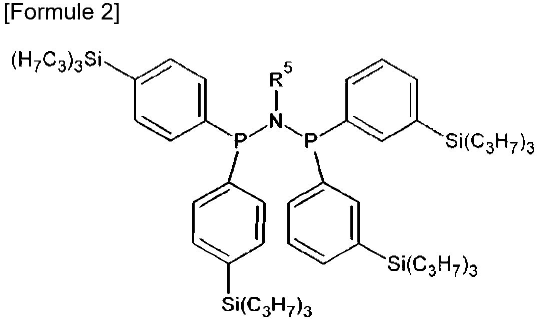 dans lequel, dans la Formule 2 ci-dessus,
R⁵ est un groupe alkyle présentant de 1 à 10 atomes de carbone, un groupe alkyle présentant de 1 à 10 atomes de carbone substitué par un groupe aryle présentant de 6 à 10 atomes de carbone, un groupe cycloalkyle présentant de 5 à 10 atomes de carbone, ou un groupe cycloalkyle présentant de 5 à 10 atomes de carbone fusionné avec un groupe aryle présentant de 6 à 10 atomes de carbone.

5. Composé ligand selon la revendication 4, dans lequel le composé ligand représenté par la Formule 2 ci-dessus est choisi parmi le groupe constitué de composés ligands représentés par la Formule 2-1 à la Formule 2-10 ci-dessous :

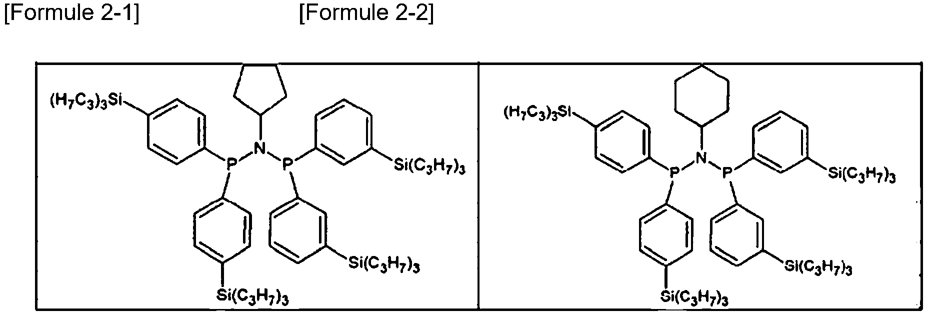

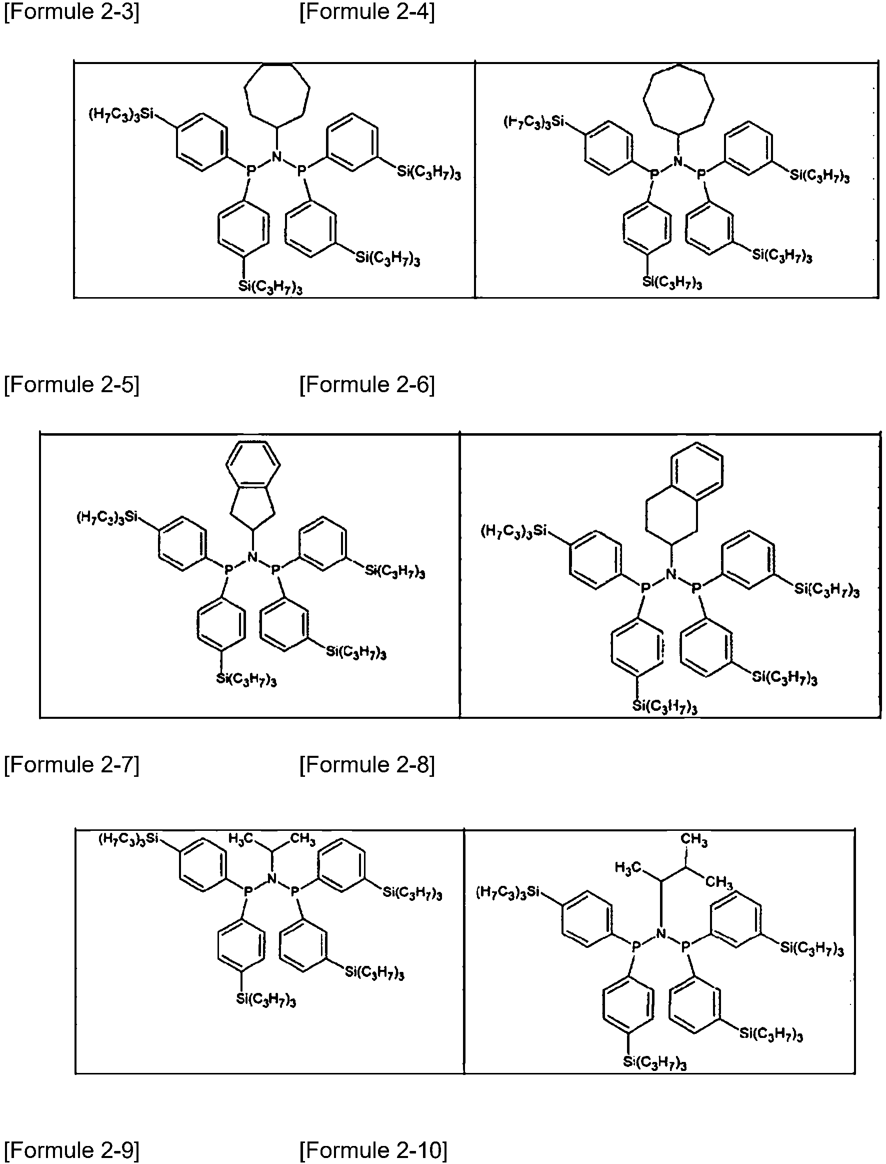

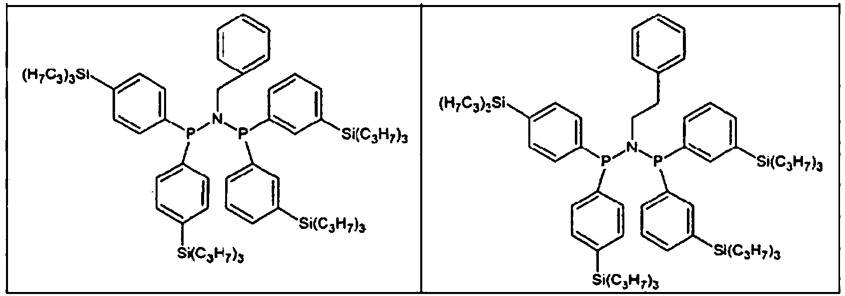

6. Composé ligand selon la revendication 1, dans lequel le composé ligand représenté par la Formule 1 ci-dessus est représenté par la Formule 3 ci-dessous : 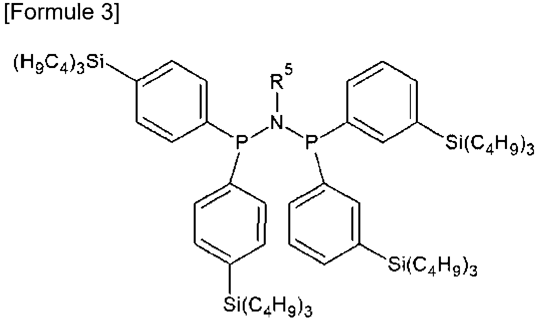 dans lequel, dans la Formule 3 ci-dessus,
R⁵ est un groupe alkyle présentant de 1 à 10 atomes de carbone, un groupe alkyle présentant de 1 à 10 atomes de carbone substitué par un groupe aryle présentant de 6 à 10 atomes de carbone, un groupe cycloalkyle présentant de 5 à 10 atomes de carbone, ou un groupe cycloalkyle présentant de 5 à 10 atomes de carbone fusionné avec un groupe aryle présentant de 6 à 10 atomes de carbone.

7. Composé ligand selon la revendication 6, dans lequel le composé ligand représenté par la Formule 3 ci-dessus est choisi parmi le groupe constitué de composés ligands représentés par la Formule 3-1 à la Formule 3-10 ci-dessous :

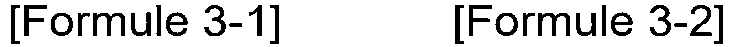

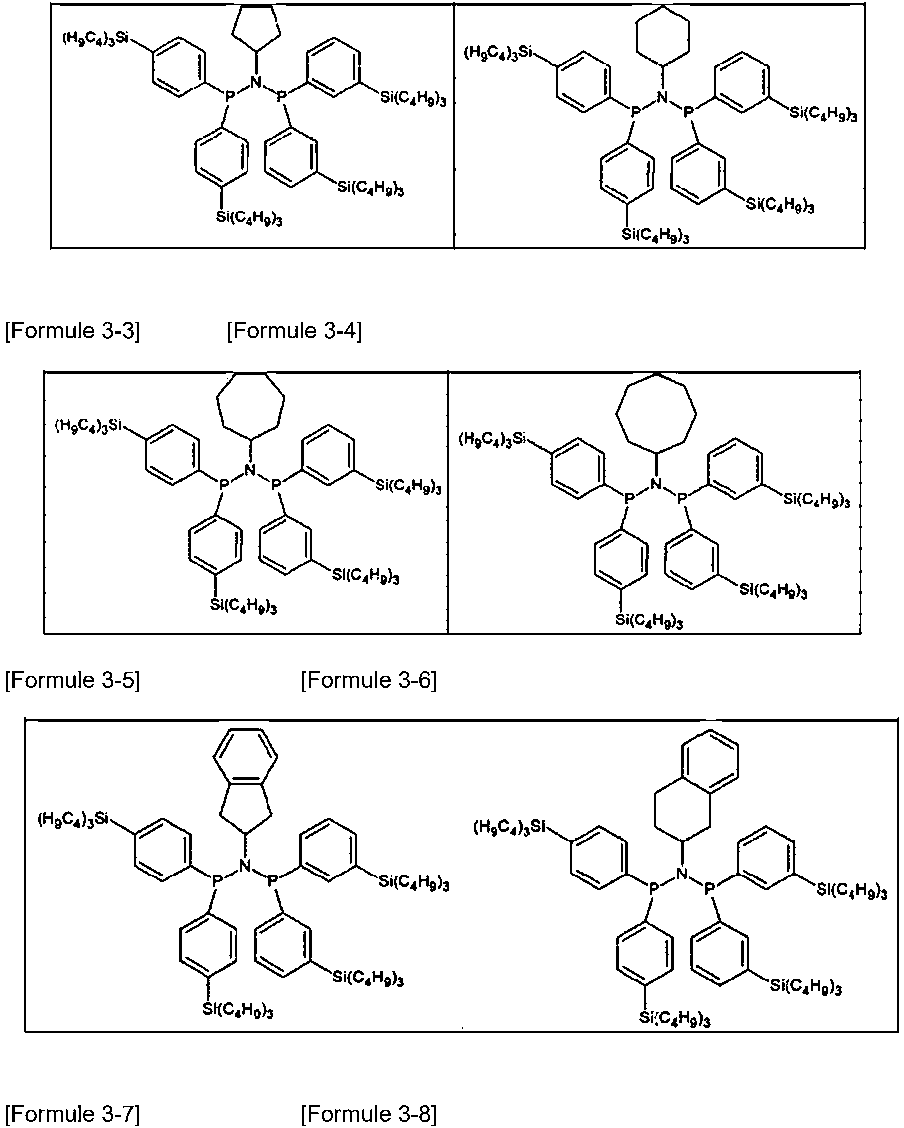

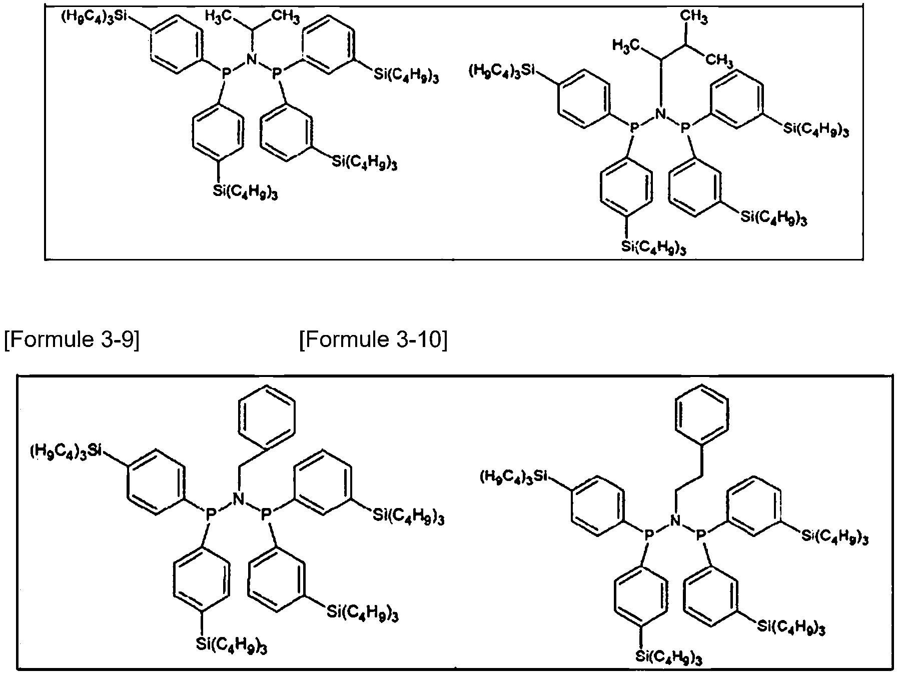

8. Composé ligand selon la revendication 1, dans lequel le composé ligand représenté par la Formule 1 ci-dessus est représenté par la Formule 4 ci-dessous : 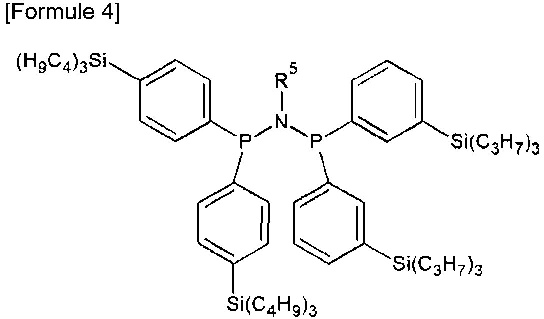 dans lequel, dans la Formule 4 ci-dessus,
R⁵ est un groupe alkyle présentant de 1 à 10 atomes de carbone, un groupe alkyle présentant de 1 à 10 atomes de carbone substitué par un groupe aryle présentant de 6 à 10 atomes de carbone, un groupe cycloalkyle présentant de 5 à 10 atomes de carbone, ou un groupe cycloalkyle présentant de 5 à 10 atomes de carbone fusionné avec un groupe aryle présentant de 6 à 10 atomes de carbone.

9. Composé ligand selon la revendication 8, dans lequel le composé ligand représenté par la Formule 4 ci-dessus est choisi parmi le groupe constitué de composés ligands représentés par la Formule 4-1 à la Formule 4-10 ci-dessous :

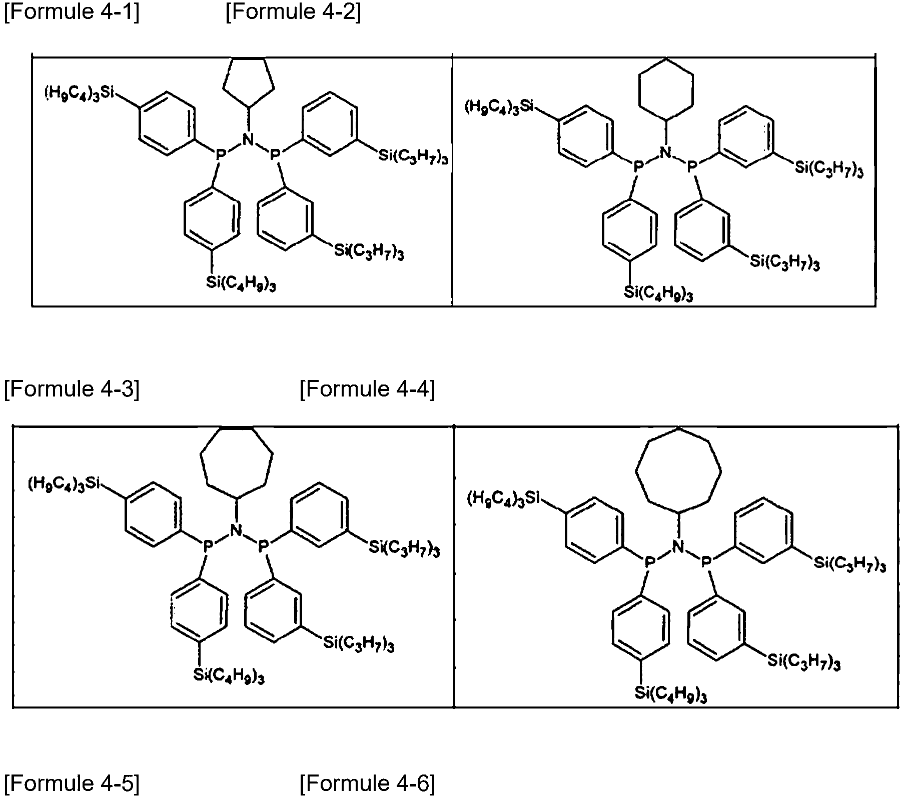

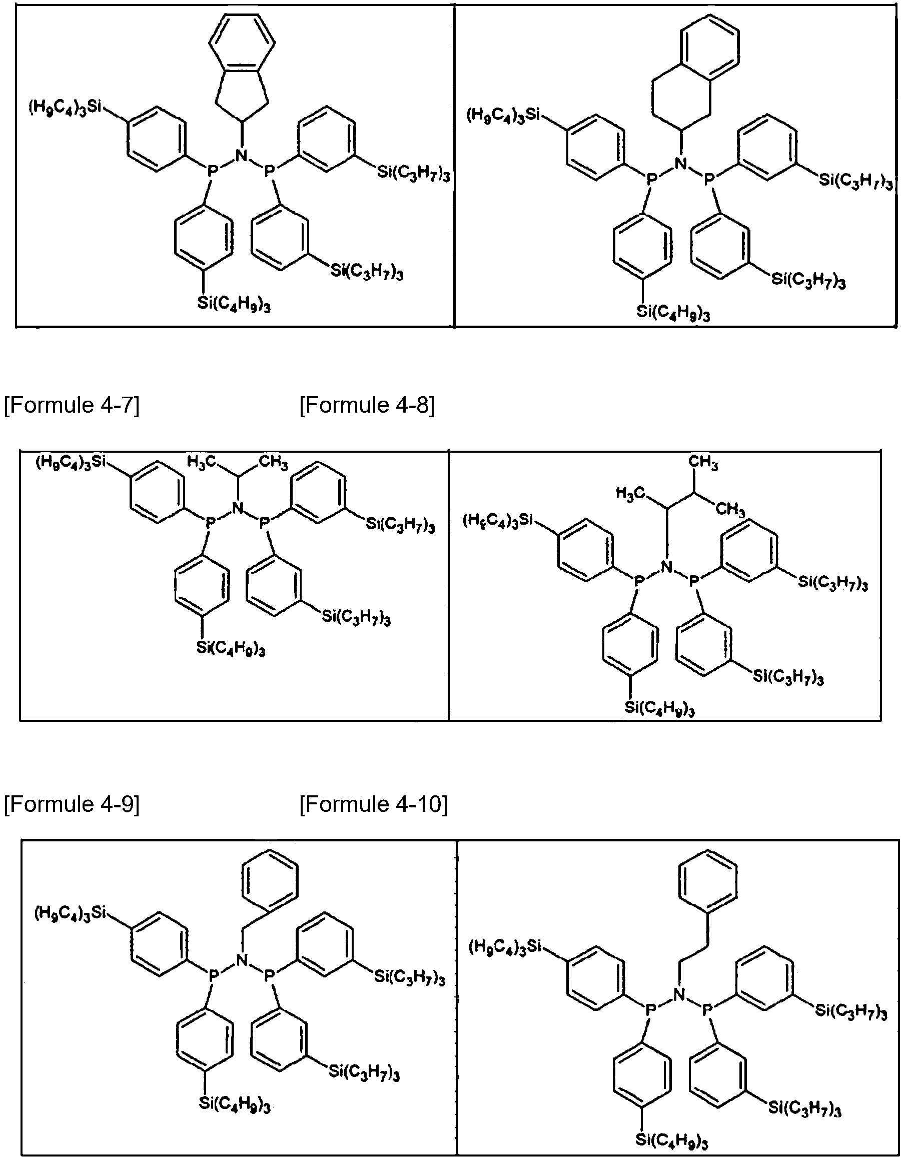

10. Composé ligand selon la revendication 1, dans lequel le composé ligand représenté par la Formule 1 ci-dessus est représenté par la Formule 5 ci-dessous : 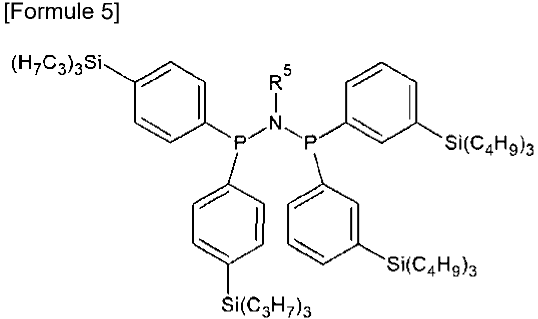 dans lequel, dans la Formule 5 ci-dessus,
R⁵ est un groupe alkyle présentant de 1 à 10 atomes de carbone, un groupe alkyle présentant de 1 à 10 atomes de carbone substitué par un groupe aryle présentant de 6 à 10 atomes de carbone, un groupe cycloalkyle présentant de 5 à 10 atomes de carbone, ou un groupe cycloalkyle présentant de 5 à 10 atomes de carbone fusionné avec un groupe aryle présentant de 6 à 10 atomes de carbone.

11. Composé ligand selon la revendication 10, dans lequel le composé ligand représenté par la Formule 5 ci-dessus est choisi parmi le groupe constitué de composés ligands représentés par la Formule 5-1 à la Formule 5-10 ci-dessous :

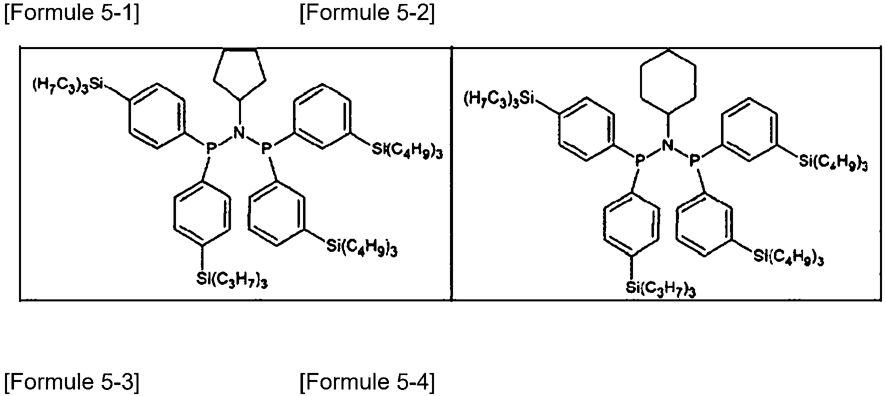

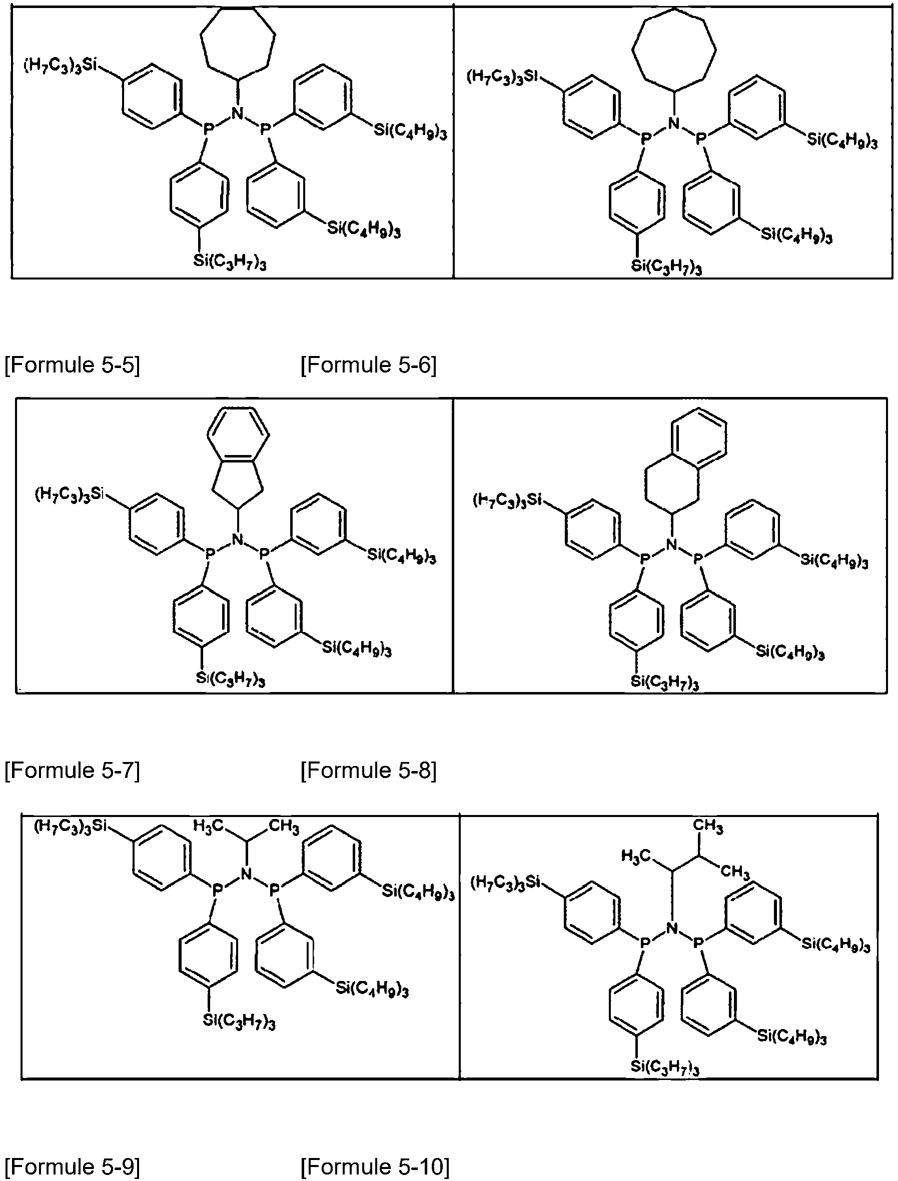

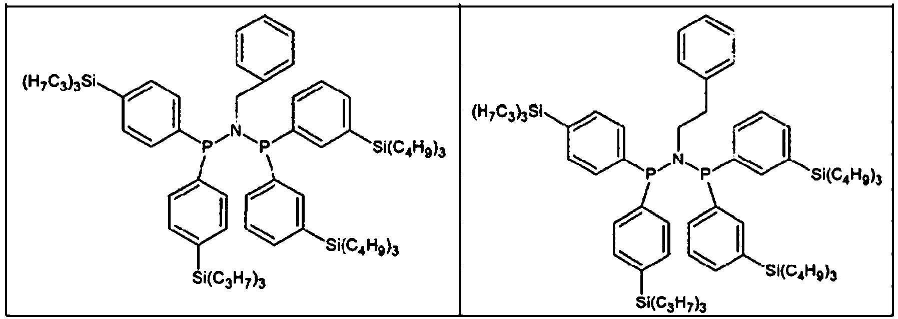

12. Composé ligand selon la revendication 1, dans lequel le composé ligand représenté par la Formule 1 ci-dessus est représenté par la Formule 6 ci-dessous : 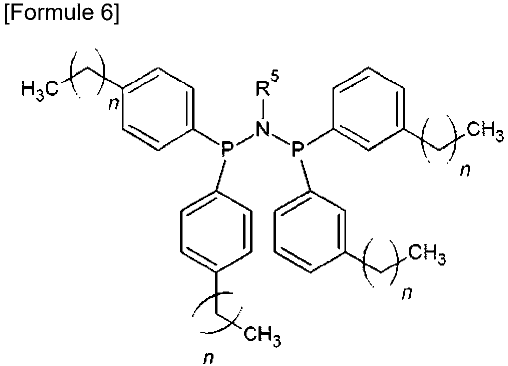 dans lequel, dans la Formule 6 ci-dessus,
R⁵ est un groupe alkyle présentant de 1 à 10 atomes de carbone, un groupe alkyle présentant de 1 à 10 atomes de carbone substitué par un groupe aryle présentant de 6 à 10 atomes de carbone, un groupe cycloalkyle présentant de 5 à 10 atomes de carbone, ou un groupe cycloalkyle présentant de 5 à 10 atomes de carbone fusionné avec un groupe aryle présentant de 6 à 10 atomes de carbone, et
n est un nombre entier choisi parmi 4 à 14.

13. Composé ligand selon la revendication 8, dans lequel le composé ligand représenté par la Formule 6 ci-dessus est choisi parmi le groupe constitué de composés ligands représentés par la Formule 6-1 à la Formule 6-10 ci-dessous :

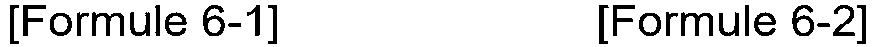

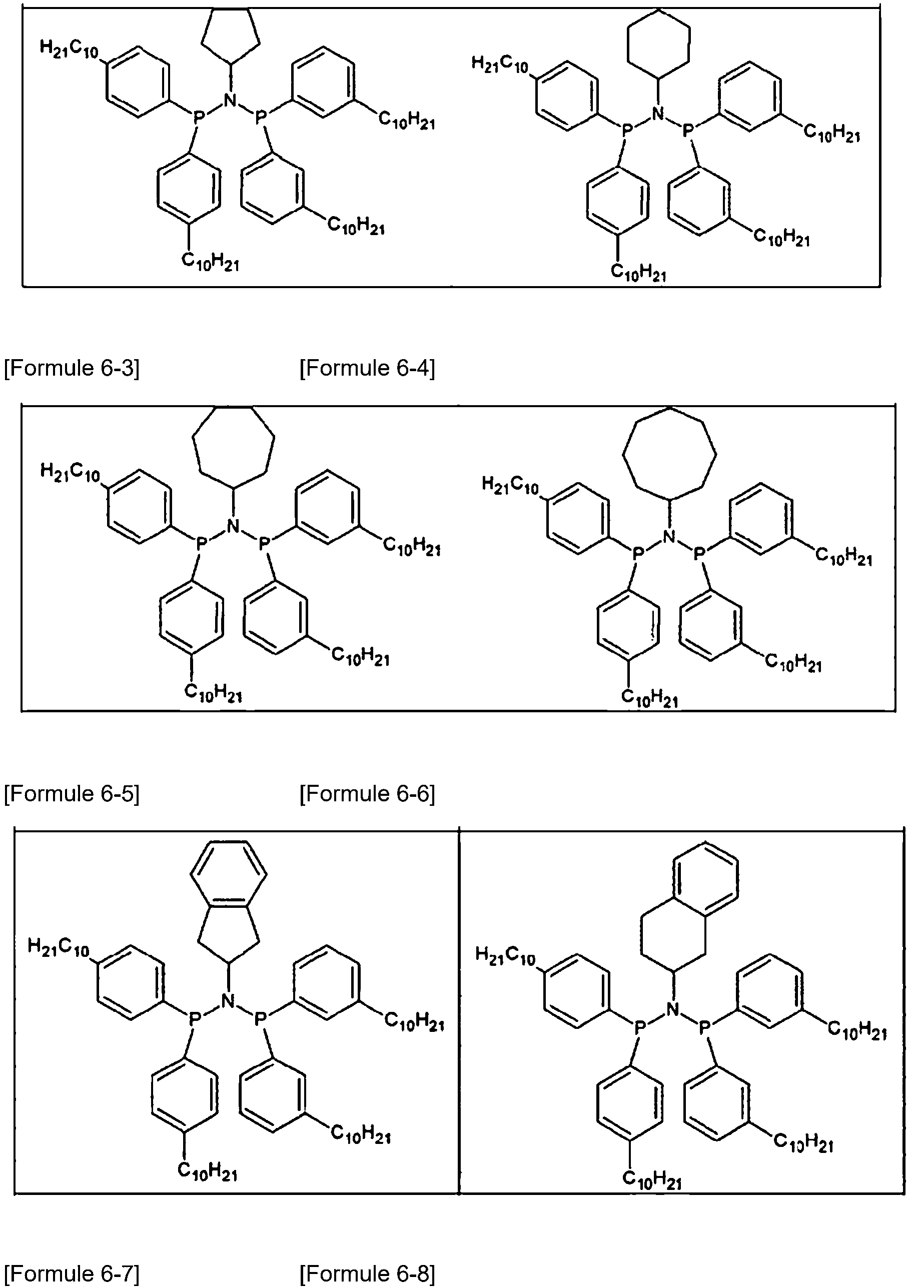

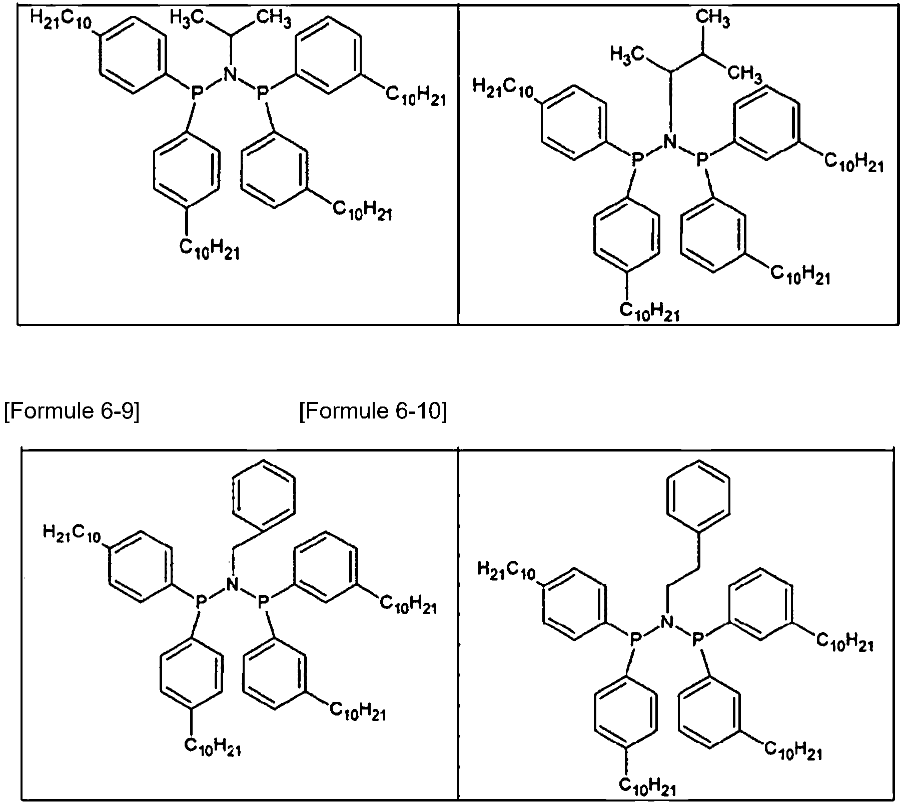

14. Composé organique de chrome comprenant le composé ligand selon la revendication 1 et du chrome coordonné au composé ligand.

15. Composition de catalyseur comprenant le composé ligand selon la revendication 1, du chrome et un co-catalyseur.
